(19) Europäisches Patentamt
European Patent Office
Office européen des brevets

(11) EP 2 707 006 B1

(12) # EUROPEAN PATENT SPECIFICATION

(45) Date of publication and mention of the grant of the patent:
**03.07.2019 Bulletin 2019/27**

(21) Application number: **12781920.9**

(22) Date of filing: **11.05.2012**

(51) Int Cl.:
***A61K 9/10*** *(2006.01)* ***A61K 31/196*** *(2006.01)*
***A61K 33/18*** *(2006.01)* ***A61P 27/02*** *(2006.01)*

(86) International application number:
**PCT/US2012/037563**

(87) International publication number:
**WO 2012/155062 (15.11.2012 Gazette 2012/46)**

(54) **STABLE POVIDONE-IODINE COMPOSITIONS WITH BROMFENAC**
STABILE POVIDON-IOD-ZUSAMMENSETZUNGEN MIT BROMFENAC
COMPOSITIONS DE POVIDONE IODÉE STABLES AVEC DE BROMFÉNAC

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.05.2011 US 201161485475 P**

(43) Date of publication of application:
**19.03.2014 Bulletin 2014/12**

(73) Proprietor: **Foresight Biotherapeutics, Inc.**
**New York, NY 10019 (US)**

(72) Inventors:
- **STEIN, Jason**
  **New York, NY 10162 (US)**
- **WEISER, Michael**
  **New York, NY 10028 (US)**
- **CAPRIOTTI, Joseph**
  **Usvi 00820 (VI)**
- **LIANG, Bo**
  **East Brunswick, NJ 08816 (US)**
- **SAMSON, C., Michael**
  **New York, NY 10016 (US)**

(74) Representative: **Warner, James Alexander et al**
**Carpmaels & Ransford LLP**
**One Southampton Row**
**London WC1B 5HA (GB)**

(56) References cited:
**WO-A1-2009/151619**
**WO-A1-2009/151619**
**WO-A1-2011/084473**
**WO-A1-2013/040347**
**WO-A2-2007/106381**
**WO-A2-2007/106381**
**US-A- 3 886 268**
**US-A1- 2010 254 934**
**US-A1- 2010 291 019**
**US-A1- 2012 027 716**
**US-B2- 7 767 217**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

BACKGROUND

Topical corticosteroids are routinely used to control ocular inflammation. Their mechanism of action involves the inhibition of the immune response and the subsequent tissue destruction that exuberant inflammation may cause. Corticosteroid has the undesirable side effect of limiting the body's intrinsic ability to fight infection. In fact, inopportune steroids usage can worsen the course of an infection secondary to mycobacteria, virus, or fungus. Thus, the use of a combined antimicrobial-steroid medication in ocular infections is recommended only under careful observation of a trained ophthalmologist because of these significant risks. In fact, TOBRADEX (Alcon), the most commonly prescribed combination ophthalmic antimicrobial-steroid drug, specifically lists 'viral disease of the cornea and conjunctiva, mycobacteria infection, and fungal infection' as absolute contraindications to its use. Clearly, these combination drugs were not intended to be used in the face of infectious conjunctivitis in which bacterial infection cannot be confirmed.
In U.S. Patent 7,767,217, it is shown that under certain specific conditions, dexamethasone can be combined with povidone-iodine (PVP-1) to form an effective antimicrobial-steroid pharmaceutical composition. However, it is also shown that most preparations which combine PVP-I (or iodine) with a steroid suffer from instability due, in part, to reactivity of the iodine with the steroid. In fact, U.S. Patent 3,886,268 demonstrates the well- known instability of steroid-iodine combinations.

BRIEF SUMMARY

Embodiments of the present invention are set out in the enclosed claims.
In one embodiment, the invention provides an ophthalmic composition suitable for topical administration to an eye, effective for treatment of a microorganism infection or a disorder of at least one tissue of the eye, comprising:

0.3 to 1% (w/w) polyvinylpyrrolidinone-iodine complex;
0.05 to 2% (w/w) bromfenac;
0.005% to 0.02% (w/w) EDTA;
0.01 o 0.5% (w/w) sodium chloride;
0.02 to 0.1% (w/w) tyloxapol;
0.5% to 2% (w/w) sodium sulfate; and
0.1 to 0.5% (w/w) hydroxyethylcellulose.

In one embodiment the invention provides an ophthalmic composition as defined above for use in a method of treating an eye disorder selected from the group consisting of a microorganism infection of at least one tissue of the eye, conjunctivitis, corneal abrasion, ulcerative infectious keratitis, epithelial keratitis, stromal keratitis and herpesvirus-related keratitis.
Embodiments disclosed herein which do not fall within the scope of the claims do not form part of the invention.
In an embodiment, disclosed herein is an ophthalmic composition suitable for topical administration to an eye, effective for treatment and/or prophylaxis of a microorganism infection or a disorder of at least one tissue of the eye, comprising povidone-iodine in a concentration between 0.01% and 10%, and a steroid selected from the group consisting of prednisolone acetate, loteprednol etabonate, difluprednate, hydrocortisone acetate, and combinations thereof. In an embodiment, the povidone-iodine is between 0.1% and 2.5% by weight. In an embodiment, the povidone-iodine is between 0.5% and 2% by weight. In an embodiment, the total weight of the povidone-iodine and the steroid is between 0.1% and 4.5% in the solution. In an embodiment, the steroid is at a concentration of between 0.01 and 2%. In an embodiment, the steroid is at a concentration of between 0.05 and 1%.
In an embodiment, disclosed herein is a pharmaceutical composition comprising povidone-iodine in a concentration between 0.01% and 10%, and a steroid selected from the group consisting of prednisolone acetate, loteprednol etabonate, difluprednate, and combinations thereof, wherein the steroid is at a concentration of between 0.05 and 1%. In an embodiment, the PVP-I is at a concentration of about 0.4%. In an embodiment, the steroid is at a concentration selected from the group consisting of about 0.1%, about 0.05% and about 0.005%.
In an embodiment, an ophthalmic composition further comprises a topical anesthetic which relieves pain. In an embodiment, a topical anesthetic is selected from the group consisting of proparacaine, lidocaine, tetracaine and a combination thereof.
In an embodiment, an ophthalmic composition further comprises a penetration enhancer which enhances the penetration of povidone-iodine into the tissues of the eye. In an embodiment, a penetration enhancer is a topical anesthetic.
In an embodiment, an ophthalmic composition further comprises an antimicrobial preservative. In an embodiment, the antimicrobial preservative is selected from the group consisting of benzalkonium chloride, thimerosal, chlorobutanol, methyl paraben, propyl paraben, phenylethyl alcohol, EDTA, sorbic acid, Onamer M and a combination thereof. In an embodiment, the antimicrobial preservative is at a concentration of about 0.001% to 1.0% by weight in said solution.

In an embodiment, an ophthalmic composition further comprises a co-solvent/surfactant. In an embodiment, the co-solvent/surfactant is selected from the group consisting of polysorbate 20, polysorbate 60, polysorbate 80, Pluronic F-68, Pluronic F-84, Pluronic P-103, cyclodextrin, tyloxapol and a combination thereof. In an embodiment, the co-solvent/surfactant is at a concentration of about 0.01% to 2% by weight in said composition.

In an embodiment, an ophthalmic composition further comprises viscosity increasing agent. In an embodiment, the viscosity increasing agent is selected from the group consisting of polyvinyl alcohol, polyvinyl pyrrolidone, methyl cellulose, hydroxy propyl methylcellulose, hydroxyethyl cellulose, carboxymethyl cellulose, hydroxy propyl cellulose, and a combination thereof. In an embodiment, the viscosity increasing agent is at a concentration of about 0.01% to 2% by weight in said solution.

In an embodiment, an ophthalmic composition suitable for topical administration to an eye, effective for treatment and/or prophylaxis of a microorganism infection or a disorder of at least one tissue of the eye, comprises povidone-iodine in a concentration between 0.01% and 10%, and bromfenac. In an embodiment, an ophthalmic composition comprises:

0.3 to 1% (w/w) polyvinylpyrrolidinone-iodine complex;
0.05 to 2% (w/w) bromfenac;
0.005% to 0.02% (w/w) EDTA;
0.01 to 0.5% (w/w) sodium chloride;
0.02 to 0.1% (w/w) tyloxapol;
0.5% to 2% (w/w) sodium sulfate; and
0.1 to 0.5% (w/w) hydroxyethylcellulose.

In an embodiment, an ophthalmic composition is in the form of a solution, suspension, emulsion, ointment, cream, gel, or a controlled-release/sustain-release vehicle.

In an embodiment, a microorganism treated or prevented by prophylaxis using a composition encompassed herein is selected from the group consisting of bacteria, viruses, fungi, and amoebae. In an aspct, bacteria is mycobacteria.

In an embodiment, a disorder treated using an ophthalmic composition encompassed herein is selected from the group consisting of a microorganism infection of at least one tissue of the eye, conjunctivitis, corneal abrasion, ulcerative infectious keratitis, epithelial keratitis, stromal keratitis and herpesvirus-related keratitis.

In an embodiment, an ophthalmic composition is used for prophylaxis of infection following corneal abrasion or ocular surgery.

In an embodiment, an ophthalmic composition comprises:

0.3 to 1% (w/w) polyvinylpyrrolidinone-iodine complex;
0.05 to 2% (w/w) steroid;
0.005% to 0.02% (w/w) EDTA;
0.01 to 0.5% (w/w) sodium chloride;
0.02 to 0.1% (w/w) tyloxapol;
0.5% to 2% (w/w) sodium sulfate; and
0.1 to 0.5% (w/w) hydroxyethylcellulose;

wherein the steroid is selected from the group consisting of prednisolone acetate, loteprednol etabonate, difluprednate, hydrocortisone acetate, and combinations thereof.

In an embodiment, an ophthalmic composition comprises:

0.4% (w/w) polyvinylpyrrolidinone-iodine complex;
0.1% (w/w) steroid;
0.01% (w/w) EDTA;
0.3% (w/w) sodium chloride salt;
0.05% (w/w) tyloxapol;
0.2% (w/w) sodium sulfate; and
0.25% (w/w) hydroxyethylcellulose;

wherein the steroid is selected from the group consisting of prednisolone acetate, loteprednol etabonate, difluprednate, hydrocortisone acetate, and combinations thereof.

In an embodiment, an ophthalmic composition retains 95% of its polyvinylpyrrolidinone-iodine and 95% of its steroid after a period of 1 month. In an embodiment, an ophthalmic composition retains 90% of its polyvinylpyrrolidinone-iodine and 90% of its steroid after a period of 3 months. In an embodiment, an ophthalmic composition retains 90% of its polyvinylpyrrolidinone-iodine and 90% of its steroid after a period of 1 month.

In an embodiment, an ophthalmic composition retains 95% of its polyvinylpyrrolidinone-iodine and 95% of its NSAID after a period of 1 month. In an embodiment, an ophthalmic composition retains 90% of its polyvinylpyrrolidinone-iodine and 90% of its NSAID after a period of 3 months. In an embodiment, an ophthalmic composition retains 90% of its polyvinylpyrrolidinone-iodine and 90% of its NSAID after a period of 1 month.

In an embodiment, an ophthalmic composition comprising polyvinylpyrrolidinone-iodine (PVP-I) and at least one steroid retains about 89% of its PVP-I after a period of 1 month, about 90% of its PVP-I after a period of 1 month, about 91% of its PVP-I after a period of 1 month, about 92% of its PVP-I after a period of 1 month, about 93% of its PVP-I after a period of 1 month, about 94% of its PVP-I after a period of 1 month, about 94% of its PVP-I after a period of 1 month, about 95% of its PVP-I after a period of 1 month, about 96% of its PVP-I after a period of 1 month, about 97% of its PVP-I after a period of 1 month, about 98% of its PVP-I after a period of 1 month, or about 99% of its PVP-I after a period of 1 month.

In an embodiment, an ophthalmic composition comprising polyvinylpyrrolidinone-iodine (PVP-I) and at least one NSAID retains about 89% of its PVP-I after a period of 1 month, about 90% of its PVP-I after a period of 1 month, about 91% of its PVP-I after a period of 1 month, about 92% of its PVP-I after a period of 1 month, about 93% of its PVP-I after a period of 1 month, about 94% of its PVP-I after a period of 1 month, about 94% of its PVP-I after a period of 1 month, about 95% of its PVP-I after a period of 1 month, about 96% of its PVP-I after a period of 1 month, about 97% of its PVP-I after a period of 1 month, about 98% of its PVP-I after a period of 1 month, or about 99% of its PVP-I after a period of 1 month.

In an embodiment, an ophthalmic composition comprising polyvinylpyrrolidinone-iodine (PVP-I) and at least one steroid retains about 89% of its PVP-I after a period of 3 months, about 90% of its PVP-I after a period of 3 months, about 91% of its PVP-I after a period of 3 months, about 92% of its PVP-I after a period of 3 months, about 93% of its PVP-I after a period of 3 months, about 94% of its PVP-I after a period of 3 months, about 94% of its PVP-I after a period of 3 months, about 95% of its PVP-I after a period of 3 months, about 96% of its PVP-I after a period of 3 months, about 97% of its PVP-I after a period of 3 months, about 98% of its PVP-I after a period of 3 months, or about 99% of its PVP-I after a period of 3 months.

In an embodiment, an ophthalmic composition comprising polyvinylpyrrolidinone-iodine (PVP-I) and at least one NSAID retains about 89% of its PVP-I after a period of 3 months, about 90% of its PVP-I after a period of 3 months, about 91 % of its PVP-I after a period of 3 months, about 92% of its PVP-I after a period of 3 months, about 93% of its PVP-I after a period of 3 months, about 94% of its PVP-I after a period of 3 months, about 94% of its PVP-I after a period of 3 months, about 95% of its PVP-I after a period of 3 months, about 96% of its PVP-I after a period of 3 months, about 97% of its PVP-I after a period of 3 months, about 98% of its PVP-I after a period of 3 months, or about 99% of its PVP-I after a period of 3 months.

In an embodiment, an ophthalmic composition comprising PVP-I and at least one steroid retains about 89% of its at least one steroid after a period of 1 month, about 90% of its at least one steroid after a period of 1 month, about 91% of its at least one steroid after a period of 1 month, about 92% of its at least one steroid after a period of 1 month, about 93% of its at least one steroid after a period of 1 month, about 94% of its at least one steroid after a period of 1 month, about 94% of its at least one steroid after a period of 1 month, about 95% of its at least one steroid after a period of 1 month, about 96% of its at least one steroid after a period of 1 month, about 97% of its at least one steroid after a period of 1 month, about 98% of its at least one steroid after a period of 1 month, or about 99% of its at least one steroid after a period of 1 month.

In an embodiment, an ophthalmic composition comprising PVP-I and at least one NSAID retains about 89% of its at least one NSAID after a period of 1 month, about 90% of its at least one NSAID after a period of 1 month, about 91% of its at least one NSAID after a period of 1 month, about 92% of its at least one NSAID after a period of 1 month, about 93% of its at least one NSAID after a period of 1 month, about 94% of its at least one NSAID after a period of 1 month, about 94% of its at least one NSAID after a period of 1 month, about 95% of its at least one NSAID after a period of 1 month, about 96% of its at least one NSAID after a period of 1 month, about 97% of its at least one NSAID after a period of 1 month, about 98% of its at least one NSAID after a period of 1 month, or about 99% of its at least one NSAID after a period of 1 month.

In an embodiment, an ophthalmic composition comprising PVP-I and at least one steroid retains about 89% of its at least one steroid after a period of 3 months, about 90% of its at least one steroid after a period of 3 months, about 91 % of its at least one steroid after a period of 3 months, about 92% of its at least one steroid after a period of 3 months, about 93% of its at least one steroid after a period of 3 months, about 94% of its at least one steroid after a period of 3 months, about 94% of its at least one steroid after a period of 3 months, about 95% of its at least one steroid after a period of 3 months, about 96% of its at least one steroid after a period of 3 months, about 97% of its at least one steroid

after a period of 3 months, about 98% of its at least one steroid after a period of 3 months, or about 99% of its at least one steroid after a period of 3 months.

In an embodiment, an ophthalmic composition comprising PVP-I and at least one NSAID retains about 89% of its at least one NSAID after a period of 3 months, about 90% of its at least one NSAID after a period of 3 months, about 91% of its at least one NSAID after a period of 3 months, about 92% of its at least one NSAID after a period of 3 months, about 93% of its at least one NSAID after a period of 3 months, about 94% of its at least one NSAID after a period of 3 months, about 94% of its at least one NSAID after a period of 3 months, about 95% of its at least one NSAID after a period of 3 months, about 96% of its at least one NSAID after a period of 3 months, about 97% of its at least one NSAID after a period of 3 months, about 98% of its at least one NSAID after a period of 3 months, or about 99% of its at least one NSAID after a period of 3 months.

In an embodiment, an ophthalmic composition is an aqueous solution.

In an embodiment of the disclosure, a method is provided for treating and/or prophylaxis of an eye disorder or a microorganism infection of at least one tissue of the eye comprising the step of administering one of more doses of an ophthalmic composition encompassed herein to the eye. In an embodiment, the prophylaxis is prophylaxis of infection following corneal abrasion or ocular surgery. In an embodiment, the eye disorder is selected from the group consisting of a microorganism infection of at least one tissue of the eye, conjunctivitis, corneal abrasion, ulcerative infectious keratitis, epithelial keratitis, stromal keratitis and herpesvirus-related keratitis. In an embodiment, the microorganism is a bacteria, virus, fungi, or amoebae. In an embodiment, the bacteria is mycobacteria.

In an embodiment, in a method of treatment of the disclosure, the sum of the povidone-iodine and the steroid is between 0.001 mg to 5 mg per dose. In an embodiment, in a method of treatment of the disclosure, each dose is between 10 microliters to 200 microliters. In an embodiment in a method of treatment of the disclosure, each dose is between 50 microliters to 80 microliters. In an embodiment, in a method of treatment of the disclosure, the administering step comprises administering a composition encompassed herein to an eye one to four times a day. In an embodiment, in a method of treatment of the disclosure, the administering step comprises administering a composition encompassed herein to an eye one to twenty-four times a day. In an embodiment, in a method of treatment of the disclosure, the method includes storing the composition for at least one month, at least three months, at least six months, or at least 1 year before the administration step.

BRIEF DESCRIPTION OF THE DRAWINGS

Figure 1 is an image depicting the HPLC-UV/(+)ESI-MS and MS/MS spectral data of dexamethasone phosphate.
Figure 2 is an image depicting the HPLC-UV/(+)ESI-MS and MS/MS spectral data of prednisolone acetate.
Figure 3 is an image depicting the HPLC-UV/(+)ESI-MS and MS/MS spectral data of loteprednol etabonate.
Figure 4 is an image depicting the HPLC-UV/(+)ESI-MS and MS/MS spectral data of difluprednate.
Figure 5 is an image depicting the HPLC/UV chromatograms of PVP-I at the concentration of 200 μg/mL for dexamethasone sodium phosphate.
Figure 6 is an image depicting the HPLC/UV chromatograms of dexamethasone sodium phosphate in PVP-I for Day 0.
Figure 7 is an image depicting the HPLC/UV chromatograms of dexamethasone sodium phosphate.
Figure 8 is an image depicting the HPLC/UV chromatograms of dexamethasone sodium phosphate in PVP-I for two weeks.
Figure 9 is an image depicting the HPLC/UV chromatograms of dexamethasone sodium phosphate in PVP-I for two weeks.
Figure 10 is an image depicting the HPLC/UV chromatograms of dexamethasone sodium phosphate in PVP-I for one month.
Figure 11 is an image depicting the HPLC/UV chromatograms of dexamethasone sodium phosphate in PVP-I for one month.
Figure 12 is an image depicting the HPLC/UV chromatograms (expanded) of dexamethasone sodium phosphate in PVP-I for one month.
Figure 13 is an image depicting the HPLC/UV chromatograms (expanded) of dexamethasone sodium phosphate in PVP-I for one month.
Figure 14 is an image depicting the mass ion chromatograms (MRM Mode) of dexamethasone sodium phosphate in reference standard samples.
Figure 15 is an image depicting the mass ion chromatograms (MRM Mode) of dexamethasone sodium phosphate in one month room temperature stability sample in the presence of PVP-I.
Figure 16 is an image depicting the mass ion chromatograms (MRM Mode) of dexamethasone sodium phosphate in one month 40°C stability sample in the presence of PVP-I.

Figure 17 is an image depicting the HPLC/UV chromatograms of PVP-I at the concentration of 20 μg/mL for prednisolone acetate.

Figure 18 is an image depicting the HPLC/UV chromatograms of prednisolone acetate in PVP-I for Day 0.

Figure 19 is an image depicting the HPLC/UV chromatograms of prednisolone acetate in PVP-I for Day 0.

Figure 20 is an image depicting the HPLC/UV chromatograms of prednisolone acetate in PVP-I for two weeks.

Figure 21 is an image depicting the HPLC/UV chromatograms of prednisolone acetate in PVP-I for two weeks.

Figure 22 is an image depicting the HPLC/UV chromatograms of prednisolone acetate in PVP-I for one month.

Figure 23 is an image depicting the HPLC/UV chromatograms of prednisolone acetate in PVP-I for one month.

Figure 24 is an image depicting the mass ion chromatograms (MRM Mode) of prednisolone acetate in reference standard samples.

Figure 25 is an image depicting the mass ion chromatograms (MRM Mode) of prednisolone acetate in one month room temperature stability sample in the presence of PVP-I.

Figure 26 is an image depicting the mass ion chromatograms (MRM Mode) of prednisolone acetate in one month 40°C stability sample in the presence of PVP-I.

Figure 27 is an image depicting the HPLC/UV chromatograms of PVP-I at the concentration of 40 μg/mL for loteprednol etabonate.

Figure 28 is an image depicting the HPLC/UV chromatograms of loteprednol etabonate in PVP-I for Day 0.

Figure 29 is an image depicting the HPLC/UV chromatograms of loteprednol etabonate in PVP-I for Day 0.

Figure 30 is an image depicting the HPLC/UV chromatograms of loteprednol etabonate in PVP-I for two weeks.

Figure 31 is an image depicting the HPLC/UV chromatograms of loteprednol etabonate in PVP-I for two weeks.

Figure 32 is an image depicting the HPLC/UV chromatograms of loteprednol etabonate in PVP-I for one month.

Figure 33 is an image depicting the HPLC/UV chromatograms of loteprednol etabonate in PVP-I for one month.

Figure 34 is an image depicting the mass ion chromatograms (MRM Mode) of loteprednol etabonate in reference standard samples.

Figure 35 is an image depicting the mass ion chromatograms (MRM Mode) of loteprednol etabonate in one month room temperature stability sample in the presence of PVP-I.

Figure 36 is an image depicting the mass ion chromatograms (MRM Mode) of loteprednol etabonate in one month 40°C stability sample in the presence of PVP-I.

Figure 37 is an image depicting the HPLC/UV chromatograms of PVP-I at the concentration of 400 μg/mL for difluprednate.

Figure 38 is an image depicting the HPLC/UV chromatograms of difluprednate in PVP-I for Day 0.

Figure 39 is an image depicting the HPLC/UV chromatograms of difluprednate in PVP-I for Day 0.

Figure 40 is an image depicting the HPLC/UV chromatograms of difluprednate in PVP-I for two weeks.

Figure 41 is an image depicting the HPLC/UV chromatograms of difluprednate in PVP-I for two weeks.

Figure 42 is an image depicting the HPLC/UV chromatograms of difluprednate in PVP-I for one month.

Figure 43 is an image depicting the HPLC/UV chromatograms of difluprednate in PVP-I for one month.

Figure 44 is an image depicting the mass ion chromatograms (MRM Mode) of difluprednate in reference standard samples.

Figure 45 is an image depicting the mass ion chromatograms (MRM Mode) of difluprednate in one month room temperature stability sample in the presence of PVP-I.

Figure 46 is an image depicting the mass ion chromatograms (MRM Mode) of difluprednate in one month 40°C stability sample in the presence of PVP-I.

## DETAILED DESCRIPTION

It is known that iodine, including preparations of PVP-I, reacts chemically with various steroids when combined with a steroid, resulting in an unstable composition, due in part to reactivity of the iodine with the steroid. U.S. Patent 3,886,268 demonstrates the well-known instability of steroid-iodine combinations. It is also known that certain non-steroidal antiinflammatory compounds ("NSAIDS") also react with iodine. However, U.S. Patent 7,767,217 illustrates that under certain specific conditions, dexamethasone, for example, can be combined with PVP-I to form an effective antimicrobial- steroid pharmaceutical composition.

### Compositions

In an embodiment, compositions disclosed herein comprise PVP-I and a steroid. In an embodiment, compositions disclosed herein comprise PVP-I and an NSAID. In another embodiment, a composition disclosed herein is a pharmaceutical composition. In another embodiment, a composition disclosed herein is an ophthalmic composition.

The disclosure provides, in part, compositions comprising PVP-I in the range of about 0.01% to about 10%

(weight/weight or weight/volume) and a steroid at a concentration of about 0.001% to about 10%. The disclosure also provides, in part, ophthalmic compositions comprising povidone-iodine in the range of about 0.01% to about 10% (weight/weight or weight/volume) and a therapeutically effective amount of a steroid at a concentration of about 0.001% to about
10%. The disclosure provides, in part, compositions comprising PVP-I in the range of about 0.01% to about 10% (weight/weight or weight/volume) and an NSAID at a concentration of about 0.001% to about 10%. The disclosure also provides, in part, ophthalmic compositions comprising povidone-iodine in the range of about 0.01% to about 10% (weight/weight or weight/volume) and a therapeutically effective amount of an NSAID at a concentration of about 0.001% to about 10%.

The affinity of free iodine for reaction with --OH, --SH and --NH functional groups is well described in the literature and forms the basis for the anti-microbial activity of iodine-containing solutions (Rackur H.J. Hosp. Infect., 1985; 6: 13-23, and references therein).

Dexamethasone, (9-Fluoro-11.beta., 17, 21-trihydroxy-16.alpha.-methylpregna- 1,4-diene-3,20-dione) for example, contains three such moieties (--OH) at the 11, 17 and 21 positions. The skilled artisan would conclude that these hydroxyl groups would be prone to covalent substitution reactions by the free iodine generated in the solution equilibrium reaction described above for PVP-I.

In preparing the present compositions, experiments of combinations of various steroids and PVP-I, as well as combinations of various NSAIDS and PVP-I, were performed. It was observed that many formulations were unsuccessful because of the rapid reaction between PVP-I and the added steroid. It was surprising to discover that separate solutions of PVP-I and prednisolone acetate, PVP-1 and loteprednol etabonate, PVP-1 and hydrocortisone acetate, and PVP-1 and difluprednate demonstrate unexpected stability, based on what was previously known in the art. It was also surprising to discover that solutions of PVP-I and bromfenac demonstrate unexpected stability, based on what was previously known in the art. In an embodiment, a combination of PVP-I and one of the steroids or NSAIDS identified above each remains stable for a month or longer.

In an embodiment, a composition comprises PVP-I and prednisolone acetate. In another embodiment, a composition is a pharmaceutical composition comprising PVP-I and prednisolone acetate. In another embodiment, a composition is an ophthalmic preparation comprising PVP-I and prednisolone acetate.

In an embodiment, a composition comprises PVP-I and loteprednol etabonate. In another embodiment, a composition is a pharmaceutical composition comprising PVP-I and loteprednol etabonate. In another embodiment, a composition is an ophthalmic preparation comprising PVP-I and loteprednol etabonate.

In an embodiment, a composition comprises PVP-I and hydrocortisone acetate. In another embodiment, a composition is a pharmaceutical composition comprising PVP-I and hydrocortisone acetate. In another embodiment, a composition is an ophthalmic preparation comprising PVP-I and hydrocortisone acetate.

In an embodiment, a composition comprises PVP-I and difluprednate. In another embodiment, a composition is a pharmaceutical composition comprising PVP-I and difluprednate. In another embodiment, a composition is an ophthalmic preparation comprising PVP-I and difluprednate.

In an embodiment, a composition comprises PVP-I and bromfenac. In another embodiment, a composition is a pharmaceutical composition comprising PVP-I and bromfenac. In another embodiment, a composition is an ophthalmic preparation comprising PVP-I and bromfenac.

Percentages for components of compositions are provided herein as weight/weight (w/w), unless otherwise indicated. For example, 0.6% PVP-I indicates 0.6% PVP-I by weight, with respect to the total weight of 100% for a composition.

In an embodiment, a composition comprises povidone-iodine (PVP-I) at a concentration in the range of about 0.1% to about 2.5%. In another embodiment, a composition comprises povidone-iodine (PVP-I) at a concentration in the range between 0.2 and 1.5%, and in yet another embodiment, between 0.3% and 1.0%. In an embodiment, a composition comprises PVP-I at a concentration in the range of about 0.2 to about 2.0%, about 0.3% to about 1.5%, about 0.36% to about 1.0%, and about 0.4% to about 0.75%. In an embodiment, a composition comprises PVP-I at a concentration of about 0.05%, about 0.1%, about 0.2%, about 0.3%, about 0.4%, about 0.5%, about 0.6%, about 0.7%, about 0.8%, about 0.9% or about 1.0%. In an embodiment, a composition comprises povidone-iodine PVP-I at a concentration of 0.05%, 0.1%, 0.15%, 0.2%, 0.25%, 0.3%, 0.35%, 0.4%, 0.45%, 0.5%, 0.55%, 0.6%, 0.65%, 0.7%, 0.75%, 0.8%, 0.85%, 0.9%, 0.95%, or 1.0%. In another embodiment, a composition comprises PVP-I at a concentration of about 2%, about 3%, about 4%, about 5%, about 6%, about 7%, about 8%, about 9% or about 10%. In another embodiment, a composition comprises PVP-I at a concentration of about 2% or less, about 3% or less, about 4% or less, about 5% or less, about 6% or less, about 7% or less, about 8% or less, about 9% or less or about 10% or less. In another embodiment, a composition comprises PVP-I at a concentration of about 1% or more, about 2% or more, about 3% or more, about 4% or more, about 5% or more, about 6% or more, about 7% or more, about 8% or more, about 9% or more or about 10% or more. In another embodiment, a composition comprises PVP-I at a concentration of 2%, 3%, 4%, 5%, 6%, 7%, 8%, 9% or 10%.

Compositions disclosed herein may comprise one or more steroids. Steroids include, but are not limited to, dexamethasone, dexamethasone alcohol, dexamethasone sodium phosphate, flurometholone acetate, fluormethalone acetate, flurometholone alcohol, lotoprednol etabonate, medrysone, prednisolone acetate, prednisolone sodium phosphate, difluprednate, rimexolone, hydrocortisone, hydrocortisone acetate, lodoxamide tromethamine, and any combinations thereof. In an embodiment, a steroid is present in the composition at a level of about 0.001% to about 10%. In an embodiment, a steroid is present in the composition or preparation at a level of 0.001%, 0.002%, 0.003%, 0.004%, 0.005%, 0.006%, 0.007%, 0.008%, 0.009%, 0.01%, 0.02%, 0.03%, 0.04%, 0.05%, 0.06%, 0.07%, 0.08%, 0.09%, 0.1%, 0.2%, 0.3%, 0.4%, 0.5%, 0.6%, 0.7%, 0.8%, 0.9%, 1.0%, 1.1%, 1.2%, 1.3%, 1.4%, 1.5%, 1.6%, 1.7%, 1.8%, 1.9%, or 2.0%. In an embodiment, a steroid is present in the composition or preparation at a level of about 0.001%, about 0.002%, about 0.003%, about 0.004%, about 0.005%, about 0.006%, about 0.007%, about 0.008%, about 0.009%, about 0.01%, about 0.02%, about 0.03%, about 0.04%, about 0.05%, about 0.06%, about 0.07%, about 0.08%, about 0.09%, about 0.1%, about 0.2%, about 0.3%, about 0.4%, about 0.5%, about 0.6%, about 0.7%, about 0.8%, about 0.9%, about 1.0%, about 1.1%, about 1.2%, about 1.3%, about 1.4%, about 1.5%, about 1.6%, about 1.7%, about 1.8%, about 1.9%, or about 2.0%. In an embodiment, a steroid is present in the composition or preparation at a level of about 0.001% or less, about 0.002% or less, about 0.003% or less, about 0.004% or less, about 0.005% or less, about 0.006% or less, about 0.007% or less, about 0.008% or less, about 0.009% or less, about 0.01% or less, about 0.02% or less, about 0.03% or less, about 0.04% or less, about 0.05% or less, about 0.06% or less, about 0.07% or less, about 0.08% or less, about 0.09% or less, about 0.1% or less, about 0.2% or less, about 0.3% or less, about 0.4% or less, about 0.5% or less, about 0.6% or less, about 0.7% or less, about 0.8% or less, about 0.9% or less, about 1.0% or less, about 1.1% or less, about 1.2% or less, about 1.3% or less, about 1.4% or less, about 1.5% or less, about 1.6% or less, about 1.7% or less, about 1.8% or less, about 1.9% or less, or about 2.0% or less. In an embodiment, a steroid is present in the composition or preparation at a level of about 0.001% or more, about 0.002% or more, about 0.003% or more, about 0.004% or more, about 0.005% or more, about 0.006% or more, about 0.007% or more, about 0.008% or more, about 0.009% or more, about 0.01% or more, about 0.02% or more, about 0.03% or more, about 0.04% or more, about 0.05% or more, about 0.06% or more, about 0.07% or more, about 0.08% or more, about 0.09% or more, about 0.1% or more, about 0.2% or more, about 0.3% or more, about 0.4% or more, about 0.5% or more, about 0.6% or more, about 0.7% or more, about 0.8% or more, about 0.9% or more, about 1.0% or more, about 1.1% or more, about 1.2% or more, about 1.3% or more, about 1.4% or more, about 1.5% or more, about 1.6% or more, about 1.7% or more, about 1.8% or more, about 1.9% or more, or about 2.0% or more.

Compositions disclosed herein may comprise one or more NSAIDS. NSAIDS include, but are not limited to, bromfenac, ketorolac, nepafenac, ketotifen fumarate, diclofenac sodium, flurbiprofen sodium, ketorlac tromethamine, suprofen, celecoxib, naproxen, rofecoxib, and any combinations thereof. In an embodiment, an NSAID is present in the composition at a level of about 0.001% to about 10%. In an embodiment, an NSAID is present in the composition or preparation at a level of 0.001%, 0.002%, 0.003%, 0.004%, 0.005%, 0.006%, 0.007%, 0.008%, 0.009%, 0.01%, 0.02%, 0.03%, 0.04%, 0.05%, 0.06%, 0.07%, 0.08%, 0.09%, 0.1%, 0.2%, 0.3%, 0.4%, 0.5%, 0.6%, 0.7%, 0.8%, 0.9%, 1.0%, 1.1%, 1.2%, 1.3%, 1.4%, 1.5%, 1.6%, 1.7%, 1.8%, 1.9%, or 2.0%. In an embodiment, an NSAID is present in the composition or preparation at a level of about 0.001%, about 0.002%, about 0.003%, about 0.004%, about 0.005%, about 0.006%, about 0.007%, about 0.008%, about 0.009%, about 0.01%, about 0.02%, about 0.03%, about 0.04%, about 0.05%, about 0.06%, about 0.07%, about 0.08%, about 0.09%, about 0.1%, about 0.2%, about 0.3%, about 0.4%, about 0.5%, about 0.6%, about 0.7%, about 0.8%, about 0.9%, about 1.0%, about 1.1%, about 1.2%, about 1.3%, about 1.4%, about 1.5%, about 1.6%, about 1.7%, about 1.8%, about 1.9%, or about 2.0%. In an embodiment, an NSAID is present in the composition or preparation at a level of about 0.001% or less, about 0.002% or less, about 0.003% or less, about 0.004% or less, about 0.005% or less, about 0.006% or less, about 0.007% or less, about 0.008% or less, about 0.009% or less, about 0.01% or less, about 0.02% or less, about 0.03% or less, about 0.04% or less, about 0.05% or less, about 0.06% or less, about 0.07% or less, about 0.08% or less, about 0.09% or less, about 0.1% or less, about 0.2% or less, about 0.3% or less, about 0.4% or less, about 0.5% or less, about 0.6% or less, about 0.7% or less, about 0.8% or less, about 0.9% or less, about 1.0% or less, about 1.1% or less, about 1.2% or less, about 1.3% or less, about 1.4% or less, about 1.5% or less, about 1.6% or less, about 1.7% or less, about 1.8% or less, about 1.9% or less, or about 2.0% or less. In an embodiment, an NSAID is present in the composition or preparation at a level of about 0.001% or more, about 0.002% or more, about 0.003% or more, about 0.004% or more, about 0.005% or more, about 0.006% or more, about 0.007% or more, about 0.008% or more, about 0.009% or more, about 0.01% or more, about 0.02% or more, about 0.03% or more, about 0.04% or more, about 0.05% or more, about 0.06% or more, about 0.07% or more, about 0.08% or more, about 0.09% or more, about 0.1% or more, about 0.2% or more, about 0.3% or more, about 0.4% or more, about 0.5% or more, about 0.6% or more, about 0.7% or more, about 0.8% or more, about 0.9% or more, about 1.0% or more, about 1.1% or more, about 1.2% or more, about 1.3% or more, about 1.4% or more, about 1.5% or more, about 1.6% or more, about 1.7% or more, about 1.8% or more, about 1.9% or more, or about 2.0% or more.

The compositions disclosed herein can be administered as solutions, suspensions, emulsions (dispersions), gels, creams, or ointments in a suitable ophthalmic vehicle. In any of the compositions of this disclosure for topical

administration, such as topical administration to the eye, the mixtures are preferably formulated as aqueous solutions at a pH of 3.5 to 6.5. Preferentially the pH is adjusted to between 4 and 5. This pH range may be achieved by the addition of acids/bases to the solution.

In an embodiment, an ophthalmic composition may comprise an optional co-solvent. In another embodiment, the solubility of the components of the present compositions may be enhanced by a surfactant or other appropriate co-solvent in the composition. Such co-solvents or surfactants include polysorbate -20, -60, and -80, a polyoxyethylene/polyoxypropylene surfactant (e.g. Pluronic F-68, F-84 and P-103), cyclodextrin, tyloxapol, PEG 35 Castor oil (Cremophor EL), polyoxyl 40 Stearate (Myrj 52), other agents known to those skilled in the art, or a combination thereof. Typically, such co-solvents are present at a level of from about 0.01% to about 2% by weight. In an embodiment, a co-solvent is present at a level of about 0.01%, about 0.02%, about 0.03%, about 0.04%, about 0.05%, about 0.06%, about 0.07%, about 0.08%, about 0.09%, about 0.1%, about 0.2%, about 0.3%, about 0.4%, about 0.5%, about 0.6%, about 0.7%, about 0.8%, about 0.9%, about 1.0%, about 1.1%, about 1.2%, about 1.3%, about 1.4%, about 1.5%, about 1.6%, about 1.7%, about 1.8%, about 1.9%, or about 2.0%.

In an embodiment, a composition may comprise an optional agent that can increase viscosity. As will be understood by the skilled artisan when armed with the present disclosure, it may be desirable to increase viscosity above that of a simple aqueous solution in order to increase ocular absorption of the active compound, to decrease variability in dispensing the formulation, to decrease physical separation of components of a suspension or emulsion of the formulation and/or to otherwise improve the ophthalmic formulation. Such viscosity-enhancing agents include, but are not limited to, polyvinyl alcohol, polyvinyl pyrrolidone, methyl cellulose, hydroxypropylmethyl cellulose, hydroxyethyl cellulose, carboxymethyl cellulose, hydroxypropyl cellulose, other agents known to those skilled in the art, or any combination thereof. Such agents are typically employed at a level of from about 0.01% to about 2% by weight. In an embodiment, such optional agents are present at about 0.01%, about 0.02%, about 0.03%, about 0.04%, about 0.05%, about 0.06%, about 0.07%, about 0.08%, about 0.09%, about 0.1%, about 0.2%, about 0.3%, about 0.4%, about 0.5%, about 0.6%, about 0.7%, about 0.8%, about 0.9%, about 1.0%, about 1.1%, about 1.2%, about 1.3%, about 1.4%, about 1.5%, about 1.6%, about 1.7%, about 1.8%, about 1.9%, or about 2.0%.

In another aspect, bioadhesive agents may comprise the compositions, in order to increase the retention time of the drug gradient over a biological substrate. The bioadhesive agents include, but are not limited to, polyvinylpyrrolidone (PVP), xanthan gum, locust bean gum, acacia gum, hydroxypropyl methylcellulose (HPMC), sodium alginate, pectin, gelatin, carbomer, polyvinylalcohol, gellan gum, tragacanth, acacia, and sodium carboxymethyl cellulose, as well as other agents known to those skilled in the art, or any combination thereof. In yet another embodiment, compositions of the invention may comprise viscoelastic agents such as methyl cellulose, carboxymethyl cellulose, hydroxyethyl cellulose, polyvinyl alcohol, dextran, chondroitin sulfate and salts thereof, and hyaluronic acid and salts thereof.

In an embodiment, an ophthalmic composition may further comprise one or more of (1) a penetration enhancer which enhances the penetration of povidone-iodine into the tissues of the eye (this may be a topical anesthetic) (2) a co-solvent or a nonionic surface agent - surfactant, which, for example, may be about 0.01% to 2% by weight; (3) a viscosity increasing agent, which, for example, may be about 0.01% to 2% by weight; and (4) a suitable ophthalmic vehicle.

The ophthalmic composition may be in the form of a solution, a suspension, an emulsion, a preparation, an ointment, a cream, a gel, or a controlled-release/sustain-release vehicle. By way of a non-limiting example, the composition may be in the form of a contact lens solution, eyewash, eyedrop, and the like.

## Methods

In an embodiment, compositions disclosed herein are useful for preparation of and use as pharmaceutical compositions. In another embodiment, compositions disclosed herein are useful for preparation of and use as compositions other than pharmaceutical compositions.

In an embodiment, compositions disclosed herein are useful for preparation of and use as ophthalmic compositions. In an aspect, a composition of the invention is useful in the treatment of infections of the conjunctiva and cornea. In another aspect, the broad spectrum antimicrobial activity of povidone-iodine enables a composition of the invention to be used to treat ocular conjunctival or corneal infection caused by mycobacteria, viruses, fungi, and amoeba. Additionally the composition is useful in the infectious prophylaxis of patients recovering from ophthalmic surgery.

In an embodiment, an ophthalmic composition is provided that is suitable for topical administration to an eye, effective for treatment and/or prophylaxis of a microorganism infection or a disorder of at least one tissue of the eye. Prophylaxis may be, for example, prophylaxis from infection following surgery, prophylaxis from infection after birth for the newborn, or prophylaxis from accidental contact with contaminating material. Accidental contact with contaminating material may occur, for example, during surgery or during food processing.

In an aspect, the ophthalmic composition may be used for treatment and/or prophylaxis of a microorganism infection. The microorganism may be a bacterium, a virus, a fungus, or an amoeba, a parasite, or a combination thereof. In an embodiment, the bacteria may be a mycobacterium.

In an aspect, an ophthalmic composition may be used to treat a disorder such as, but not limited to, conjunctivitis, corneal abrasion, ulcerative infectious keratitis, epithelial keratitis, stromal keratitis, herpesvirus-related keratitis, ocular surface irregularity, tear deficiency, dry syndrome, meibomian gland dysfunction, blepharitis and uveitis. In another aspect, an ophthalmic composition may be used for prophylaxis of disorders such as conjunctivitis, corneal abrasion, ulcerative infectious keratitis, epithelial keratitis, stromal keratitis, herpesvirus-related keratitis, ocular surface irregularity, tear deficiency, dry syndrome, meibomian gland dysfunction, blepharitis and uveitis.

In another embodiment, the disclosure is directed to a method for treating and/or prophylaxis of an eye disorder or a microorganism infection of at least one tissue of the eye comprising the step of administering one of more doses of an ophthalmic composition, discussed above, to the eye. The eye disorder may be, for example, a microorganism infection of at least one tissue of the eye, conjunctivitis, corneal abrasion, ulcerative infectious keratitis, epithelial keratitis, stromal keratitis, herpes virus-related keratitis, ocular surface irregularity, tear deficiency, dry syndrome, meibomian gland dysfunction, and blepharitis. The microorganism may be bacteria (e.g., mycobacteria), virus, fungi, or amoebae.

In an embodiment, the dose volume administered to a subject may be between about 10 microliters and about 200 microliters, in another embodiment, between about 20 microliters and 100 microliters, and in another embodiment, between about 50 microliters and about 80 microliters, or about one drop per eye. Two or more drops may be added to an eye. Treatment of an eye may be effected by adding a single drop of composition disclosed herein, or by adding two or more drops, as required to achieve the desired result.

In an embodiment, administration frequency may be between 1 and 24 times a day. In an embodiment, administration frequency may be between 1 and 48 times a day. In another embodiment, administration frequency may be between 2 and 24 times a day. In another embodiment, administration frequency may be between 2 and 4 times a day. In another embodiment, administration frequency may be twice a day. In another embodiment, administration frequency may be once a day. In another embodiment, administration frequency may be less frequent than once a day. In another embodiment, administration frequency may be on demand, as therapeutic treatment is required or desired. In another embodiment, administration frequency may be 1, 2, 3, 4, 5, 6, 7, 8, 9, 10, 11, 12, 13, 14, 15, 16, 17, 18, 19, 20, 21, 22, 23, 24, 48, or 96 times a day.

In an embodiment, a composition disclosed herein is used for prophylaxis and/or treatment of a non-ophthalmic tissue by contacting the tissue with the composition.

The invention is further described by the following examples. Example 1 and part of Example 2 are reference examples. It should be recognized that variations based on the inventive features are within the skill of the ordinary artisan, and that the scope of the invention should not be limited by the examples. To properly determine the scope of the present disclosure, an interested party should consider the claims herein

EXAMPLES

Example 1: Stability Testing For Steroids Combined With Povidone Iodine

The objective of this study was to determine whether povidone iodine (PVP-1) at the concentration of 4 mg/mL (0.4%) reacts with any of four different steroids (dexamethasone sodium phosphate, prednisolone acetate, loteprednol etabonate, and difluprednate), the active ingredients, in pharmaceutical formulations under both room temperature and 40 °C for a time period of one month.

Dexamethasone sodium phosphate ophthalmic solution (USP, 0.1%) from Alcon Laboratories, prednisolone acetate ophthalmic suspension (USP, 1%) from Alcon Laboratories, loteprednol etabonate ophthalmic suspension (0.5%) from Baush & Lomb, and difluprednate ophthalmic emulsion (0.05%) from Sirion Therapeutics were used for this study. PVP-I was prepared in water at the concentration of 100 mg/mL (10%). One milliliter of the solution, suspension, or emulsion was mixed with 40 μL of 10% PVP-I in 1.5 mL amber glass vials, followed by storage under both room temperature and 40 °C for 2 weeks and one month. The resultant samples in the presence of PVP-I were analyzed using HPLC. The four steroid levels were measured against the reference standard samples stored under room temperature in the absence of PVP-I (0.4%). The One Month stability test samples were analyzed with the reference standard sample using LC-MS/MS Method in MRM mode with three characteristic ion transitions to confirm the identity of four steroids in stability testing samples. The presence of each of the four steroids in the respective pharmaceutical formulations tested was confirmed by LC/UV-MS and MS/MS. Thus, the four pharmaceutical formulations can be used in the study.

After storage under room temperature and 40 °C at the presence of PVP-I (0.4%), the levels of dexamethasone phosphate in two week samples were only 83.04% and 84.57% of those in room temperature and 40 °C Day 0 samples, respectively. The respective data are 84.24% and 84.09% for one month testing, indicating that dexamethasone phosphate was not stable in the presence of PVP-I (0.4%) under the current testing conditions. Three degradation products (D1, D2, and D3) were observed.

After storage under room temperature and 40 °C in the presence of PVP-I (0.4%), the levels of prednisolone acetate in two week testing samples were 99.24% and 96.60% of those in room temperature and 40 °C Day 0 samples,

respectively. The respective data are 95.66% and 96.79% for one month testing. Identical mass ion chromatograms and same intensities of mass ion response were observed in the reference standard and one month stability testing samples. The results from both HPLC/UV and LC-MS/MS analysis indicate that prednisolone acetate was stable in the presence of PVP-I (0.4%) under the current testing conditions.

After storage under room temperature and 40 °C in the presence of PVP-I (0.4%), the levels of loteprednol etabonate in two week testing samples were 101.43% and 100.07% of those in room temperature and 40 °C Day 0 samples, respectively. The respective data are 100.72% and 96.02% for one month testing. Identical mass ion chromatograms and same intensities of mass ion response were observed in the reference standard and one month stability testing samples. The results from both HPLC/UV and LC-MS/MS analysis indicate that loteprednol etabonate was stable in the presence of PVP-I (0.4%) under the current testing conditions.

After storage under room temperature and 40 °C in the presence of PVP-I (0.4%), the levels of difluprednate in two week testing samples were 103.23% and 99.30% of those in room temperature and 40 °C Day 0 samples, respectively. The respective data are 104.47% and 100.24% for one month testing. Identical mass ion chromatograms and same intensities of mass ion response were observed in the reference standard and one month stability testing samples. The results from both HPLC/UV and LC-MS/MS analysis indicate that difluprednate was stable in the presence of PVP-I (0.4%) under the current testing conditions.

## 1. MATERIALS

### 1.1 Test Pharmaceutical Formulations

The four steroids and their related pharmaceutical formulations are listed in Table I and Table II.

### 1.2 Povidone Iodine

Povidone iodine (USP) was obtained from Spectrum Chemicals. Lot No. and expiration date are YQ0429 and January 31, 2011, respectively.

### 1.3 Solvents, Reagents, and Supplies

OmniSolv® Water was obtained from EM Science. Acetonitrile, methanol, and ammonium acetate were purchased from Sigma-Aldrich.

### 1.4 Suppliers and Equipment

#### 1.4.1 Supplies

- Serological Pipettes, Kimble Glass Inc
- Wiretrol Micropipettes, Drummond® Scientific Company
- Autosampler Vials, Sun International
- Automatic Pipettes, Gilson

#### 1.4.2 Equipment

- Sartorius Balances, BP301S, Sartorius Corporation

## 2. METHODS

### 2.1 Preparation of Stability Test Samples

#### 2.1.1 Preparation of PVP-I Solution (10%, 100mg/mL)

Weigh 1 g of PVP-I and dissolve in 10 mL of water.

2.1.2 Preparation of Stability Test Samples

2.1.2.1 Preparation of Dexamethasone Sodium Phosphate Stability Test Samples

Aliquot 1 mL of ophthalmic solution (USP, 0.1%) into eight amber HPLC vials to give the following samples: Dexamethasone Sodium Phosphate-1, 2, 3, 4, 5, 6, 7, 8, and 9.

Added 40 μL of PVP-I stock solution (10%) into Dexamethasone Sodium Phosphate-3, 4, 5, and 6, and mixed well to give the following samples:

Dexamethasone Sodium Phosphate+ PVP-I-3, 4, 5, and 6
Store Dexamethasone Sodium Phosphate+PVP-I-3 and 4 on the lab bench at room temperature and store Dexamethasone Sodium Phosphate+PVP-I-5 and 6 in a stability test chamber at 40 °C.

Added 40 μL of water into Dexamethasone Sodium Phosphate-7, 8, and 9, and mixed well to give the following samples:

Dexamethasone Sodium Phosphate+$H_2O$-7, 8, and 9
Stored Dexamethasone Sodium Phosphate+$H_2O$-9 on the lab bench at room temperature and store Dexamethasone Sodium Phosphate+$H_2O$-7 and 8 in a stability test chamber at 40 °C.

Used Dexamethasone Sodium Phosphate+ PVP-I-3 and -5 and Dexamethasone Sodium Phosphate+$H_2O$-7 for two week stability test. Used Dexamethasone Sodium Phosphate+ PVP-I-4 and -6 and Dexamethasone Sodium Phosphate+$H_2O$-8 for one month stability test. Used Dexamethasone Sodium Phosphate+$H_2O$-9 to prepare reference standard.

Stored Dexamethasone Sodium Phosphate-1 and 2 on the lab bench at room temperature. On Week 4, added 40 μL of PVP-I (10%, freshly prepared) and mix well to give Dexamethasone Sodium Phosphate+PVP-I-1 and 2. Used the resultant samples as time zero samples for HPLC analysis.

2.1.2.2 Preparation of Prednisolone Acetate Stability Test Samples

Aliquot ted 1 mL of ophthalmic suspension (USP, 1%) into eight amber HPLC vials to give the following samples:

Prednisolone Acetate-1, 2, 3, 4, 5, 6, 7, 8, and 9
Added 40 μL of PVP-I stock solution (10%) into Prednisolone Acetate -3, 4, 5, and 6, and mixed well to give the following samples:

Prednisolone Acetate + PVP-I-3, 4, 5, and 6
Stored Prednisolone Acetate +PVP-I-3 and 4 on the lab bench at room temperature and stored Prednisolone Acetate +PVP-I-5 and 6 in a stability test chamber at 40 °C.

Added 40 μL of water into Prednisolone Acetate -7, 8, and 9, and mixed well to give the following samples:

Prednisolone Acetate +$H_2O$-7, 8, and 9
Stored Prednisolone Acetate +$H_2O$-9 on the lab bench at room temperature and stored Prednisolone Acetate +$H_2O$-7 and 8 in a stability test chamber at 40 °C.

Used Prednisolone Acetate + PVP-I-3 and -5 and Prednisolone Acetate +$H_2O$-7 for two week stability test. Used Prednisolone Acetate + PVP-I-4 and -6 and Prednisolone Acetate +$H_2O$-8 for one month stability test. Used Prednisolone Acetate +$H_2O$-9 to prepare reference standard.

Stored Prednisolone Acetate -1 and 2 on the lab bench at room temperature. On Week 4, added 40 μL of PVP-I (10%, freshly prepared) and mixed well to give Prednisolone Acetate +PVP-I-1 and 2. Used the resultant samples as time zero samples for HPLC analysis.

2.1.2.3 Preparation of Difluprednate Stability Test Samples

Aliquotted 1 mL of Ophthalmic emulsion (0.05%) into eight amber HPLC vials to give the following samples:

Difluprednate-1, 2, 3, 4, 5, 6, 7, 8, and 9

Added 40 μL of PVP-I stock solution (10%) into Difluprednate-3, 4, 5, and 6, and mixed well to give the following samples:

Difluprednate+ PVP-I-3, 4, 5, and 6
Stored Difluprednate+PVP-I-3 and 4 on the lab bench at room temperature and stored Difluprednate+PVP-I-5 and 6 in a stability test chamber at 40 °C.

Added 40 μL of water into Difluprednate-7, 8, and 9, and mixed well to give the following samples:

Difluprednate+$H_2O$-7, 8, and 9
Stored Difluprednate+$H_2O$-9 on the lab bench at room temperature and stored Difluprednate+$H_2O$-7 and 8 in a stability test chamber at 40 °C.

Used Difluprednate+ PVP-I-3 and -5 and Difluprednate+$H_2O$-7 for two week stability test. Used Difluprednate+ PVP-I-4 and -6 and Difluprednate+$H_2O$-8 for one month stability test. Used Difluprednate+$H_2O$-9 to prepare reference standard.

Stored Difluprednate-1 and 2 on the lab bench at room temperature. On Week 4, added 40 μL of PVP-I (10%, freshly prepared) and mix well to give Difluprednate+PVP-I-1 and 2. Used the resultant samples as time zero samples for HPLC analysis.

2.1.2.4 Preparation of Loteprednol Etabonate Stability Test Samples

Aliquotted 1 mL of ophthalmic solution (USP, 0.1%) into eight amber HPLC vials to give the following samples:

Loteprednol Etabonate-1, 2, 3, 4, 5, 6, 7, 8, and 9
Added 40 μL of PVP-I stock solution (10%) into Loteprednol Etabonate-3, 4, 5, and 6, and mix well to give the following samples:

Loteprednol Etabonate+ PVP-I-3, 4, 5, and 6
Stored Loteprednol Etabonate+PVP-I-3 and 4 on the lab bench at room temperature and stored Loteprednol Etabonate+PVP-I-5 and 6 in a stability test chamber at 40 °C.

Added 40 μL of water into Loteprednol Etabonate-7, 8, and 9, and mixed well to give the following samples:

Loteprednol Etabonate+$H_2O$-7, 8, and 9
Stored Loteprednol Etabonate+$H_2O$-9 on the lab bench at room temperature and stored Loteprednol Etabonate+$H_2O$-7 and 8 in a stability test chamber at 40 °C.

Used Loteprednol Etabonate+ PVP-I-3 and -5 and Loteprednol Etabonate+$H_2O$-7 for two week stability test. Used Loteprednol Etabonate+ PVP-I-4 and -6 and Loteprednol Etabonate+$H_2O$-8 for one month stability test. Used Loteprednol Etabonate+$H_2O$-9 to prepare reference standard.

Stored Loteprednol Etabonate-1 and 2 on the lab bench at room temperature. On Week 4, added 40 μL of PVP-I (10%, freshly prepared) and mix well to give Loteprednol Etabonate+PVP-I-1 and 2. Used the resultant samples as time zero samples for HPLC analysis.

2.2 Preparation of Stability Test Samples for HPLC/UV Analysis

2.2.1 Preparation of PVP-I Solution for HPLC/UV Analysis

2.2.1.1 Preparation of PVP-I-4 mg/mL

Mixed 40 μL of PVP-I (10%) with 1 mL of water to give PVP-I-4 mg/mL.

2.2.1.2 Preparation of PVP-I Solution for Dexamethasone Sodium Phosphate Testing

Mixed 100 μL of PVP-I-4 mg/mL with 1.9 mL of water to give PVP-I-200 μg/L for HPLC analysis.

2.2.1.3 Preparation of PVP-I Solution for Prednisolone Acetate Testing

Mixed 100 μL of PVP-I-4 mg/mL with 9.9 mL of acetonitrile:water (1:1) to give PVP-I-40 μg/L.

Mixed 750 μL of PVP-I-40 μg/L with 750 μL of acetonitrile:water (1:1) to give PVP-I-20 μg/L for HPLC analysis.

2.2.1.4 Preparation of PVP-I Solution for Difluprednate Testing

Mixed 100 μL of PVP-I-4 mg/mL with 0.9 mL of methanol to give PVP-I-400 μg/L for HPLC analysis.

2.2.1.5 Preparation of PVP-I Solution for Loteprednol Etabonate Testing

Mixed 100 μL of PVP-I-4 mg/mL with 9.9 mL of acetonitrile:water (1:1) to give PVP-I-40 μg/L for HPLC analysis.

2.2.2 Preparation of Dexamethasone Sodium Phosphate for HPLC/UV Analysis

2.2.2.1 Preparation of Dexamethasone Sodium Phosphate Standard

Mixed 100 μL of Dexamethasone Sodium Phosphate +$H_2O$-9 with 1.9 mL of $H_2O$ in an HPLC vial to give Dexamethasone Sodium Phosphate+ $H_2O$-9-50 μg/mL.

2.2.2.2 Preparation of Dexamethasone Sodium Phosphate Stability Test Samples

Mixed 100 μL of Dexamethasone Sodium Phosphate+PVP-1, 2, 3, 4, 5, or 6 with 1.9 mL of $H_2O$ in an HPLC vial to give Dexamethasone Sodium Phosphate+PVP-1, 2, 3, 4, 5, or 6-50μg/mL for HPLC analysis.

2.2.2.3 Preparation of Control Dexamethasone Sodium Phosphate Stability Test Samples

Mixed 100 μL of Dexamethasone Sodium Phosphate+$H_2O$-7, or 8 with 1.9 mL of $H_2O$ in an HPLC vial to give Dexamethasone Sodium Phosphate+$H_2O$-7, or 8-50 μg/mL for HPLC analysis.

2.2.3 Preparation of Prednisolone Acetate for HPLC/UV Analysis

2.2.3.1 Preparation of Prednisolone Acetate Standard

Mixed 100 μL of Prednisolone Acetate+$H_2O$-9 with 9.9 mL of acetonitrile:water (1:1) to give Prednisolone Acetate+$H_2O$-9-100 μg/mL.

Mixed 750 μL of Prednisolone Acetate+$H_2O$-9-100 μg/mL with 750 μL of acetonitrile:H2O (1:1) in HPLC vial to give Prednisolone Acetate+$H_2O$-9-50 μg/mL for HPLC analysis.

2.2.3.2 Preparation of Prednisolone Acetate Stability Test Samples

Mixed 100 μL of Prednisolone Acetate+PVP-I-1, 2, 3, 4, 5, or 6 with 9.9 mL of acetonitrile:water (1:1) to give Prednisolone Acetate+PVP-I-1, 2, 3, 4, 5, or 6-100 μg/mL.

Mixed 750 μL of give Prednisolone Acetate+PVP-I-1, 2, 3, 4, 5, or 6-100 μg/mL with 750 μL of acetonitrile:H2O (1:1) in HPLC vial to give Prednisolone Acetate+PVP-I-1, 2, 3, 4, 5, or 6-50 μg/mL for HPLC analysis.

2.2.3.3 Preparation of Control Prednisolone Acetate Stability Test Samples

Mixed 100 μL of Prednisolone Acetate+$H_2O$-7, or 8 with 9.9 mL of acetonitrile:water (1:1) to give Prednisolone Acetate+$H_2O$-7, or 8-100 μg/mL.

Mixed 750 μL of give Prednisolone Acetate+$H_2O$-7, or 8-100 μg/mL with 750 μL of acetonitrile:H2O (1:1) in HPLC vial to give Prednisolone Acetate+$H_2O$-7, or 8-50 μg/mL for HPLC analysis.

2.2.4 Preparation of Loteprednol Etabonate for HPLC/UV Analysis

2.2.4.1 Preparation of Loteprednol Etabonate Standard

Mixed 100 μL of Loteprednol Etabonate+$H_2O$-9 with 9.9 mL of acetonitrile:water (1:1) to give Loteprednol

Etabonate+$H_2O$-9-50 μg/mL.

2.2.4.2 Preparation of Loteprednol Etabonate Stability Test Samples

Mixed 100 μL of Loteprednol Etabonate+PVP-I-1, 2, 3, 4, 5, or 6 with 9.9 mL of acetonitrile:water (1:1) to give Loteprednol Etabonate+PVP-I-1, 2, 3, 4, 5, or 6-50 μg/mL.

2.2.4.3 Preparation of Control Loteprednol Etabonate Stability Test Samples

Mixed 100 μL of Loteprednol Etabonate+$H_2O$-7, or 8 with 9.9 mL of acetonitrile:water (1:1) to give Loteprednol Etabonate+$H_2O$-7, or 8-50 μg/mL.

2.2.5 Preparation of Difluprednate for HPLC/UV Analysis

2.2.5.1 Preparation of Difluprednate Standard

Mixed 100 μL of Difluprednate +$H_2O$-9 with 0.9 mL of methanol in an HPLC vial to give Difluprednate+ $H_2O$-9-50 μg/mL.

2.2.5.2 Preparation of Difluprednate Stability Test Samples

Mixed 100 μL of Difluprednate+PVP-1, 2, 3, 4, 5, or 6 with 0.9 mL of methanol in an HPLC vial to give Difluprednate+PVP-1, 2, 3, 4, 5, or 6-50μg/mL for HPLC analysis.

2.2.5.3 Preparation of Control Difluprednate Stability Test Samples

Mixed 100 μL of Difluprednate+$H_2O$-7, or 8 with 0.9 mL of methanol in an HPLC vial to give Difluprednate+$H_2O$-7, or 8-50 μg/mL for HPLC analysis.

2.3 Preparation of Stability Test Samples for LC-MS/MS Analysis

2.3.1 Preparation of Dexamethasone Sodium Phosphate for LC-MS/MS Analysis

2.3.1.1 Preparation of Dexamethasone Sodium Phosphate Standard

Mixed 100 μL of Dexamethasone Sodium Phosphate+ $H_2O$-9-50 μg/mL with 0.9 mL of water in an HPLC vial.

2.3.1.2 Preparation of Dexamethasone Sodium Phosphate Stability Test Samples

Mixed 100 μL of Dexamethasone Sodium Phosphate+PVP-4, or 6-50μg/mL with 0.9 mL of water in an HPLC vial.

2.3.2 Preparation of Prednisolone Acetate for HPLC Analysis

2.3.2.1 Preparation of Prednisolone Acetate Standard

Mixed 100 μL of Prednisolone Acetate+$H_2O$-9-50 μg/mL with 0.9 mL of acetonitrile:water (1:1) in an HPLC vial.

2.3.2.2 Preparation of Prednisolone Acetate Stability Test Samples

Mixed 100 μL of Prednisolone Acetate+PVP-I-4, or 6-50 μg/ with 0.9 mL of acetonitrile:water (1:1) in an HPLC vial.

2.3.3 Preparation of Loteprednol Etabonate for HPLC Analysis

2.3.3.1 Preparation of Loteprednol Etabonate Standard

Mixed 100 μL of Loteprednol Etabonate+$H_2O$-9-50 μg/mL with 0.9 mL of acetonitrile:water (1:1) in an HPLC vial.

2.3.3.2 Preparation of Loteprednol Etabonate Stability Test Samples

Mixed 100 μL of Loteprednol Etabonate+PVP-I-4, or 6-50 μg/mL with 0.9 mL of acetonitrile:water (1:1) in an HPLC vial.

2.3.4 Preparation of Difluprednate for HPLC Analysis

2.3.4.1 Preparation of Difluprednate Standard

Mixed 100 μL of Difluprednate+ $H_2O$-9-50 μg/mL with 0.9 mL of methanol in an HPLC vial.

2.3.4.2 Preparation of Difluprednate Stability Test Samples

Mixed 100 μL of Difluprednate+PVP-4, or 6-50μg/mL for HPLC analysis with 0.9 mL of methanol in an HPLC vial.

2.4 HPLC/UV Chromatography

2.4.1 HPLC Method 1 (for Dexamethasone Sodium Phosphate)

| | |
|---|---|
| HPLC System: | SHIMADZU HPLC system (Pump: LC-10ADVP; Autosampler: SIL-HTC) |
| UV: | SPD-10A Vvp @239 and 210nm |
| Column: | Waters XTerra MS C18 3.5μm, 2.1x150mm, S/N 019435216117 |
| Column Temperature: | Room Temperature |
| Autosampler Temperature: | Room Temperature |
| Injection Vol.: | 10 μL |
| Mobile Phase A: | 0.01M NH4OAc in H2O |
| Mobile Phase B: | ACN |

Gradient:

| Time (min) | Flow (mL/min) | A | B |
|---|---|---|---|
| Initial | 0.2 | 100 | 0 |
| 40 | 0.2 | 40 | 60 |
| 45 | 0.2 | 2 | 98 |
| 50 | 0.2 | 2 | 98 |
| 51 | 0.2 | 100 | 100 |
| 70 | 0.2 | Stop | |

2.4.2 HPLC Method 2 (for Prednisolone Acetate)

The same as Method 1 except the gradient was changed as follows:

| Time (min) | Flow (mL/min) | A | B |
|---|---|---|---|
| Initial | 0.2 | 100 | 0 |
| 40 | 0.2 | 30 | 70 |
| 45 | 0.2 | 2 | 98 |
| 50 | 0.2 | 2 | 98 |
| 51 | 0.2 | 100 | 100 |
| 70 | 0.2 | Stop | |

2.4.3 HPLC Method 3 (for Loteprednol Etabonate and Difluprednate)

The same as Method 1 except the gradient was changed as follows:

| Time (min) | Flow (mL/min) | A | B |
|---|---|---|---|
| Initial | 0.2 | 100 | 0 |
| 40 | 0.2 | 20 | 80 |
| 45 | 0.2 | 2 | 98 |
| 50 | 0.2 | 2 | 98 |
| 51 | 0.2 | 100 | 100 |
| 70 | 0.2 | Stop | |

2.4.4 Date Integration and Calculation

The software provided with the HPLC system (LCSolution™ software, version 1.23, installed by SHIMADZU) was used to integrate the peak area.

The measured peak area was converted into concentrations (µg/mL) using the following equation:

$$C_x = A_x \times Cs \div A_x$$

where,

$C_x$ = Concentration (µg/mL) of analyte in stability samples

$A_x$ = Peak area from analyte in stability samples

Cs = Concentration (µg/mL) of analyte in standard samples

$A_s$ = Peak area from analyte in standard samples

2.5 Liquid Chromatography/Tandem Mass Spectrometry (LC-MS/MS)

HPLC Methods: The same as HPLC Method 1,2, and 3 under Section 2.4. MS Conditions:

| | |
|---|---|
| Mass Spectrometer: | API 3000 LC/MS/MS System |
| Ionization Mode: | ESI in Positive mode |
| ESI: | 5,000 V |
| Temperature: | 350 °C |
| Nebulizer Gas Flow (NEB): | 12 psi |
| Curtain Gas Flow (CUR): | 12 units |
| Turbo-Ion Spray Gas Flow: | 7,000-8,000 mL/min |
| Collision Gas (CAD): | 6 units |
| DP: | 30 |
| FP: | 80 |
| EP: | 8 |
| CXP: | 10 |

Precursor Ion, Product Ion, Collision Energy, and HPLC Retention Time

| Compound | Precursor ion (m/z) | Product ion (m/z) | Collision Energy (eV) | Retention Time (min) |
|---|---|---|---|---|
| Dexamethasone Phosphate | 473.3 | 355.2 | 20 | ~21.82 |
| | 473.3 | 337.2 | 20 | ~21.82 |
| | 473.3 | 237.2 | 35 | ~21.82 |
| Prednisolone Acetate | 403.1 | 325.2 | 20 | ~27.62 |
| | 403.1 | 307.2 | 20 | ~27.62 |
| | 403.1 | 147.1 | 30 | ~27.62 |
| Loteprednol Etabonate | 467.3 | 359.2 | 20 | ~33.15 |
| | 467.3 | 265.2 | 30 | ~33.15 |
| | 467.3 | 147.1 | 35 | ~33.15 |
| Difluprednate | 509.3 | 303.2 | 20 | ~31.85 |
| | 509.3 | 279.2 | 20 | ~31.85 |
| | 509.3 | 101.1 | 30 | ~31.85 |

3. RESULTS

3.1 LC/MS and MS/MS Analyses of Four Formulations

The four formulations used in this study were analyzed by HPLC -UV and MS and MS/MS. The HPLC-UV chromatograms and ESI-MS and MS/MS spectral data were presented in Figure 1 to Figure 4.

The presence of four steroids in the pharmaceutical formulations was confirmed by LC/UV-MS and MS/MS. Thus, the four pharmaceutical formulations can be used for this study.

3.2 HPLC System Suitability Testing

The four standard samples at the concentration of 50 μg/mL were analyzed using HPLC/UV methods developed at PharmaOn. The data are summarized in Table III.

As shown in Table III, the system used in this study was suitable to determine the levels of four steroids in the stability test samples.

3.3 HPLC/UV and LC-MS/MS Analysis of Stability Testing Samples

3.3.1 Dexamethasone Sodium Phosphate

3.3.1.1 PVP-I Sample

PVP-I in solvent at the same concentration as in stability test samples of dexamethasone sodium phosphate was analyzed using HPLC Method 1. The HPLC/UV chromatograms are depicted in Figure 5.

No dexamethasone phosphate was observed in PVP-I sample.

3.3.1.2 Dexamethasone Sodium Phosphate Stability Samples

The Day 0, Two Week, and One Month stability test samples were analyzed with reference standard samples (stored at room temperature in the absence of PVP I) using HPLC Method 1. The sample in the absence of PVP-I with the same concentration of dexamethasone phosphate as those stability samples at the presence of PVP-I was stored in the same stability chamber at 40°C for one month as control sample. The control sample was analyzed under the same conditions. The concentrations of dexamethasone phosphate in the stability samples were calculated. The data were summarized in Table IV. The HPLC/UV chromatograms of all reference standards and stability testing samples are depicted in Figure 6 to Figure 13.

The One Month stability test samples were analyzed with the reference standard sample using LC-MS/MS Method in MRM mode with three characteristic ion transitions to confirm the identity of dexamethasone phosphate in

stability testing samples. The mass ion chromatograms are presented in Figure 14 to Figure 16.

Identity of dexamethasone phosphate in reference standard sample and two One month stability test samples was confirmed by LC-MS/MS.

After storage at room temperature and 40°C in the presence of PVP-I (0.4%), the levels of dexamethasone phosphate in two weeks samples were only 83.04% and 84.57% of those in room temperature and 40°C Day 0 samples, respectively (Table IV). The respective data are 84.24% and 84.09% for one month testing (Table IV), indicating that dexamethasone phosphate was not stable in the presence of PVP-I (0.4%) under the current testing conditions.

As shown in Figure 6 to Figure 13, three additional peaks, Degradation Product 1, 2, and 3 (D1, D2, and D2), were observed in both Two Week and/or One Month stability testing samples at the presence of PVP I.

3.3.2 Prednisolone Acetate

3.3.2.1 PVP-I Sample

PVP-I in solvent at the same concentration as in stability test samples of prednisolone acetate was analyzed using HPLC Method 2. The HPLC/UV chromatograms are depicted in Figure 17.

No prednisolone acetate was observed in PVP-I sample.

3.3.2.2 Prednisolone Acetate Stability Samples

The Day 0, Two Week, and One Month stability test samples were analyzed with reference standard samples (stored at room temperature in the absence of PVP I) using HPLC Method 2. The sample in the absence of PVP-I with the same concentration of prednisolone acetate as those stability samples at the presence of PVP-I was stored in the same stability chamber at 40 oC for two week and one month as control samples. The control samples were analyzed under the same conditions. The concentrations of prednisolone acetate in the stability samples were calculated. The data were summarized in Table V. The HPLC/UV chromatograms of all reference standards and stability testing samples are depicted in Figure 18 to Figure 23.

The One Month stability test samples were analyzed with the reference standard sample using LC-MS/MS Method in MRM mode with three characteristic ion transitions to confirm the identity of prednisolone acetate in stability testing samples. The mass ion chromatograms are presented in Figure 24 to Figure 26.

After storage at room temperature and 40°C in the presence of PVP-I (0.4%), the levels of prednisolone acetate in two week testing samples were 99.24% and 96.60% of those in room temperature and 40°C Day 0 samples, respectively (Table V). The respective data are 95.66% and 96.79% for one month testing (Table V). Identical mass ion chromatograms and same intensities of mass ion response were observed in the reference standard and one month stability testing samples. The results from both HPLC/UV and LC-MS/MS analysis indicate that prednisolone acetate was stable in the presence of PVP-I (0.4%) under the current testing conditions.

3.3.3 Loteprednol Etabonate

3.3.3.1 PVP-I Sample

PVP-I in solvent at the same concentration as in stability test samples of loteprednol etabonate was analyzed using HPLC Method 3. The HPLC/UV chromatograms are depicted in Figure 27.

No loteprednol etabonate was observed in PVP-I sample.

3.3.3.2 Loteprednol Etabonate Stability Samples

The Day 0, Two Week, and One Month stability test samples were analyzed with reference standard samples (stored at room temperature in the absence of PVP I) using HPLC Method 3. The sample in the absence of PVP-I with the same concentration of loteprednol etabonate as those stability samples at the presence of PVP-I was stored in the same stability chamber at 40°C for two week and one month as control samples. The control samples were analyzed under the same conditions. The concentrations of loteprednol etabonate in the stability samples were calculated. The data were summarized in Table VI. The HPLC/UV chromatograms of all reference standards and stability testing samples are depicted in Figure 28 to Figure 33.

The One Month stability test samples were analyzed with the reference standard sample using LC-MS/MS Method in MRM mode with three characteristic ion transitions to confirm the identity of loteprednol etabonate in stability testing samples. The mass ion chromatograms are presented in Figure 34 to Figure 36.

After storage at room temperature and 40°C in the presence of PVP-I (0.4%), the levels of loteprednol etabonate

in two week testing samples were 101.43% and 100.07% of those in room temperature and 40°C Day 0 samples, respectively (Table VI). The respective data are 100.72% and 96.02% for one month testing (Table VI). Identical mass ion chromatograms and same intensities of mass ion response were observed in the reference standard and one month stability testing samples. The results from both HPLC/UV and LC-MS/MS analysis indicate that loteprednol etabonate was stable in the presence of PVP-I (0.4%) under the current testing conditions.

3.3.4 Difluprednate

3.3.4.1 PVP-I Sample

PVP-I in solvent at the same concentration as in stability test samples of difluprednate was analyzed using HPLC Method 3. The HPLC-UV chromatograms are depicted in Figure 37.

No difluprednate was observed in PVP-I sample.

3.3.4.2 Difluprednate Stability Samples

The Day 0, Two Week, and One Month stability test samples were analyzed with reference standard samples (stored at room temperature in the absence of PVP I) using HPLC Method 3. The sample in the absence of PVP-I with the same concentration of difluprednate as those stability samples at the presence of PVP-I was stored in the same stability chamber at 40°C for two week and one month as control samples. The control samples were analyzed under the same conditions. The concentrations of difluprednate in the stability samples were calculated. The data were summarized in Table VII. The HPLC/UV chromatograms of all reference standards and stability testing samples are depicted in Figure 38 to Figure 43.

The One Month stability test samples were analyzed with the reference standard sample using LC-MS/MS Method in MRM mode with three characteristic ion transitions to confirm the identity of difluprednate in stability testing samples. The mass ion chromatograms are presented in Figure 44 to Figure 46.

After storage at room temperature and 40°C in the presence of PVP-I (0.4%), the levels of difluprednate in two week testing samples were 103.23% and 99.30% of those in room temperature and 40°C Day 0 samples, respectively (Table VII). The respective data are 104.47% and 100.24% for one month testing (Table VII). Identical mass ion chromatograms and same intensities of mass ion response were observed in the reference standard and one month stability testing samples. The results from both HPLC/UV and LC-MS/MS analysis indicate that difluprednate was stable in the presence of PVP-I (0.4%) under the current testing conditions.

**TABLES**

Table I

| Four Pharmaceutical Formulations | | | |
|---|---|---|---|
| Steroids Name | Formulation/Product | Manufacture/Vendor | Lot No. |
| Dexamethasone Sodium Phosphate | Ophthalmic solution USP, 0.1% | Alcon Laboratories | 153643F |
| Prednisolone Acetate | Ophthalmic Suspension USP, 1% | Alcon Laboratories | 148757F |
| Loteprednol Etabonate | Ophthalmic Suspension, 0.5% | Baush & Lomb | 437291 |
| Difluprednate | Ophthalmic emulsion, 0.05% | Sirion Therapeutics | SIR9F001 |

Table II

| Four Steroids | | | |
|---|---|---|---|
| Name | Structure | MW | *Rt* (min) |
| Dexamethasone Sodium Phosphate | 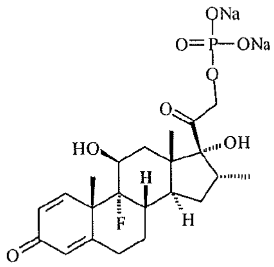 | 516.41 | ~21.13 |
| Prednisolone Acetate | 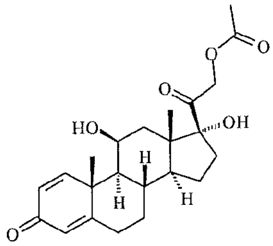 | 402.49 | ~26.51 |
| Loteprednol Etabonate | 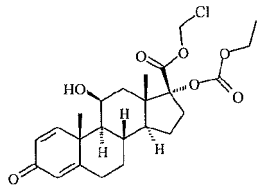 | 466.96 | ~32.15 |
| Difluprednate | 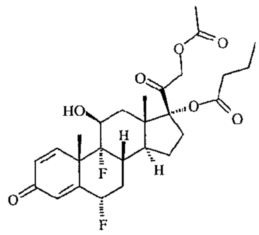 | 508.56 | ~31.04 |

Table III

| Summary of System Suitability Testing | | | |
|---|---|---|---|
| Dexamethasone Sodium Phosphate | | | |
| Replicate | HPLC Run No. | *Rt* (min) | Peak Area |
| 1 | 09701005_002 | 21.16 | 4860116 |
| 2 | 09701005_003 | 21.12 | 4887168 |
| 3 | 09701005_004 | 21.16 | 4845056 |
| 4 | 09701005_005 | 21.12 | 4841633 |
| 5 | 09701005_006 | 21.11 | 4815314 |
| Mean | | 21.13 | 4849857 |
| SD | | 0.024 | 26369 |
| CV (%) | | 0.11 | 0.54 |

(continued)

| Prednisolone Acetate | | | |
|---|---|---|---|
| Replicate | HPLC Run No. | *Rt* (min) | Peak Area |
| 1 | 09701005_012 | 26.53 | 5275846 |
| 2 | 09701005_013 | 26.52 | 5280425 |
| 3 | 09701005_014 | 26.54 | 5197617 |
| 4 | 09701005_015 | 26.39 | 5262924 |
| 5 | 09701005_016 | 26.55 | 5237854 |
| Mean | | 26.51 | 5250933 |
| SD | | 0.066 | 34088 |
| CV (%) | | 0.25 | 0.65 |
| Loteprednol Etabonate | | | |
| Replicate | HPLC Run No. | *Rt* (min) | Peak Area |
| 1 | 09701005_017 | 32.19 | 4352552 |
| 2 | 09701005_018 | 32.27 | 4272956 |
| 3 | 09701005_019 | 32.11 | 4368753 |
| 4 | 09701005_020 | 32.11 | 4281766 |
| 5 | 09701005_021 | 32.08 | 4292832 |
| Mean | | 32.15 | 4313772 |
| SD | | 0.078 | 43748 |
| CV (%) | | 0.24 | 1.01 |
| Difluprednate | | | |
| Replicate | HPLC Run No. | *Rt* (min) | Peak Area |
| 1 | 09701005_007 | 31.02 | 4746034 |
| 2 | 09701005_008 | 31.02 | 4715228 |
| 3 | 09701005_009 | 31.04 | 4761819 |
| 4 | 09701005_010 | 31.06 | 4715455 |
| 5 | 09701005_011 | 31.07 | 4728211 |
| Mean | | 31.04 | 4733349 |
| SD | | 0.023 | 20288 |
| CV (%) | | 0.07 | 0.43 |

Table IV

Analytical Data Summary of Dexamethasone Sodium Phosphate Stability Testing in PVP-I (0.4 %)

| Samples | HPLC Run No. | *Rt* (min) | Peak Area | Nominal Conc. (μg/mL)[a] | Calc Conc. (μg/mL)[b] | DF[c] | Calc Conc. (mg/mL)[d] | % of Std | % of Day 0 |
|---|---|---|---|---|---|---|---|---|---|
| Day 0 | | | | | | | | | |
| Std1 | 09701006_002 | 20.96 | 4964292 | | | | | | |
| Std2 | 09701006_003 | 21.23 | 4873676 | | | | | | |
| Mean | | | 4918984 | 50 | - | 20 | 1.0000 | - | - |
| Room Temp 1 | 09701006_004 | 21.17 | 5084959 | | 51.69 | 20 | 1.0337 | 103.37 | - |
| Room Temp 2 | 09701006_005 | 21.20 | 5093624 | | 51.78 | 20 | 1.0355 | 103.55 | - |
| Mean | | | 5089292 | | 51.73 | 20 | 1.0346 | 103.46 | - |
| 2 Weeks | | | | | | | | | |
| Std1 | 09701004_001 | 23.91 | 5019426 | | | | | | |
| Std2 | 09701004_002 | 23.07 | 5004047 | | | | | | |
| Mean | | | 5011737 | 50 | - | 20 | 1.0000 | - | - |
| Room Temp | 09701004_003 | 23.08 | 4305845 | | 42.96 | 20 | 0.8592 | 85.92 | 83.04 |
| 40 °C | 09701004_004 | 23.08 | 4385137 | | 43.75 | 20 | 0.8750 | 87.50 | 84.57 |
| One Month | | | | | | | | | |
| Std1 | 09701007_023 | 20.99 | 4845855 | | | | | | |
| Std2 | 09701007_024 | 21.03 | 4810095 | | | | | | |
| Mean | | | 4827975 | 50 | - | 20 | 1.0000 | - | - |
| Room Temp | 09701007_025 | 21.06 | 4207982 | | 43.58 | 20 | 0.8716 | 87.16 | 84.24 |
| 40 °C | 09701007_026 | 21.08 | 4216932 | | 43.67 | 20 | 0.8734 | 87.34 | 84.42 |
| 40 °C | 09701007_027 | 21.07 | 4184100 | | 43.33 | 20 | 0.8666 | 86.66 | 83.76 |
| Mean | | | 4200516 | | 43.50 | 20 | 0.8700 | 87.00 | 84.09 |
| 40 °C Control[e] | 09701007_028 | 21.11 | 4471624 | | 46.31 | 20 | 0.9262 | 92.62 | 92.62 |

[a]: Nominal concentration in HPLC samples; [b]: Calculated concentration in HPLC samples; [c]: Dilution factor; [d]: Calculated concentration in stability samples; [e]: Stored at 40 °C without PVP-I.

Table V

| Analytical Data Summary of Prednisolone Acetate Stability Testing in PVP-I (0.4 %) | | | | | | | | | |
|---|---|---|---|---|---|---|---|---|---|
| Samples | HPLC Run No. | *Rt* (min) | Peak Area | Nominal Conc. (μg/mL) [a] | Calc Conc. (μg/mL) [b] | DF[c] | Calc Conc. (mg/mL)[d] | % of Std | % of Day 0 |
| Day 0 | | | | | | | | | |
| Std1<br>Std2 | 09701006_010<br>09701006_011 | 26.74<br>26.75 | 5112497<br>5081143 | | | | | | |
| Mean | | | 5096820 | 50 | - | 200 | 10.000 | - | - |
| Room Temp 1<br>Room Temp 1 | 09701006_012<br>09701006_013 | 26.76<br>26.77 | 5342803<br>5323574 | | 52.41<br>52.22 | 200<br>200 | 10.483<br>10.445 | 104.83<br>104.45 | -<br>- |
| Mean | | | 5333189 | | 52.32 | 20 | 10.464 | 104.64 | - |
| 2 Weeks | | | | | | | | | |
| Std1<br>Std2 | 09701004_012<br>09701004_013 | 27.70<br>27.73 | 5305927<br>5317386 | | | | | | |
| Mean | | | 5311657 | 50 | - | 200 | 10.000 | - | - |
| Room Temp | 09701004_014 | 27.74 | 5515685 | | 51.92 | 200 | 10.384 | 103.84 | 99.24 |
| 40 °C | 09701004_015 | 27.71 | 5369264 | | 50.54 | 200 | 10.108 | 101.08 | 96.60 |
| 40 °C Control[e] | 09701004_016 | 27.61 | 5351149 | | 50.37 | 200 | 10.074 | 100.74 | 100.74 |
| One Month | | | | | | | | | |
| Std1<br>Std2 | 09701007_012<br>09701007_013 | 26.78<br>26.79 | 5181293<br>5127543 | | | | | | |
| Mean | | | 5154418 | 50 | - | 200 | 10.000 | - | - |
| Room Temp | 09701007_014 | 26.81 | 5159554 | | 50.05 | 200 | 10.010 | 100.10 | 95.66 |
| 40 °C | 09701007_015 | 26.78 | 5220242 | | 50.64 | 200 | 10.128 | 101.28 | 96.79 |
| 40 °C Control[e] | 09701007_016 | 26.80 | 5169543 | | 50.15 | 200 | 10.029 | 100.29 | 100.29 |

[a]: Nominal concentration in HPLC samples; [b]: Calculated concentration in HPLC samples; [c]: Dilution factor; [d]: Calculated concentration in stability samples; [e]: Stored at 40 °C without PVP-1.

Table VI

| Analytical Data Summary of Loteprednol Etabonate Testing in PVP-I (0.4 %) | | | | | | | | | |
|---|---|---|---|---|---|---|---|---|---|
| Samples | HPLC Run No. | *Rt* (min) | Peak Area | Nominal Conc. (μg/mL)[a] | Calc Conc. (μg/mL)[b] | DF[c] | Calc Conc. (mg/mL)[d] | % of Std | % of Day 0 |
| Day 0 | | | | | | | | | |
| Std1<br>Std2 | 09701006_014<br>09701006_015 | 32.41<br>32.41 | 4172610<br>4193226 | | | | | | |
| Mean | | | 4182918 | 50 | - | 100 | 5.0000 | - | - |
| Room Temp 1 | 09701006_016 | 32.45 | 4224688 | | 50.50 | 100 | 5.0499 | 101.00 | - |
| Room Temp 2 | 09701006_017 | 32.27 | 4180845 | | 49.98 | 100 | 4.9975 | 99.95 | - |
| Mean | 09701006_017 | 32.27 | 4202767 | | 50.24 | 20 | 5.0237 | 100.48 | - |
| 2 Weeks | | | | | | | | | |
| Std1<br>Std2 | 09701004_017<br>09701004_018 | 32.87<br>33.02 | 4460467<br>4431159 | | | | | | |
| Mean | | | 4445813 | 50 | - | 100 | 5.0000 | - | - |
| Room Temp | 09701004_019 | 33.03 | 4530572 | | 50.95 | 100 | 5.0953 | 101.91 | 101.43 |
| 40 °C | 09701004_020 | 32.99 | 4470012 | | 50.27 | 100 | 5.0272 | 100.54 | 100.07 |
| 40 °C Control[e] | 09701004_021 | 32.98 | 4521010 | | 50.85 | 100 | 5.0846 | 101.69 | 101.69 |
| One Month | | | | | | | | | |
| Std1<br>Std2 | 09701007_017<br>09701007_018 | 32.45<br>32.30 | 4074874<br>4068504 | | | | | | |
| Mean | | | 4071689 | 50 | - | 100 | 5.0000 | - | - |
| Room Temp | 09701007_019 | 32.34 | 4120353 | | 50.60 | 100 | 5.0598 | 101.20 | 100.72 |
| 40 °C | 09701007_020 | 32.48 | 3928248 | | 48.24 | 100 | 4.8239 | 96.48 | 96.02 |
| 40 °C Control[e] | 09701007_021 | 32.46 | 3975565 | | 48.82 | 100 | 4.8820 | 97.64 | 97.64 |

[a]: Nominal concentration in HPLC samples; [b]: Calculated concentration in HPLC samples; [c]: Dilution factor; [d]: Calculated concentration in stability samples; [e]: Stored at 40 °C without PVP-I.

Table VII

Analytical Data Summary of Difluprednate Stability Testing in PVP-I (0.4 %)

| Samples | HPLC Run No. | *Rt* (min) | Peak Area | Nominal Conc. (μg/mL)[a] | Calc Conc. (μg/mL)[b] | DF[c] | Calc Conc. (mg/mL)[d] | % of Std | % of Day 0 |
|---|---|---|---|---|---|---|---|---|---|
| Day 0 | | | | | | | | | |
| Std1<br>Std2 | 09701006_006<br>09701006_007 | 31.17<br>31.10 | 4647615<br>4757011 | | | | | | |
| Mean | | | 4702313 | 50 | - | 10 | 0.5000 | - | - |
| Room Temp 1 | 09701006_008 | 31.17 | 4503933 | | 47.89 | 10 | 0.4789 | 95.78 | - |
| Room Temp 2 | 09701006_009 | 31.16 | 4548076 | | 48.36 | 10 | 0.4836 | 96.72 | - |
| Mean | 09701006 009 | 31.16 | 4526005 | | 48.13 | 20 | 0.4813 | 96.25 | - |
| 2 Weeks | | | | | | | | | |
| Std1<br>Std2 | 09701004_007<br>09701004_008 | 31.76<br>31.76 | 4849758<br>4871971 | | | | | | |
| Mean | | | 4860865 | 50 | - | 10 | 0.5000 | - | - |
| Room Temp | 09701004_009 | 31.75 | 4829559 | | 49.68 | 10 | 0.4968 | 99.36 | 103.23 |
| 40 °C | 09701004_010 | 31.74 | 4645691 | | 47.79 | 10 | 0.4779 | 95.57 | 99.30 |
| 40 °C Control[e] | 09701004 011 | 31.85 | 4350242 | | 44.75 | 10 | 0.4475 | 89.50 | 89.50 |
| One Month | | | | | | | | | |
| Std1<br>Std2 | 09701007_007<br>09701007_008 | 31.26<br>31.21 | 4519656<br>4538123 | | | | | | |
| Mean | | | 4528890 | 50 | - | 10 | 0.5000 | - | - |
| Room Temp | 09701007_009 | 31.20 | 4554140 | | 50.28 | 10 | 0.5028 | 100.56 | 104.47 |
| 40 °C | 09701007_010 | 31.21 | 4369678 | | 48.24 | 10 | 0.4824 | 96.48 | 100.24 |
| 40 °C Control[e] | 09701007_011 | 31.24 | 4432171 | | 48.93 | 10 | 0.4893 | 97.86 | 97.86 |

[a]: Nominal concentration in HPLC samples; [b]: Calculated concentration in HPLC samples; [c]: Dilution factor; [d]: Calculated concentration in stability samples; [e]: Stored at 40 °C without PVP-I.

Example 2: Stability Testing For Steroids and NSAIDS Combined With 0.6% Povidone Iodine

Steroids and NSAIDS were mixed with PVP-I at the concentration of 0.6% w/w on Day 1. The resultant mixtures will be split to glass vials and stored at room temperature. fluorometholone alcohol, medrysone, prednisone sodium phosphate, rimexolone, hydrocortisone, hydrocortisone acetate, lodoxamide tromethamine, nepafenac, bromfenac, and ketorolac. Testing timepoints included day 0 (Time Zero), and week 4. Tests were conducted at room temperature. The testing samples were analyzed using liquid chromatography and tandem mass spectrometry (LC/MS/MS) methods at Day 0, and Week 4. The steroids and NSAIDS standards were also analyzed and steroids and NSAIDS levels in testing samples were determined.

Rimexolone, hydrocortisone acetate, lodoxamide, and bromfenac samples appeared to be stable. Nepafenac was generally stable, but to a lesser degree. Prednisone sodium phosphate was stable to a lesser degree than nepafenac. In an embodiment, a result wherein about 10% or greater reduction in concentration of a compound of interest is observed is an indication that the compound is not stable. In an embodiment, a result wherein a reduction in the concentration of a compound of interest is observed, but about less than 10% reduction in concentration of a compound of interest is observed, is an indication that the compound is semi-stable. In an embodiment, a result wherein there is substantially no reduction in concentration of a compound of interest observed is an indication that the compound is stable.

Table VIII illustrates the analytical data summary of bromfenac stability testing in 0.6% PVP-I at room temperature. Table IX illustrates the analytical data summary of hydrocortisone acetate stability testing in 0.6% PVP-I at room temperature. Table X illustrates the analytical data summary of rimexolone stability testing in 0.6% PVP-I at room temperature. Table XI illustrates the analytical data summary of prednisone sodium phosphate stability testing in 0.6% PVP-I at room temperature. Table XII illustrates the analytical data summary of nepafenac stability testing in 0.6% PVP-I at room temperature. Table XIII illustrates the analytical data summary of fluorometholone stability testing in 0.6% PVP-I at room temperature. For Tables VIII-XIII, a: Nominal concentration in HPLC samples; b: Calculated concentration in HPLC samples; c: Dilution factor; d: Calculated concentration in stability samples; e: Spiked 50μL of $H_2O$ and stored at room temperature without PVP-I.

Table VIII: Bromfenac testing.

| Samples | *Rt* (min) | Peak Area | Nominal Conc. (μg/mL)[a] | Calc Conc. (μg/mL)[b] | DF[c] | Calc Conc. (μg/mL)[d] | % of Std | % of Day 0 |
|---|---|---|---|---|---|---|---|---|
| Standard 1 | 24.925 | 11390037 | 90 | - | 10 | 900 | - | - |
| Standard 2 | 25.034 | 11288449 | 90 | - | 10 | 900 | - | - |
| Mean | 24.980 | 11339243 | 90 | - | 10 | 900 | - | - |
| Day 0 | | | | | | | | |
| Replicate 1 | 24.900 | 11310534 | 90 | 89.77 | 10 | 897.7 | 99.74 | - |
| Replicate 2 | 24.889 | 11107933 | 90 | 88.16 | 10 | 881.6 | 97.96 | - |
| Mean | 24.895 | 11209234 | 90 | 88.97 | 10 | 889.7 | 98.86 | - |
| Four Weeks | | | | | | | | |
| Replicate 1 | 24.960 | 11211003 | 90 | 88.98 | 10 | 889.8 | 98.87 | 100.01 |
| Replicate 2 | 24.963 | 11066657 | 90 | 87.84 | 10 | 878.4 | 97.6 | 98.73 |
| Mean | 24.962 | 11138830 | 90 | 88.41 | 10 | 884.1 | 98.23 | 99.37 |
| Control[e] | 24.978 | 11342445 | 90 | 90.03 | 10 | 900.3 | 100.03 | 101.19 |

Table IX: Hydrocortisone acetate testing.

| Samples | *Rt* (min) | Peak Area | Nominal Conc. (μg/mL) [a] | Calc Conc. (μg/mL) [b] | DF[c] | Calc Conc. (μg/mL) [d] | % of Std | % of Day 0 |
|---|---|---|---|---|---|---|---|---|
| Standard 1 | 29.087 | 9578995 | 100 | - | 50 | 5000 | - | - |
| Standard 2 | 29.215 | 9456921 | 100 | - | 50 | 5000 | - | - |
| Mean | 29.151 | 9517958 | 100 | - | 50 | 5000 | - | - |
| Day 0 | | | | | | | | |
| Replicate 1 | 29.067 | 9672596 | 100 | 101.62 | 50 | 5081 | 101.62 | - |
| Replicate 2 | 29.107 | 9472035 | 100 | 99.52 | 50 | 4976 | 99.52 | - |
| Mean | 29.087 | 9572316 | 100 | 100.57 | 50 | 5029 | 100.57 | - |
| Four Weeks | | | | | | | | |
| Replicate 1 | 29.125 | 9627042 | 100 | 101.15 | 50 | 5058 | 101.15 | 100.58 |
| Replicate 2 | 29.127 | 9699896 | 100 | 101.91 | 50 | 5096 | 101.91 | 101.33 |
| Mean | 29.126 | 9663469 | 100 | 101.53 | 50 | 5077 | 101.53 | 100.95 |
| Control[e] | 29.178 | 9676282 | 100 | 101.66 | 50 | 5083 | 101.66 | 101.08 |

Table X: Rimexolone testing.

| Samples | *Rt* (min) | Peak Area | Nominal Conc. (μg/mL)[a] | Calc Conc. (μg/mL)[b] | DF[c] | Calc Conc. (μg/mL) [d] | % of Std | % of Day 0 |
|---|---|---|---|---|---|---|---|---|
| Standard 1 | 39.98 | 3399891 | 100 | - | 100 | 10,000 | - | - |
| Standard 2 | 39.961 | 3404392 | 100 | - | 100 | 10,000 | - | - |
| Mean | 39.971 | 3402142 | 100 | - | 100 | 10,000 | - | - |
| Day 0 | | | | | | | | |
| Replicate 1 | 40.004 | 3362494 | 100 | 98.83 | 100 | 9883 | 98.83 | - |
| Replicate 2 | 40.018 | 3418997 | 100 | 100.5 | 100 | 10050 | 100.5 | - |
| Mean | 40.011 | 3390746 | 100 | 99.67 | 100 | 9967 | 99.67 | - |
| Four Weeks | | | | | | | | |
| Replicate 1 | 40.035 | 3398853 | 100 | 99.9 | 100 | 9990 | 99.9 | 100.23 |
| Replicate 2 | 39.948 | 3375059 | 100 | 99.2 | 100 | 9920 | 99.2 | 99.53 |
| Mean | 39.992 | 3386956 | 100 | 99.55 | 100 | 9955 | 99.55 | 99.88 |
| Control[e] | 20.117 | 3303121 | 100 | 97.09 | 100 | 9709 | 97.09 | 97.41 |

Table XI: Prednisone sodium phosphate testing.

| Samples | *Rt* (min) | Peak Area | Nominal Conc. (μg/mL)[a] | Calc Conc. (μg/mL)[b] | DF[c] | Calc Conc. (μg/mL)[d] | % of Std | % of Day 0 |
|---|---|---|---|---|---|---|---|---|
| Standard 1 | 26.61 | 8422981 | 100 | - | 50 | 5000 | - | - |
| Standard 2 | 26.748 | 8470831 | 100 | - | 50 | 5000 | - | - |
| Mean | 26.679 | 8446906 | 100 | - | 50 | 5000 | - | - |
| Day 0 | | | | | | | | |
| Replicate 1 | 26.843 | 8272276 | 100 | 97.93 | 50 | 4897 | 97.93 | - |
| Replicate 2 | 26.717 | 8243394 | 100 | 97.59 | 50 | 4880 | 97.59 | - |
| Mean | 26.780 | 8257835 | 100 | 97.76 | 50 | 4888 | 97.76 | - |
| Four Weeks | | | | | | | | |
| Replicate 1 | 26.608 | 7853275 | 100 | 92.97 | 50 | 4649 | 92.97 | 95.1 |
| Replicate 2 | 26.738 | 7946048 | 100 | 94.07 | 50 | 4704 | 94.07 | 96.23 |
| Mean | 26.673 | 7899661.5 | 100 | 93.52 | 50 | 4676 | 93.52 | 95.66 |
| Control[e] | 26.477 | 8495335 | 100 | 100.57 | 50 | 5029 | 100.57 | 102.87 |

Table XII: Nepafenac testing (270nm).

| Samples | *Rt* (min) | Peak Area | Nominal Conc. (μg/mL) [a] | Calc Conc. (μg/mL) [b] | DF[c] | Calc Conc. (μg/mL) [d] | % of Std | % of Day 0 |
|---|---|---|---|---|---|---|---|---|
| Standard 1 | 34.589 | 727 | 50 | - | 100 | 5,000 | - | - |
| Standard 2 | 34.580 | 729 | 50 | - | 100 | 5,000 | - | - |
| Mean | 34.585 | 728 | 50 | - | 100 | 5,000 | - | - |
| Day 0 | | | | | | | | |
| Replicate 1 | 34.568 | 715 | 50 | 49.11 | 100 | 4911 | 98.22 | - |
| Replicate 2 | 34.548 | 722 | 50 | 49.59 | 100 | 4959 | 99.18 | - |
| Mean | 34.558 | 719 | 50 | 49.35 | 100 | 4935 | 98.7 | - |
| Four Weeks | | | | | | | | |
| Replicate 1 | 34.538 | 703 | 50 | 48.28 | 100 | 4828 | 96.56 | 97.83 |
| Replicate 2 | 34.577 | 694 | 50 | 47.66 | 100 | 4766 | 95.32 | 96.58 |
| Mean | 34.558 | 698.5 | 50 | 47.97 | 100 | 4797 | 95.94 | 97.2 |
| Control[e] | 34.570 | 719 | 50 | 49.38 | 100 | 4938 | 98.76 | 100.06 |

Table XIII: Fluorometholone testing.

| Samples | *Rt* (min) | Peak Area | Nominal Conc. (μg/mL)[a] | Calc Conc. (μg/mL)[b] | DF[c] | Calc Conc. (μg/mL)[d] | % of Std | % of Day 0 |
|---|---|---|---|---|---|---|---|---|
| Standard 1 | 38.664 | 1872 | 50 | - | 20 | 1,000 | - | - |
| Standard 2 | 38.614 | 1877 | 50 | - | 20 | 1,000 | - | - |
| Mean | 38.639 | 1875 | 50 | - | 20 | 1,000 | - | - |
| Day 0 | | | | | | | | |
| Replicate 1 | 38.648 | 1901 | 50 | 50.71 | 20 | 1014 | 101.42 | - |
| Replicate 2 | 38.646 | 1896 | 50 | 50.57 | 20 | 1011 | 101.14 | - |
| Mean | 38.647 | 1899 | 50 | 50.64 | 20 | 1013 | 101.28 | - |
| Four Weeks | | | | | | | | |
| Replicate 1 | 38.611 | 1861 | 50 | 49.64 | 20 | 993 | 99.28 | 98.03 |
| Replicate 2 | 38.613 | 1877 | 50 | 50.07 | 20 | 1001 | 100.14 | 98.87 |
| Mean | 38.612 | 1869 | 50 | 49.85 | 20 | 997 | 99.7 | 98.44 |
| Control[e] | 38.602 | 1860 | 50 | 49.61 | 20 | 992 | 99.22 | 97.97 |

It is to be understood that at least some of the descriptions of the invention have been simplified to focus on elements that are relevant for a clear understanding of the invention, while eliminating, for purposes of clarity, other elements that those of ordinary skill in the art will appreciate may also comprise a portion of the invention. However, because such elements are well known in the art, and because they do not necessarily facilitate a better understanding of the invention, a description of such elements is not provided herein.

Further, to the extent that the method does not rely on the particular order of steps set forth herein, the particular order of the steps should not be construed as limitation on the claims. The methods of the present disclosure should not be limited to the performance of their steps in the order written, and one skilled in the art can readily appreciate that the steps may be varied and still remain within the scope of the present disclosure.

## Claims

**1.** An ophthalmic composition suitable for topical administration to an eye, effective for treatment of a microorganism infection or a disorder of at least one tissue of the eye, comprising:

0.3 to 1% (w/w) polyvinylpyrrolidinone-iodine complex;
0.05 to 2% (w/w) bromfenac;
0.005% to 0.02% (w/w) EDTA;
0.01 to 0.5% (w/w) sodium chloride;
0.02 to 0.1% (w/w) tyloxapol;
0.5% to 2% (w/w) sodium sulfate; and
0.1 to 0.5% (w/w) hydroxyethylcellulose.

**2.** An ophthalmic composition according to claim 1 for use in a method of treating an eye disorder selected from the group consisting of a microorganism infection of at least one tissue of the eye, conjunctivitis, corneal abrasion, ulcerative infectious keratitis, epithelial keratitis, stromal keratitis and herpesvirus-related keratitis.

3. The ophthalmic composition for use as claimed in claim 2 wherein said treatment is treatment of infection following corneal abrasion or ocular surgery.

4. The ophthalmic composition for use as claimed in claim 2, wherein said microorganism is a bacteria, virus, fungi, or amoebae.

5. The ophthalmic composition for use as claimed in claim 4, wherein said bacteria is mycobacteria.

6. The ophthalmic composition for use as claimed in claim 2 wherein the ophthalmic composition is used in one or more doses between 10 microliters to 200 microliters.

7. The ophthalmic composition for use as claimed in claim 2 wherein the ophthalmic composition is used in one or more doses between 50 microliters to 80 microliters.

8. The ophthalmic composition for use as claimed in claim 2 wherein said medicament is administered to said eye one to four times a day.

9. The ophthalmic composition for use as claimed in claim 2 wherein said medicament is administered to said eye one to twenty-four times a day.

**Patentansprüche**

1. Ophthalmische Zusammensetzung, die für die topische Verabreichung ans Auge geeignet ist und zur Behandlung einer Mikroorganismus-Infektion oder einer Störung mindestens eines Gewebes des Auges wirksam ist, umfassend:

   0,3 bis 1 % (w/w) Polyvinylpyrrolidinon-Iod-Komplex;
   0,05 bis 2 % (w/w) Bromfenac;
   0,005 bis 0,02 % (w/w) EDTA;
   0,01 bis 0,5 % (w/w) Natriumchlorid;
   0,02 bis 0,1 % (w/w) Tyloxapol;
   0,5 bis 2 % (w/w) Natriumsulfat; und
   0,1 bis 0,5 % (w/w) Hydroxyethylcellulose.

2. Ophthalmische Zusammensetzung nach Anspruch 1 zur Verwendung bei einer Methode zur Behandlung einer Augenstörung aus der Gruppe bestehend aus einer Mikroorganismus-Infektion mindestens eines Gewebes des Auges, Konjunktivitis, kornealer Abrasio, ulzeröser infektiöser Keratitis, Epithelkeratitis, Stromakeratitis und Herpesvirus-bedingter Keratitis.

3. Ophthalmische Zusammensetzung zur Verwendung nach Anspruch 2, wobei es sich bei der Behandlung um die Behandlung einer Infektion nach kornealer Abrasio oder Augenchirurgie handelt.

4. Ophthalmische Zusammensetzung zur Verwendung nach Anspruch 2, wobei es sich bei dem Mikroorganismus um ein Bakterium, ein Virus, einen Pilz oder eine Amöbe handelt.

5. Ophthalmische Zusammensetzung zur Verwendung nach Anspruch 4, wobei es sich bei dem Bakterium um ein Mycobakterium handelt.

6. Ophthalmische Zusammensetzung zur Verwendung nach Anspruch 2, wobei die ophthalmische Zusammensetzung in einer oder mehreren Dosen zwischen 10 Mikroliter bis 200 Mikroliter verwendet wird.

7. Ophthalmische Zusammensetzung zur Verwendung nach Anspruch 2, wobei die ophthalmische Zusammensetzung in einer oder mehreren Dosen zwischen 50 Mikroliter bis 80 Mikroliter verwendet wird.

8. Ophthalmische Zusammensetzung zur Verwendung nach Anspruch 2, wobei das Medikament ein- bis viermal pro Tag an das Auge verabreicht wird.

9. Ophthalmische Zusammensetzung zur Verwendung nach Anspruch 2, wobei das Medikament ein- bis vierundzwanzigmal pro Tag an das Auge verabreicht wird.

## Revendications

1. Composition ophtalmique appropriée pour une administration topique à un oeil, efficace pour le traitement d'une infection par un microorganisme ou d'un trouble d'au moins un tissu de l'oeil, comprenant :

   de 0,3 à 1 % (m/m) de complexe polyvinylpyrrolidone-iode ;
   de 0,05 à 2 % (m/m) de bromfénac ;
   de 0,005 % à 0,02 % (m/m) d'EDTA ;
   de 0,01 à 0,5 % (m/m) de chlorure de sodium ;
   de 0,02 à 0,1 % (m/m) de tyloxapol ;
   de 0,5 % à 2 % (m/m) de sulfate de sodium ; et
   de 0,1 à 0,5 % (m/m) d'hydroxyéthylcellulose.

2. Composition ophtalmique selon la revendication 1 pour une utilisation dans une méthode de traitement d'un trouble oculaire choisi dans le groupe constitué par une infection par un microorganisme d'au moins un tissu de l'oeil, la conjonctivite, l'abrasion de la cornée, la kératite infectieuse ulcéreuse, la kératite épithéliale, la kératite stromale et la kératite liée au virus de l'herpès.

3. Composition ophtalmique pour une utilisation telle que revendiquée dans la revendication 2, dans laquelle ledit traitement est un traitement d'une infection suite à une abrasion de la cornée ou une opération oculaire.

4. Composition ophtalmique pour une utilisation telle que revendiquée dans la revendication 2, dans laquelle ledit microorganisme est des bactéries, des virus, des champignons ou des amibes.

5. Composition ophtalmique pour une utilisation telle que revendiquée dans la revendication 4, dans laquelle ladite bactérie est une mycobactérie.

6. Composition ophtalmique pour une utilisation telle que revendiquée dans la revendication 2, dans laquelle la composition ophtalmique est utilisée sous la forme d'une ou plusieurs doses entre 10 microlitres à 200 microlitres.

7. Composition ophtalmique pour une utilisation telle que revendiquée dans la revendication 2, dans laquelle la composition ophtalmique est utilisée sous la forme d'une ou plusieurs doses entre 50 microlitres à 80 microlitres.

8. Composition ophtalmique pour une utilisation telle que revendiquée dans la revendication 2, dans laquelle ledit médicament est administré audit oeil une à quatre fois par jour.

9. Composition ophtalmique pour une utilisation telle que revendiquée dans la revendication 2, dans laquelle ledit médicament est administré audit oeil une à vingt-quatre fois par jour.

HPLC-UV/(+)ESI-MS and MS/MS Spectral Data of Dexamethasone Phosphate

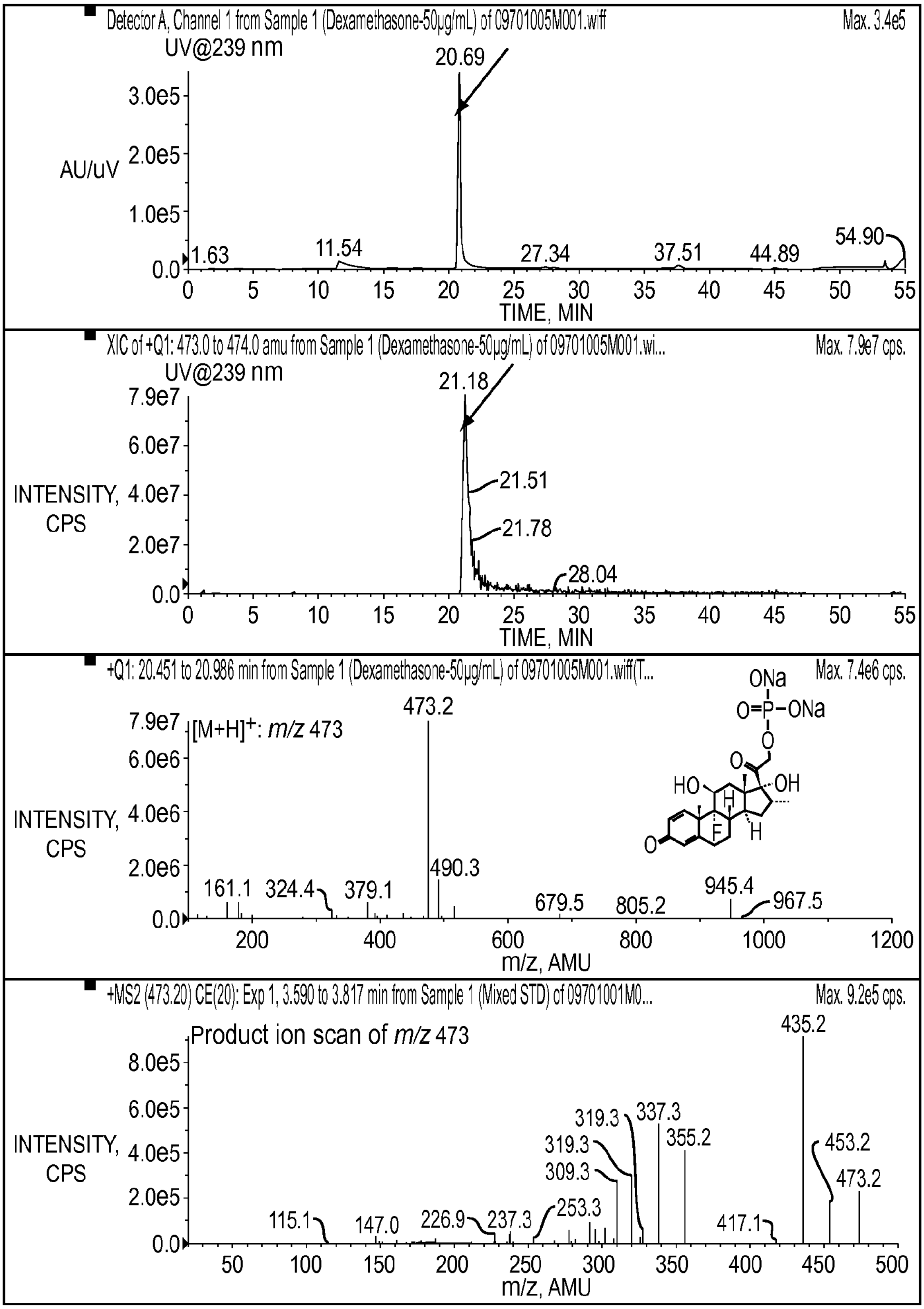


FIG. 1

HPLC-UV/(+)ESI-MS and MS/MS Spectral Data of Prednisolone Acetate

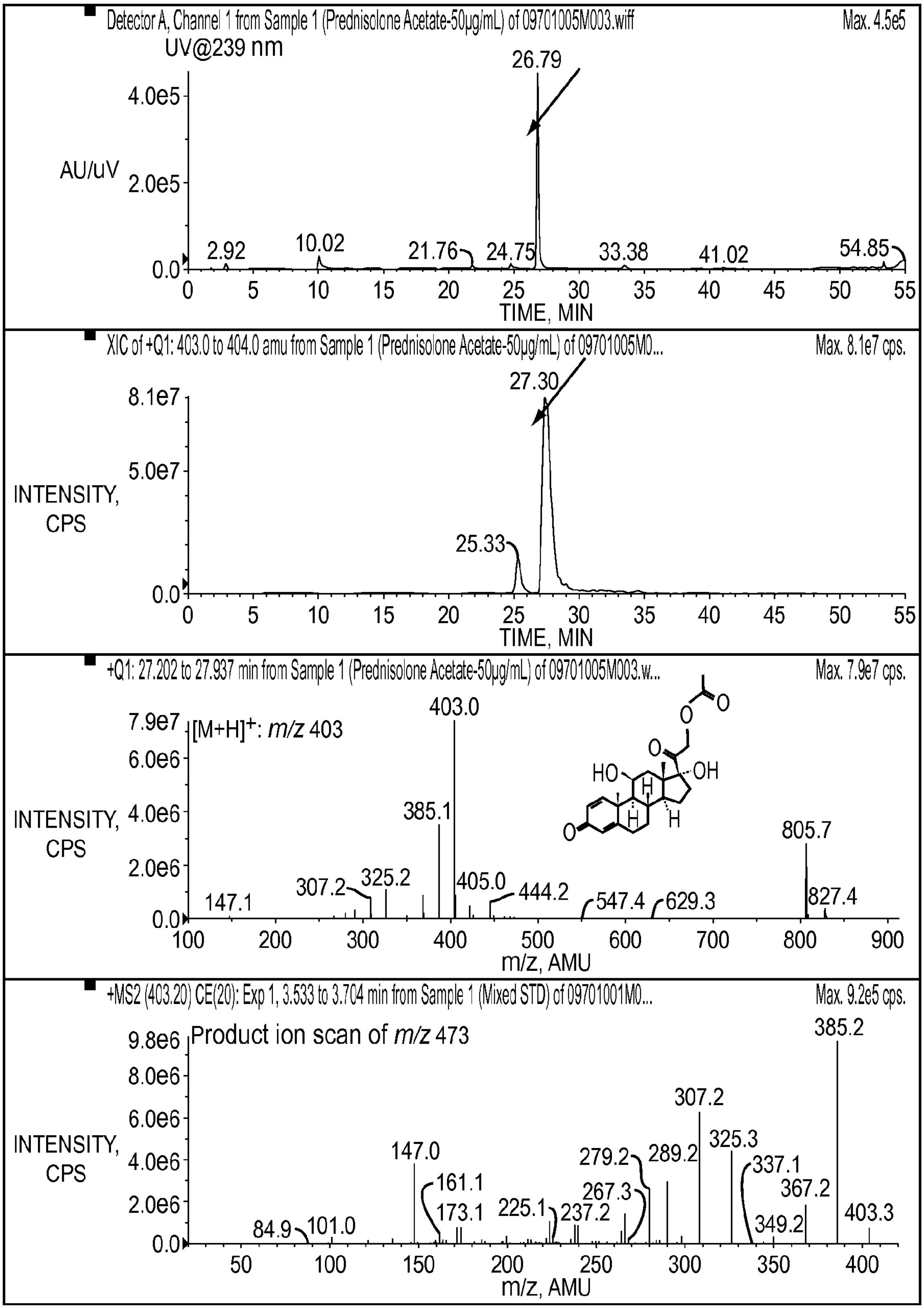


FIG. 2

HPLC-UV/(+)ESI-MS and MS/MS Spectral Data of Loteprednol Etabonate

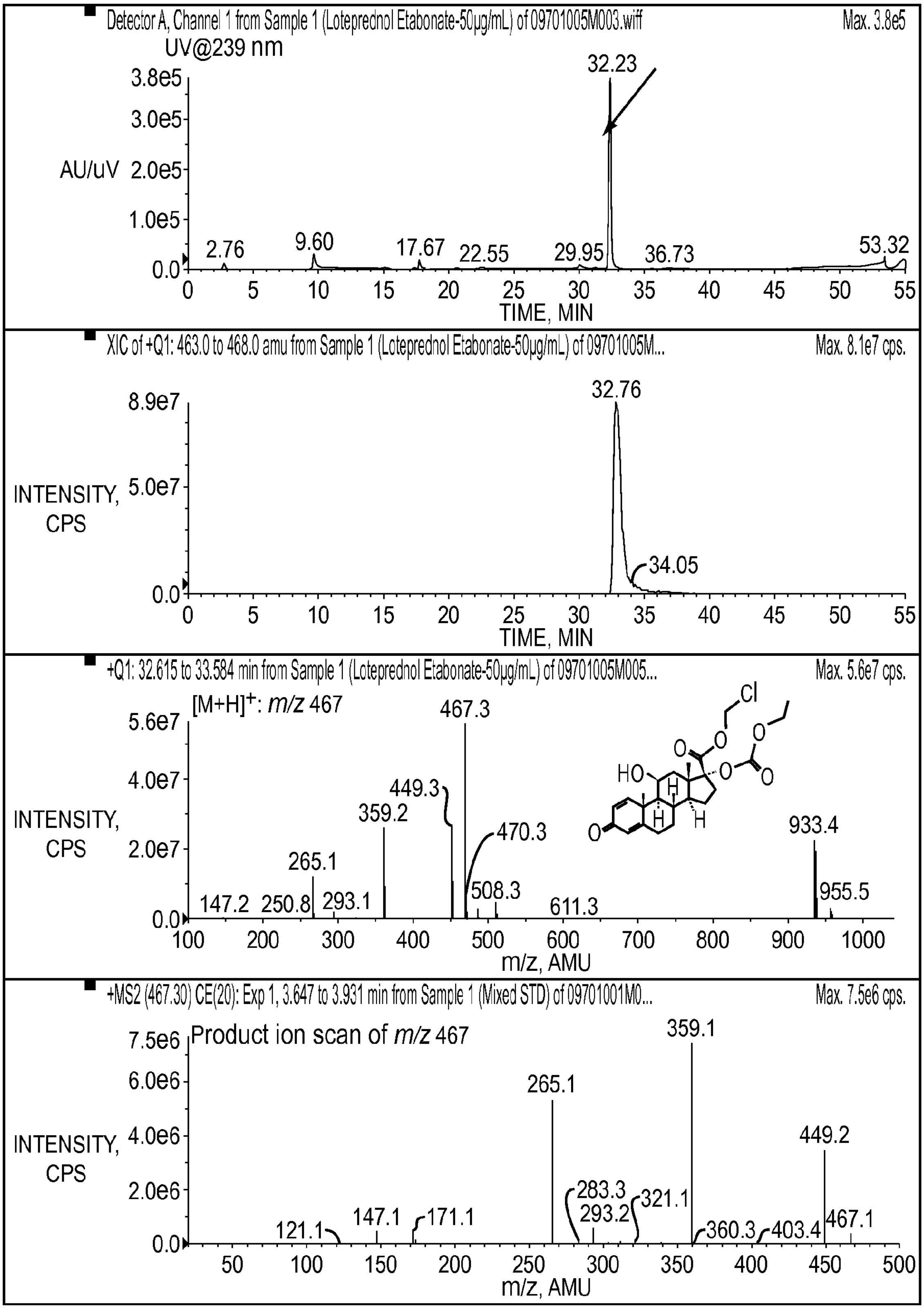


FIG. 3

HPLC-UV/(+)ESI-MS and MS/MS Spectral Data of Difluprednate

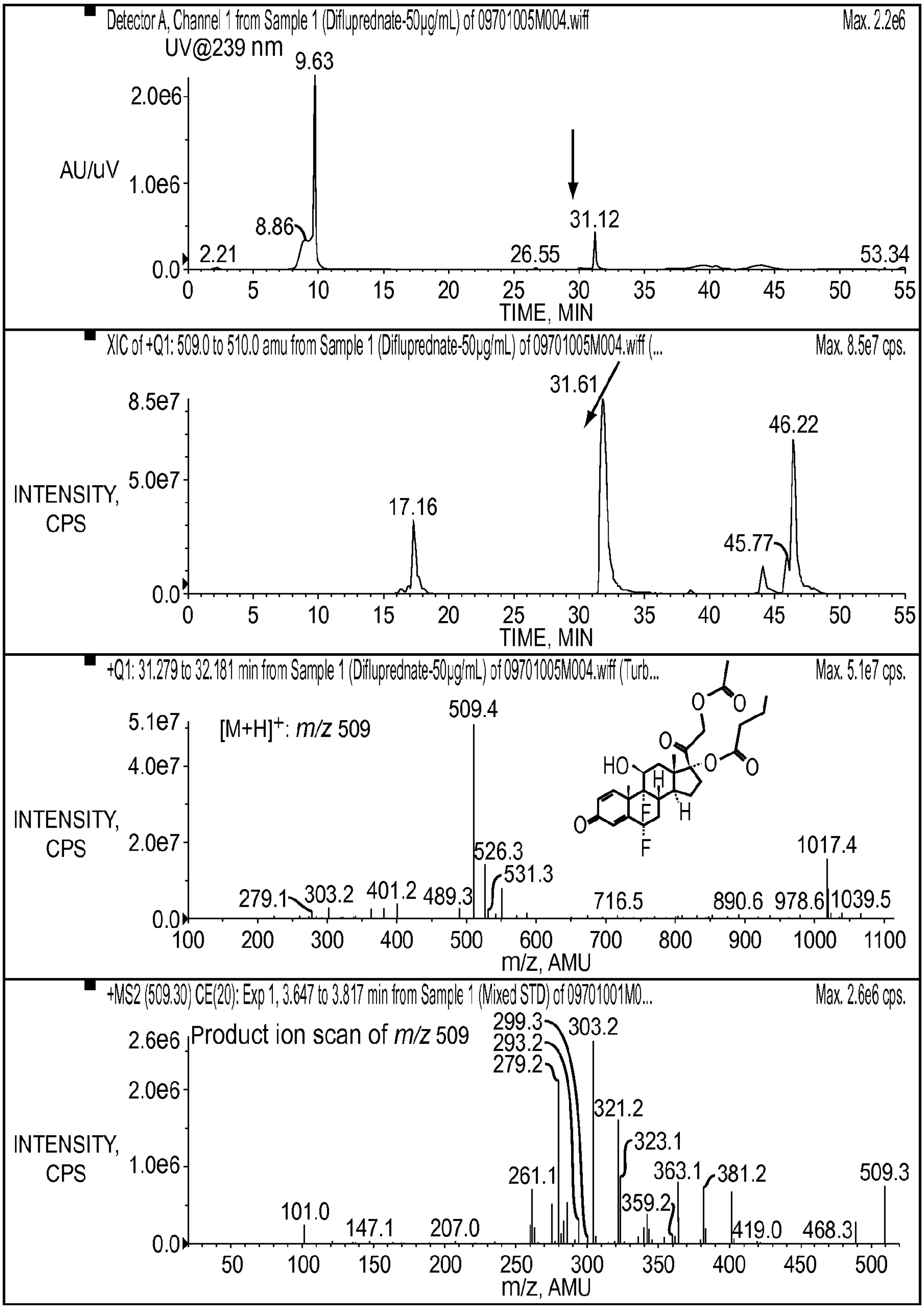


FIG. 4

HPLC/UV Chromatograms of PVP-I at the Concentration of 200 µg/mL for Dexamethasone Sodium Phosphate

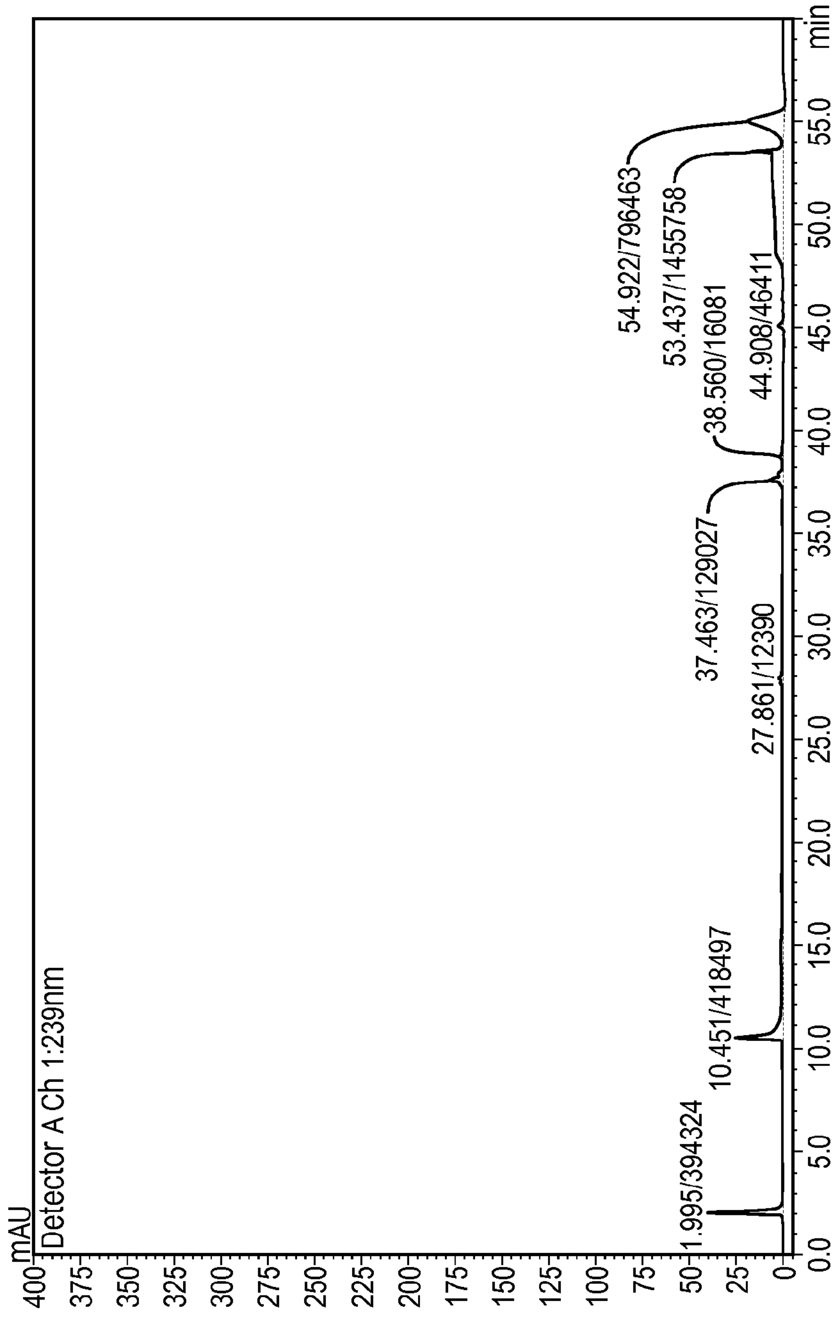


FIG. 5

HPLC/UV Chromatograms of Dexamethasone Sodium Phosphate
in PVP-I for Day 0

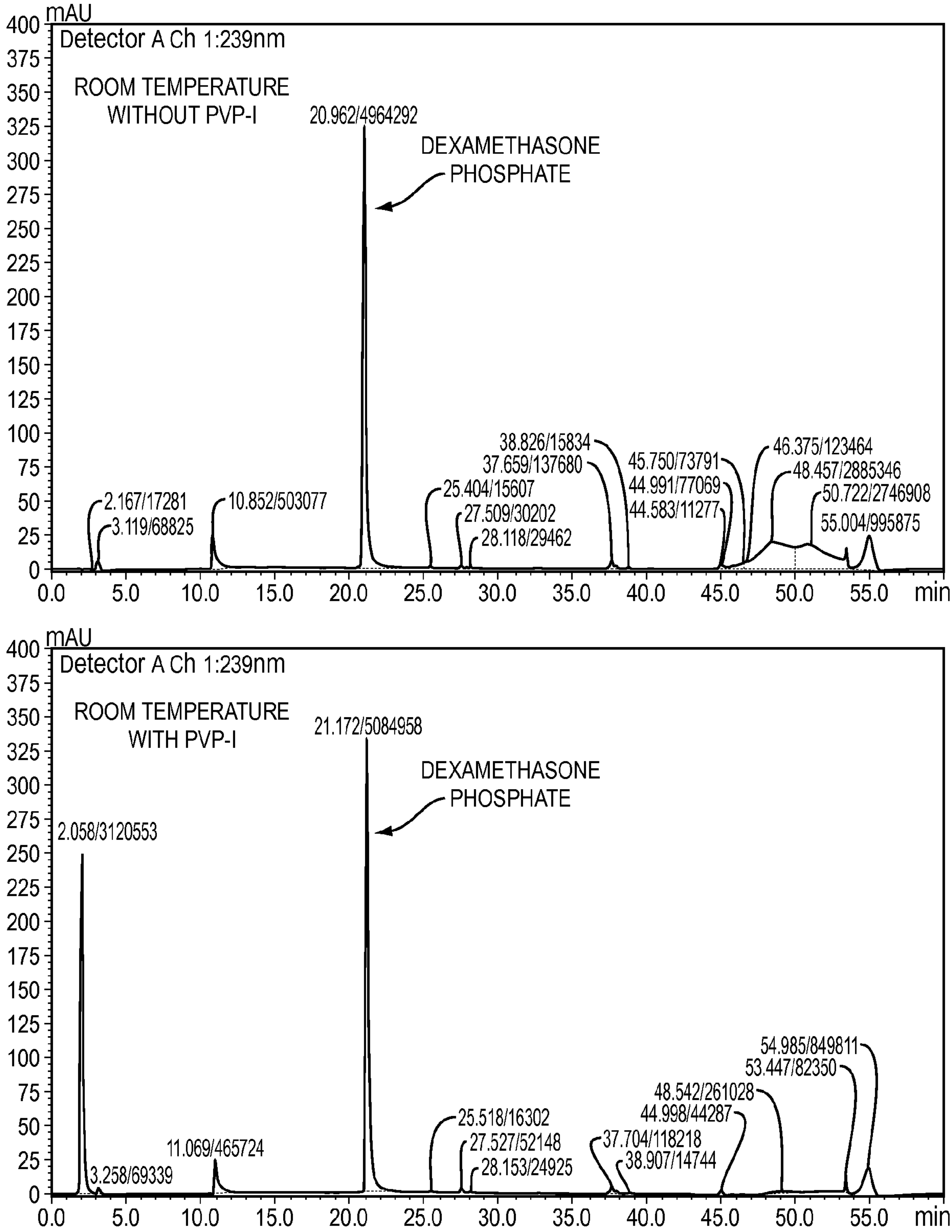


FIG. 6

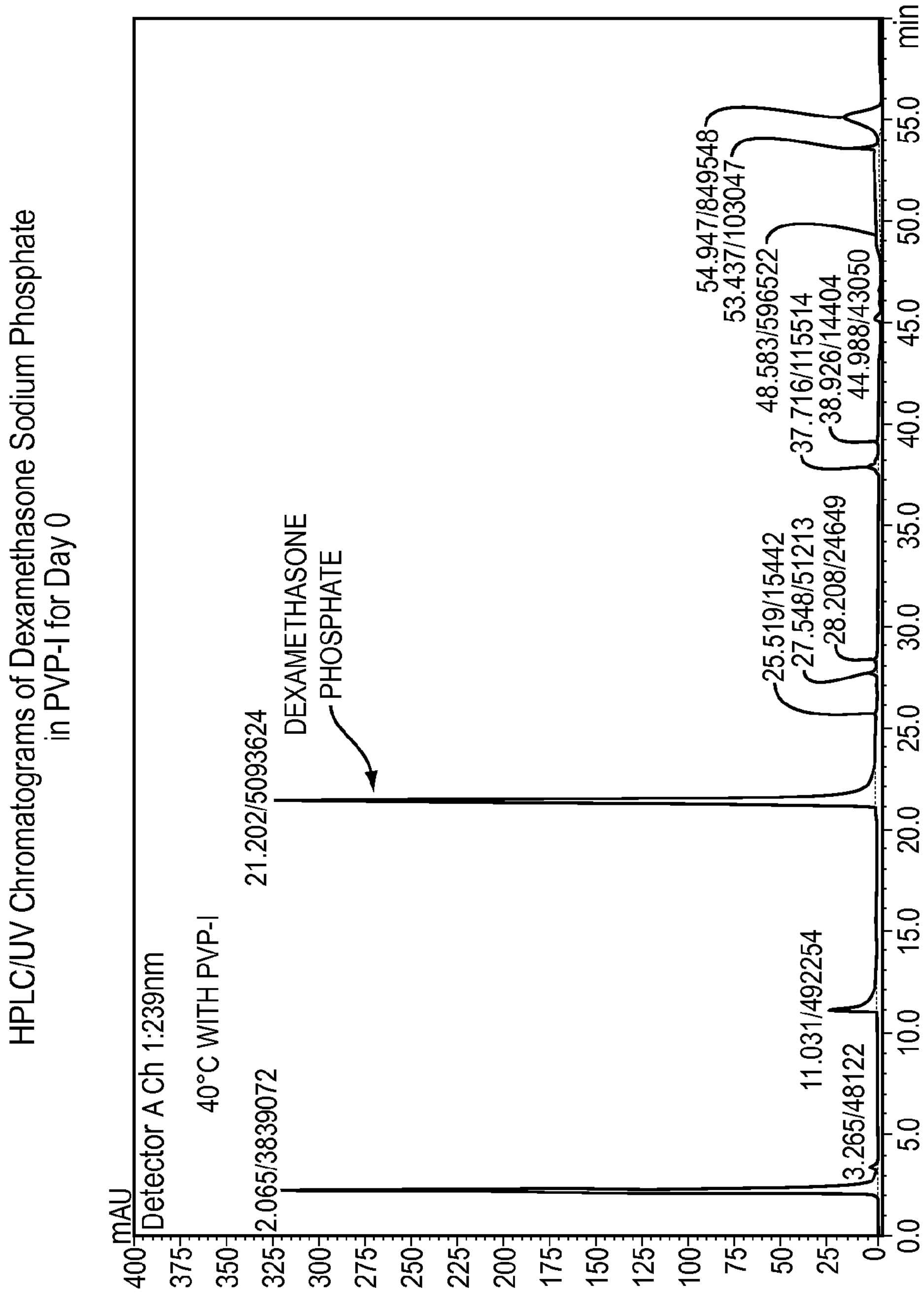
HPLC/UV Chromatograms of Dexamethasone Sodium Phosphate
in PVP-I for Day 0
mAU
Detector A Ch 1:239nm
40°C WITH PVP-I
2.065/3839072
3.265/48122
11.031/492254
21.202/5093624
DEXAMETHASONE
PHOSPHATE
25.519/15442
27.548/51213
28.208/24649
37.716/115514
38.926/14404
44.988/43050
48.583/596522
53.437/103047
54.947/849548
400
375
350
325
300
275
250
225
200
175
150
125
100
75
50
25
0
0.0
5.0
10.0
15.0
20.0
25.0
30.0
35.0
40.0
45.0
50.0
55.0
min

FIG. 7

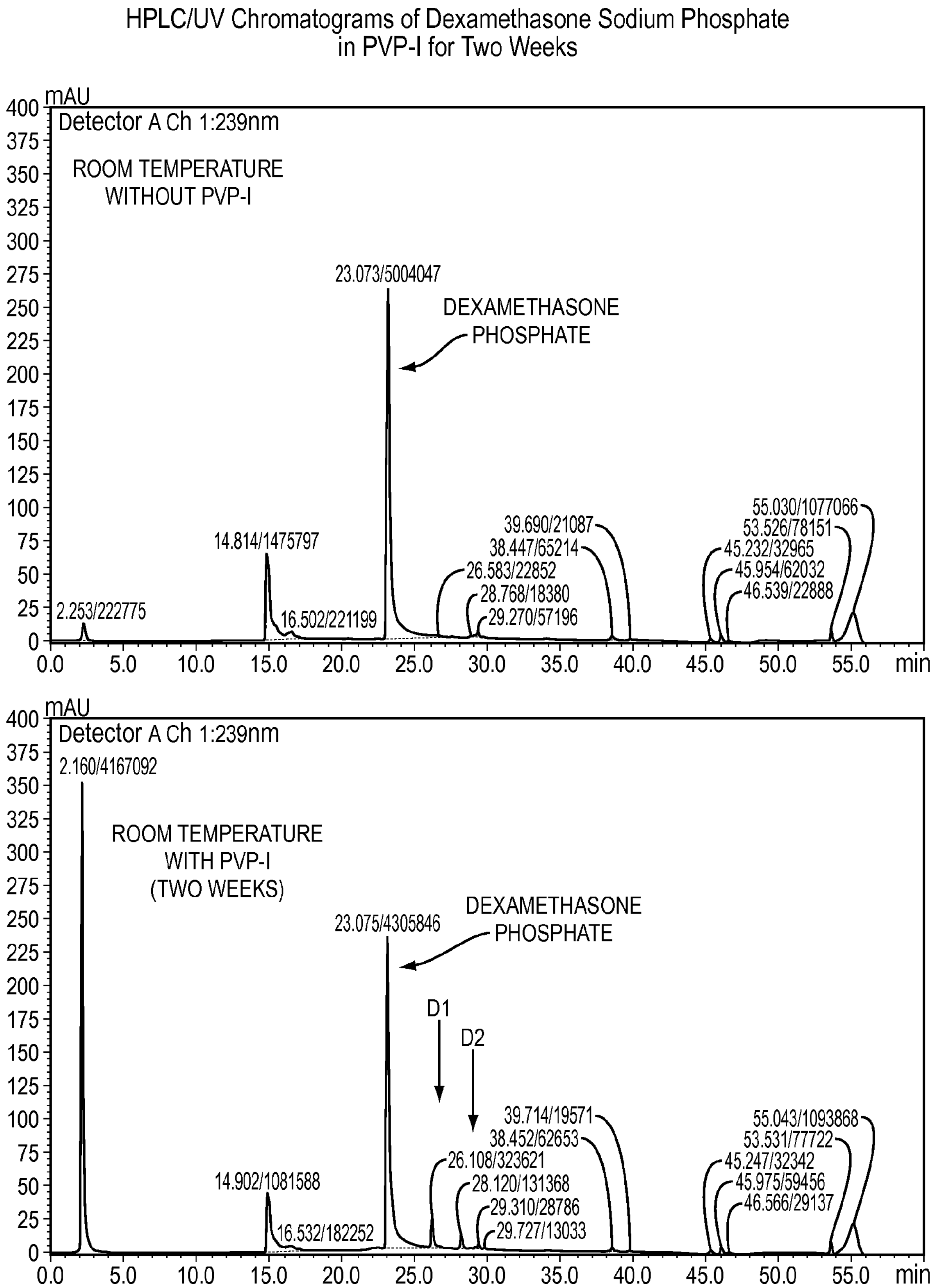
HPLC/UV Chromatograms of Dexamethasone Sodium Phosphate
in PVP-I for Two Weeks
mAU
Detector A Ch 1:239nm
ROOM TEMPERATURE
WITHOUT PVP-I
23.073/5004047
DEXAMETHASONE
PHOSPHATE
14.814/1475797
2.253/222775
16.502/221199
39.690/21087
38.447/65214
26.583/22852
28.768/18380
29.270/57196
55.030/1077066
53.526/78151
45.232/32965
45.954/62032
46.539/22888
min
mAU
Detector A Ch 1:239nm
2.160/4167092
ROOM TEMPERATURE
WITH PVP-I
(TWO WEEKS)
23.075/4305846
DEXAMETHASONE
PHOSPHATE
D1
D2
39.714/19571
38.452/62653
26.108/323621
28.120/131368
29.310/28786
29.727/13033
14.902/1081588
16.532/182252
55.043/1093868
53.531/77722
45.247/32342
45.975/59456
46.566/29137
min

FIG. 8

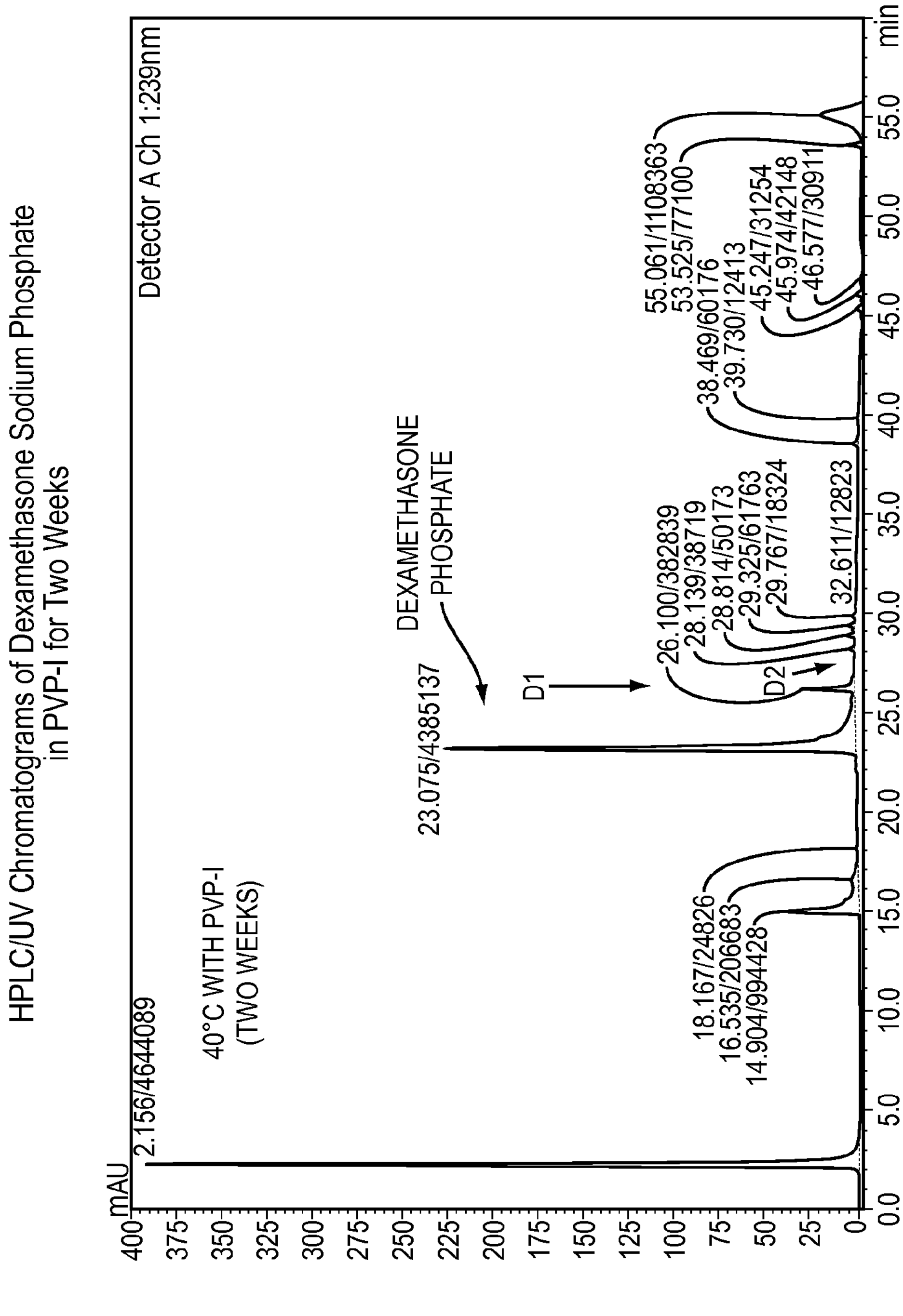
HPLC/UV Chromatograms of Dexamethasone Sodium Phosphate in PVP-I for Two Weeks
Detector A Ch 1:239nm
mAU
400
375
350
325
300
275
250
225
200
175
150
125
100
75
50
25
0
0.0
5.0
10.0
15.0
20.0
25.0
30.0
35.0
40.0
45.0
50.0
55.0
min
2.156/4644089
40°C WITH PVP-I
(TWO WEEKS)
18.167/24826
16.535/206683
14.904/994428
23.075/4385137
DEXAMETHASONE
PHOSPHATE
D1
D2
26.100/382839
28.139/38719
28.814/50173
29.325/61763
29.767/18324
32.611/12823
38.469/60176
39.730/12413
45.247/31254
45.974/42148
46.577/30911
55.061/1108363
53.525/77100

FIG. 9

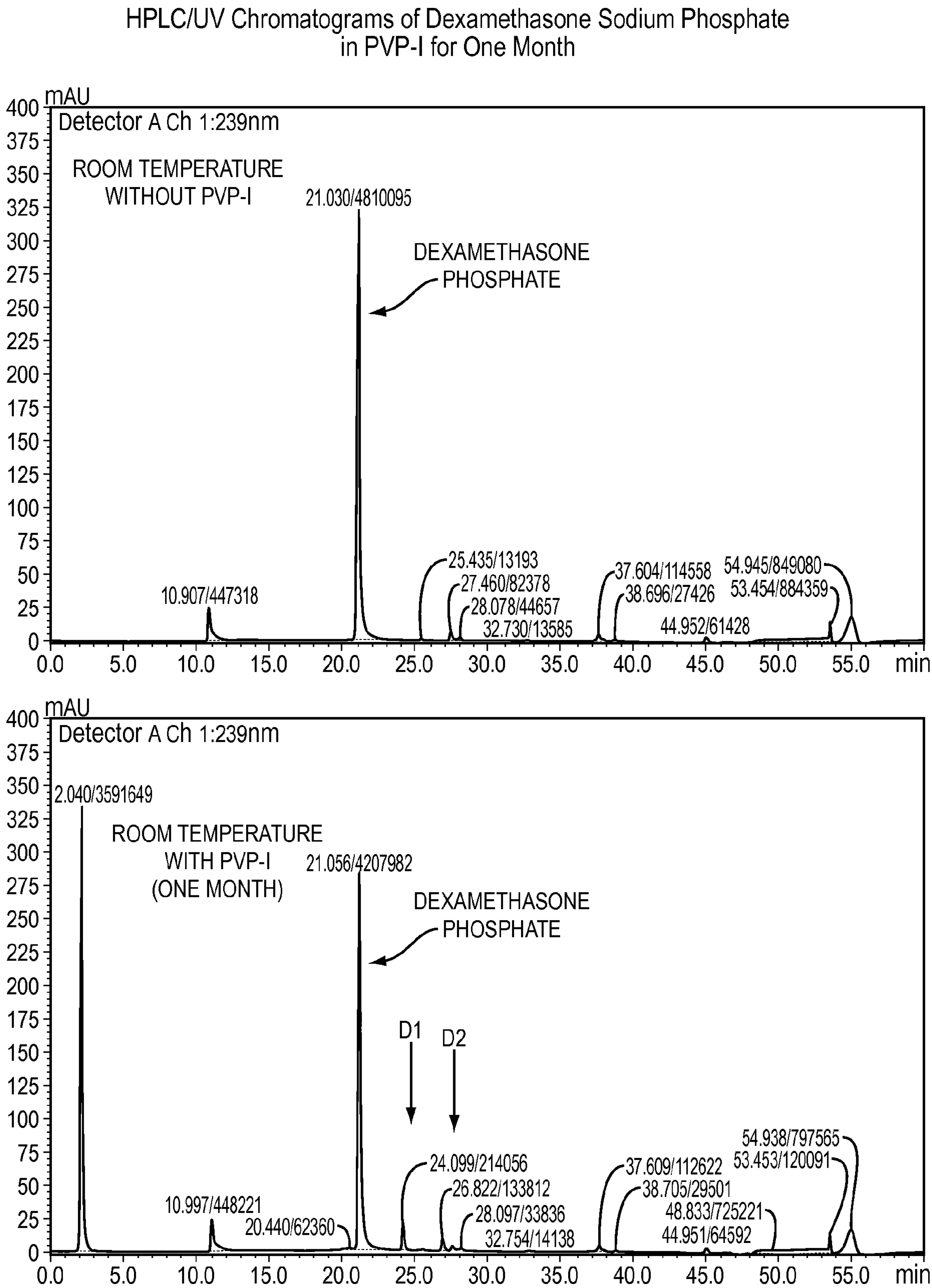
HPLC/UV Chromatograms of Dexamethasone Sodium Phosphate in PVP-I for One Month
mAU
Detector A Ch 1:239nm
ROOM TEMPERATURE WITHOUT PVP-I
21.030/4810095
DEXAMETHASONE PHOSPHATE
10.907/447318
25.435/13193
27.460/82378
28.078/44657
32.730/13585
37.604/114558
38.696/27426
44.952/61428
54.945/849080
53.454/884359
min
mAU
Detector A Ch 1:239nm
2.040/3591649
ROOM TEMPERATURE WITH PVP-I (ONE MONTH)
21.056/4207982
DEXAMETHASONE PHOSPHATE
D1
D2
10.997/448221
20.440/62360
24.099/214056
26.822/133812
28.097/33836
32.754/14138
37.609/112622
38.705/29501
48.833/725221
44.951/64592
54.938/797565
53.453/120091
min

FIG. 10

HPLC/UV Chromatograms of Dexamethasone Sodium Phosphate
in PVP-I for One Month

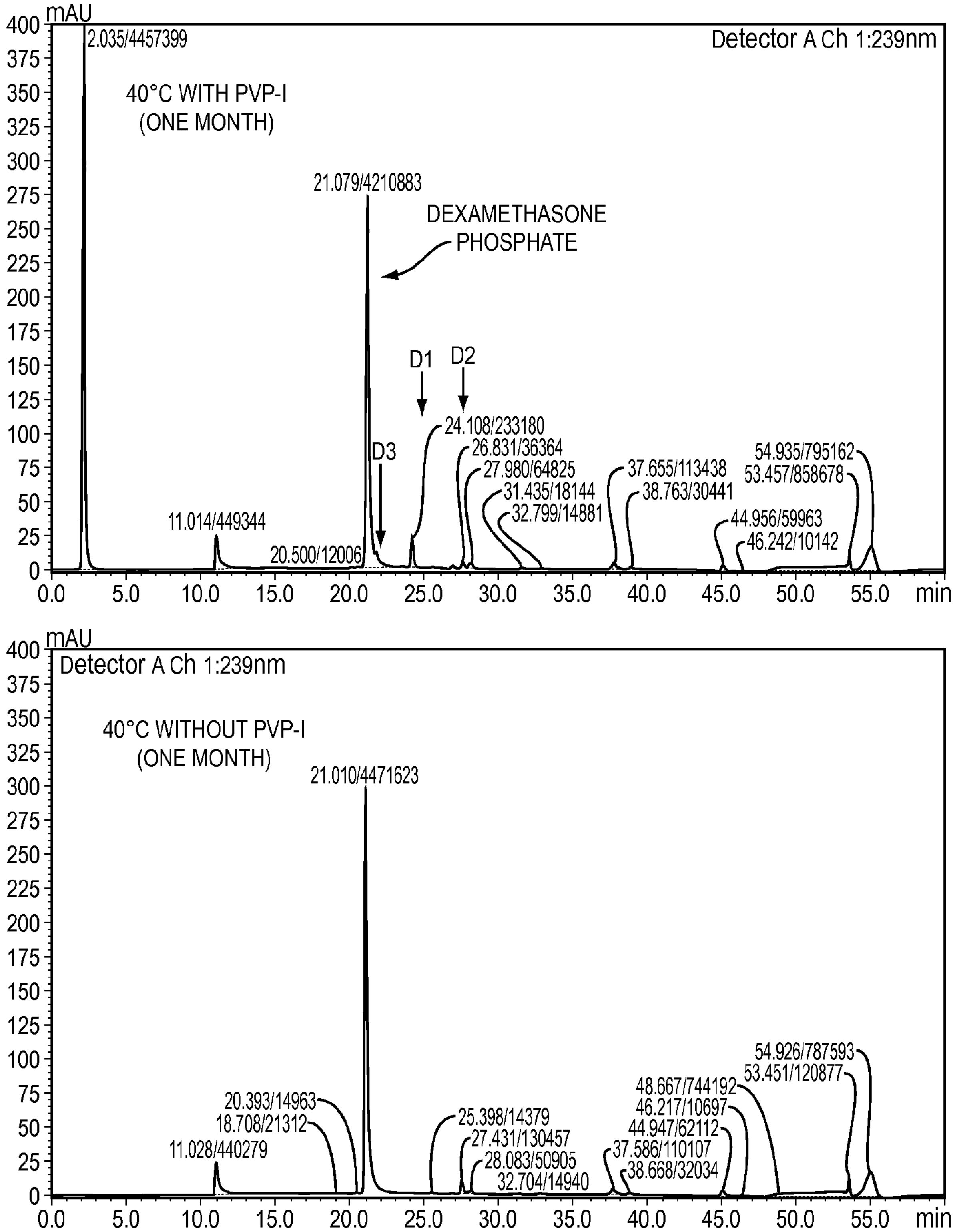


FIG. 11

HPLC/UV Chromatograms (Expanded) of Dexamethasone Sodium Phosphate in PVP-I for One Month

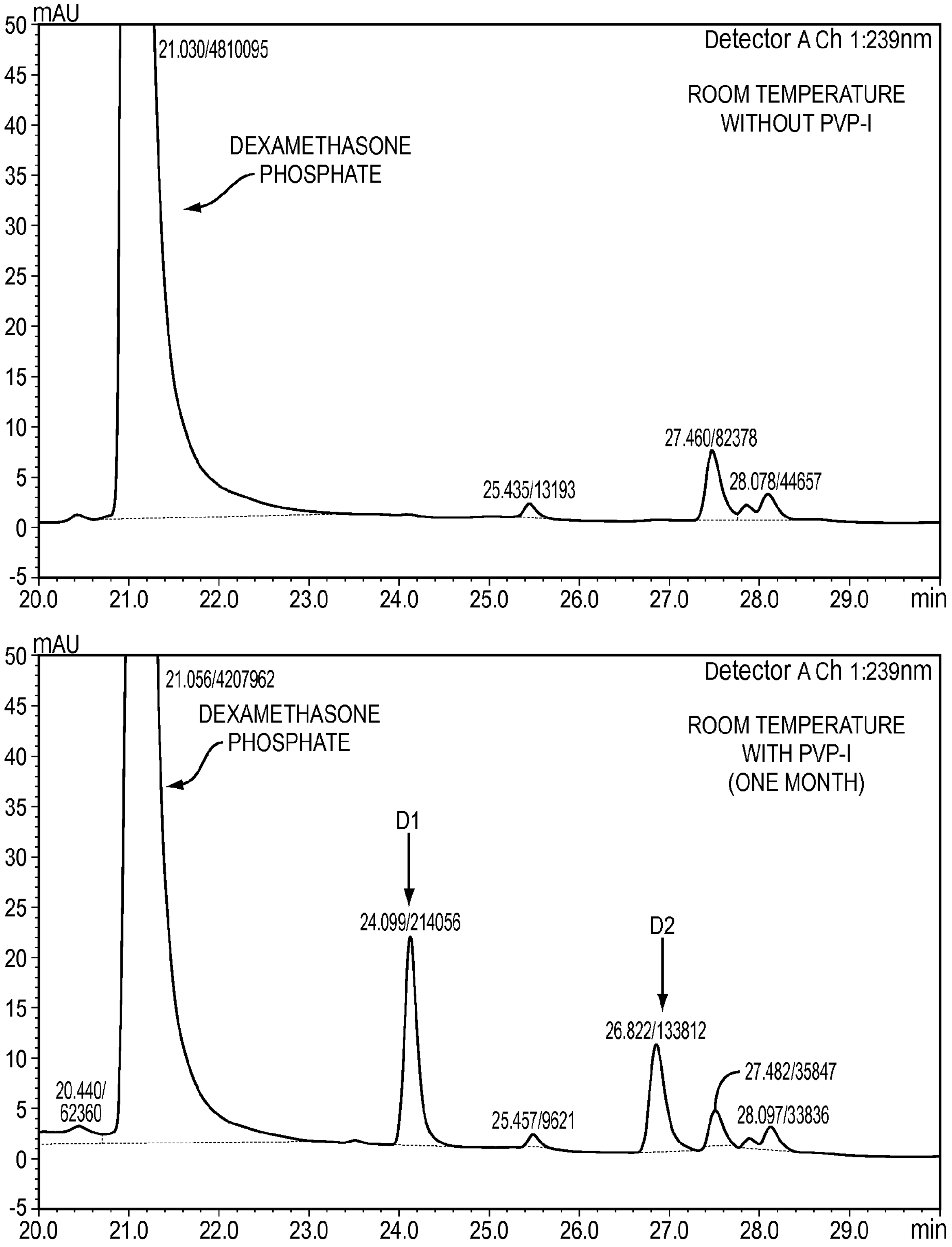


FIG. 12

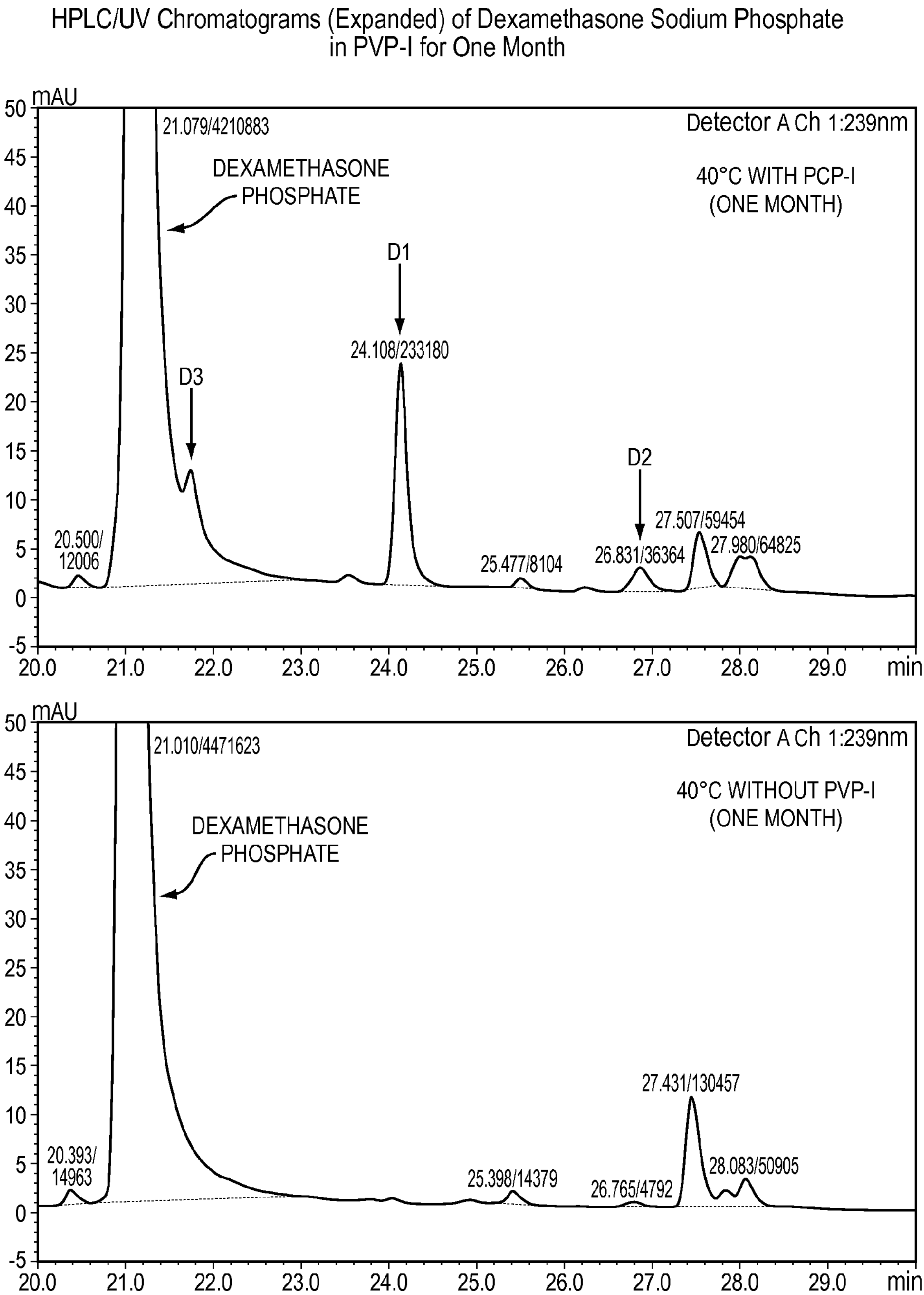
HPLC/UV Chromatograms (Expanded) of Dexamethasone Sodium Phosphate in PVP-I for One Month
mAU
Detector A Ch 1:239nm
40°C WITH PCP-I
(ONE MONTH)
21.079/4210883
DEXAMETHASONE PHOSPHATE
D1
24.108/233180
D3
D2
20.500/ 12006
25.477/8104
26.831/36364
27.507/59454
27.980/64825
min
mAU
Detector A Ch 1:239nm
40°C WITHOUT PVP-I
(ONE MONTH)
21.010/4471623
DEXAMETHASONE PHOSPHATE
27.431/130457
20.393/ 14963
25.398/14379
26.765/4792
28.083/50905
min

FIG. 13

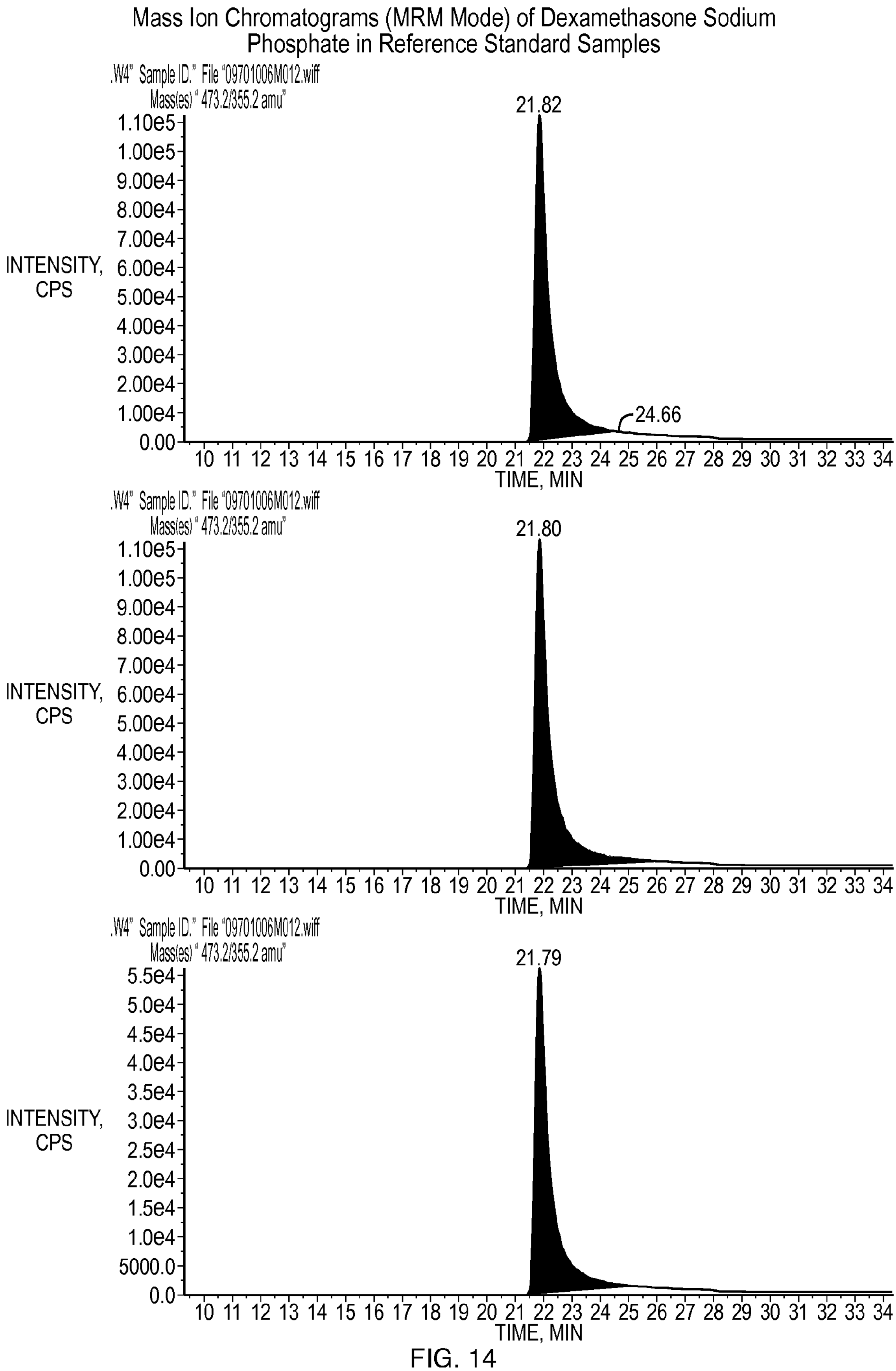
Mass Ion Chromatograms (MRM Mode) of Dexamethasone Sodium Phosphate in Reference Standard Samples
.W4" Sample ID." File "09701006M012.wiff
Mass(es) " 473.2/355.2 amu"
21.82
24.66
INTENSITY, CPS
1.10e5
1.00e5
9.00e4
8.00e4
7.00e4
6.00e4
5.00e4
4.00e4
3.00e4
2.00e4
1.00e4
0.00
10 11 12 13 14 15 16 17 18 19 20 21 22 23 24 25 26 27 28 29 30 31 32 33 34
TIME, MIN
.W4" Sample ID." File "09701006M012.wiff
Mass(es) " 473.2/355.2 amu"
21.80
INTENSITY, CPS
1.10e5
1.00e5
9.00e4
8.00e4
7.00e4
6.00e4
5.00e4
4.00e4
3.00e4
2.00e4
1.00e4
0.00
10 11 12 13 14 15 16 17 18 19 20 21 22 23 24 25 26 27 28 29 30 31 32 33 34
TIME, MIN
.W4" Sample ID." File "09701006M012.wiff
Mass(es) " 473.2/355.2 amu"
21.79
INTENSITY, CPS
5.5e4
5.0e4
4.5e4
4.0e4
3.5e4
3.0e4
2.5e4
2.0e4
1.5e4
1.0e4
5000.0
0.0
10 11 12 13 14 15 16 17 18 19 20 21 22 23 24 25 26 27 28 29 30 31 32 33 34
TIME, MIN

FIG. 14

Mass Ion Chromatograms (MRM Mode) of Dexamethasone Sodium Phosphate in One Month Room Temperature Stability Sample in the Presence of PVP-I

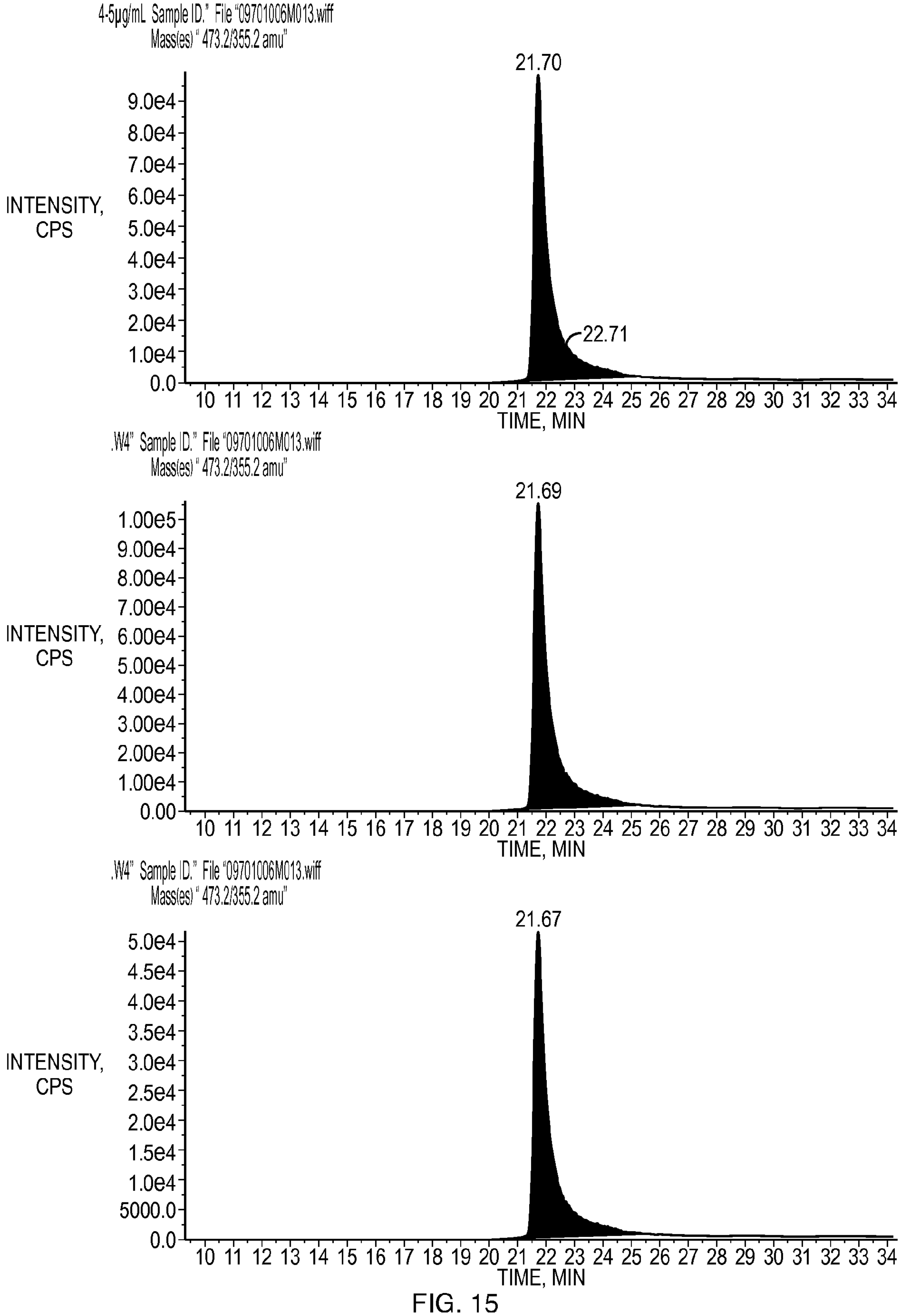


FIG. 15

Mass Ion Chromatograms (MRM Mode) of Dexamethasone Sodium Phosphate in One Month 40°C Stability Sample in the Presence of PVP-I

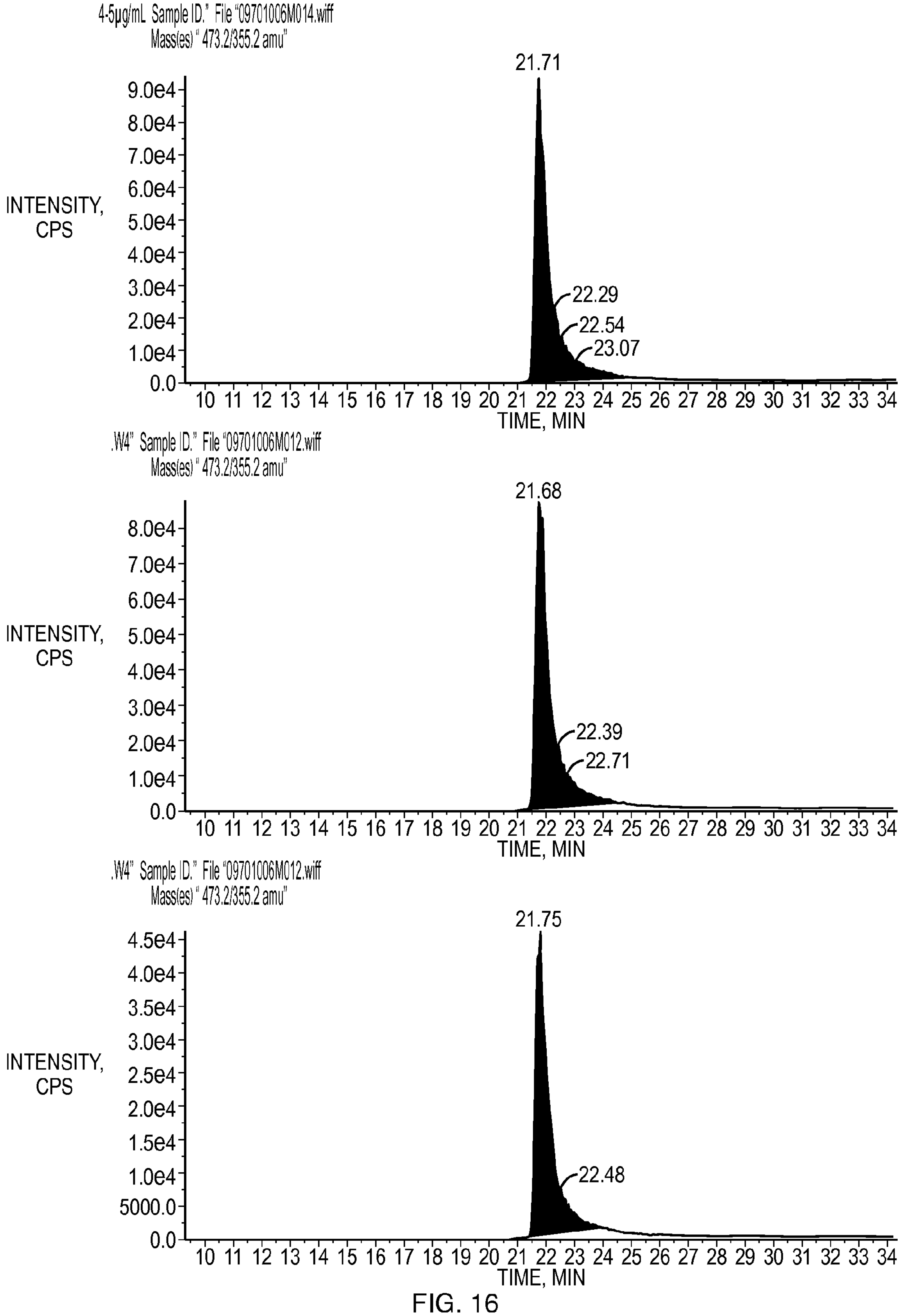


FIG. 16

HPLC/UV Chromatograms of PVP-I at the Concentration of 20 µg/mL for Prednisolone Acetate

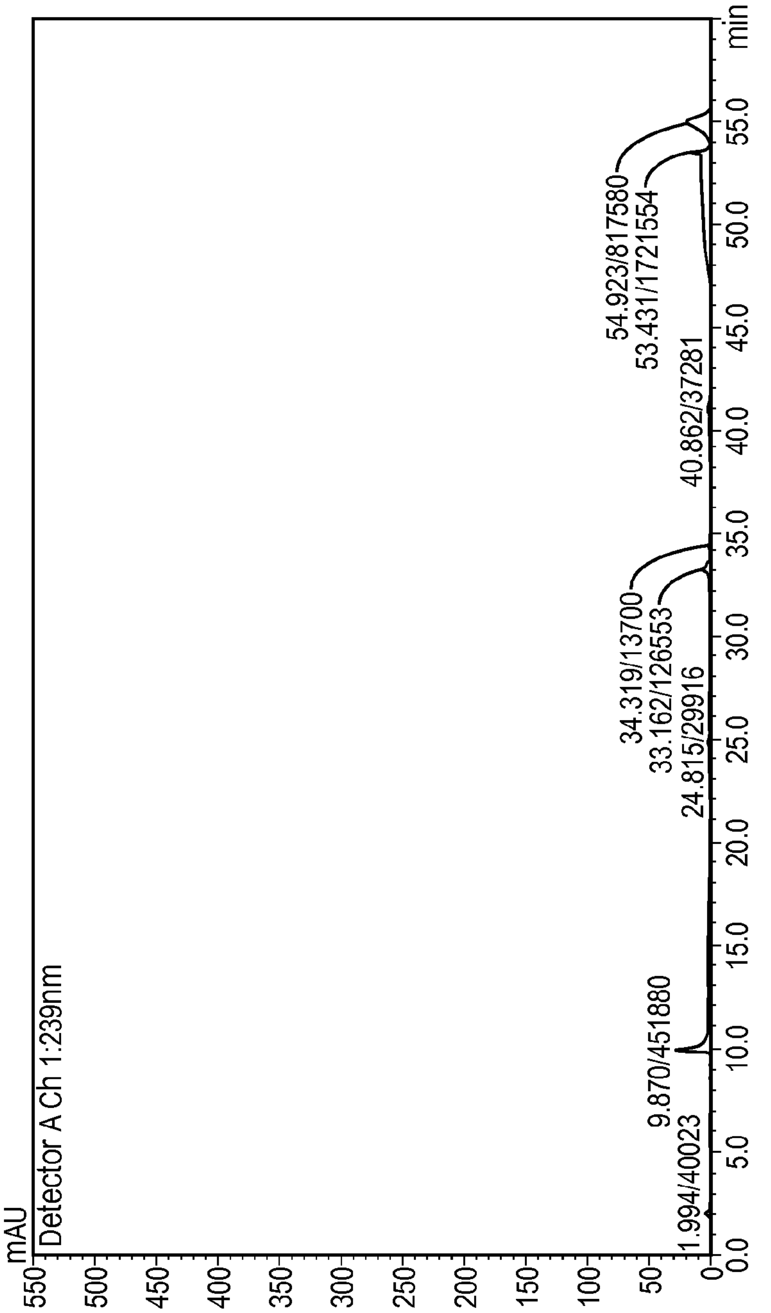


FIG. 17

HPLC/UV Chromatograms of Prednisolone Acetate in PVP-I for Day 0

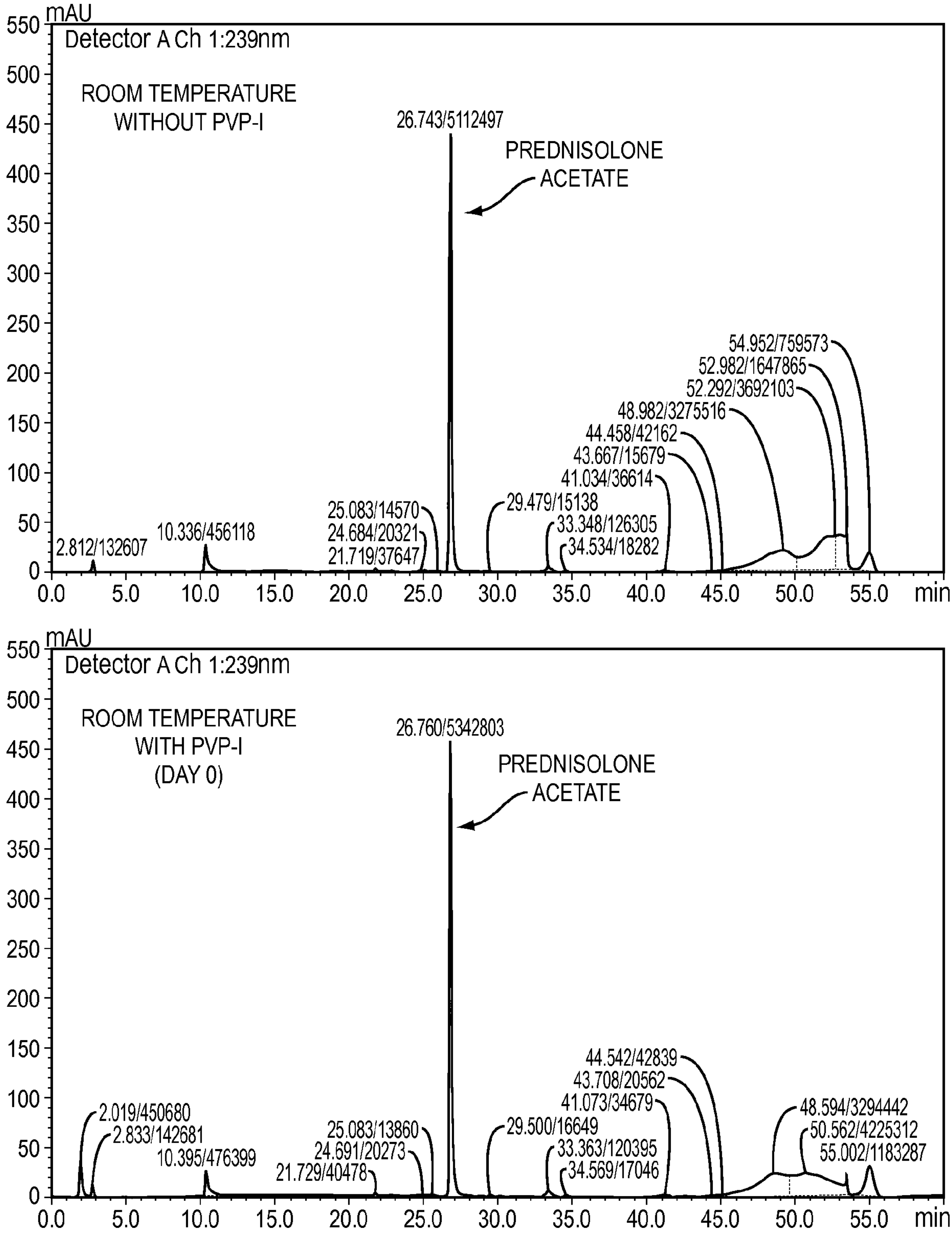


FIG. 18

HPLC/UV Chromatograms of Prednisolone Acetate in PVP-I for Day 0

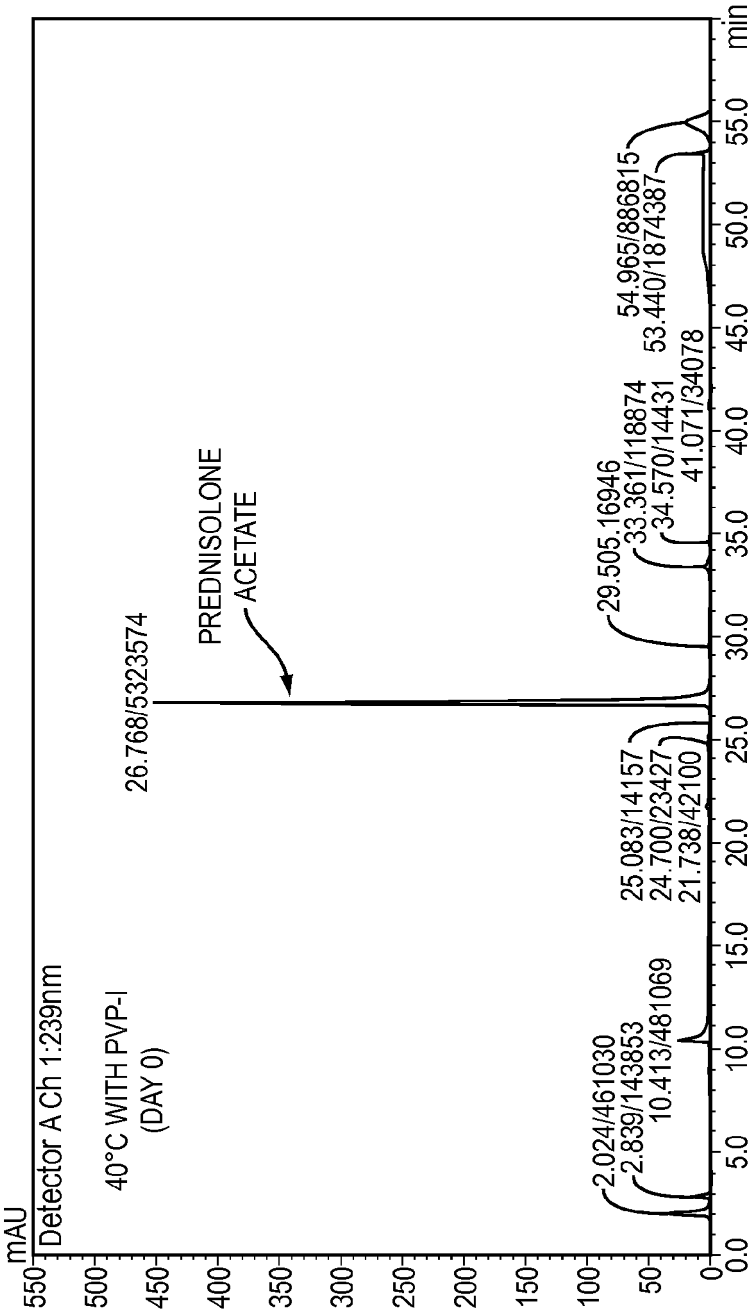


FIG. 19

HPLC/UV Chromatograms of Prednisolone Acetate in PVP-I for Two Weeks

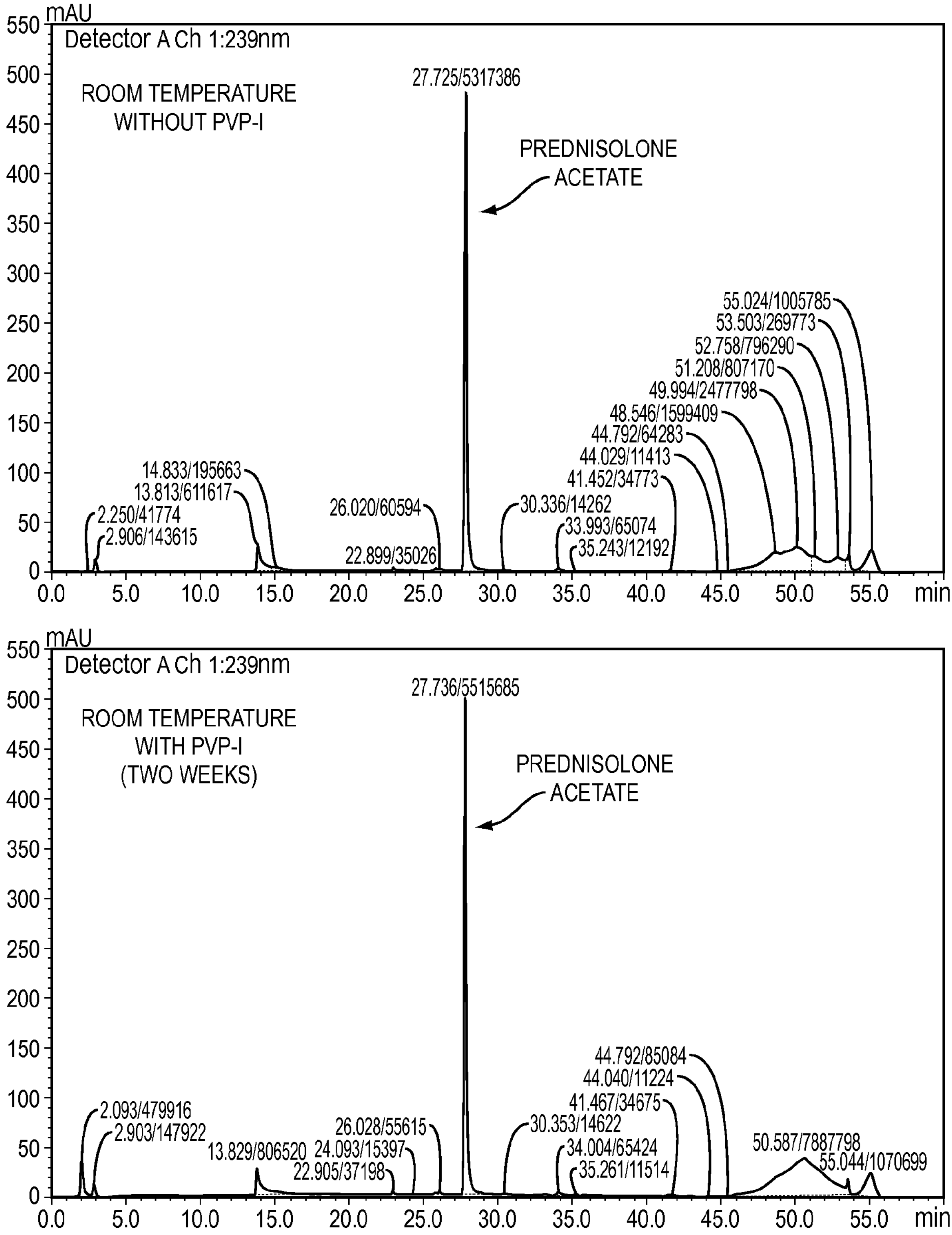


FIG. 20

HPLC/UV Chromatograms of Prednisolone Acetate in PVP-I for Two Weeks

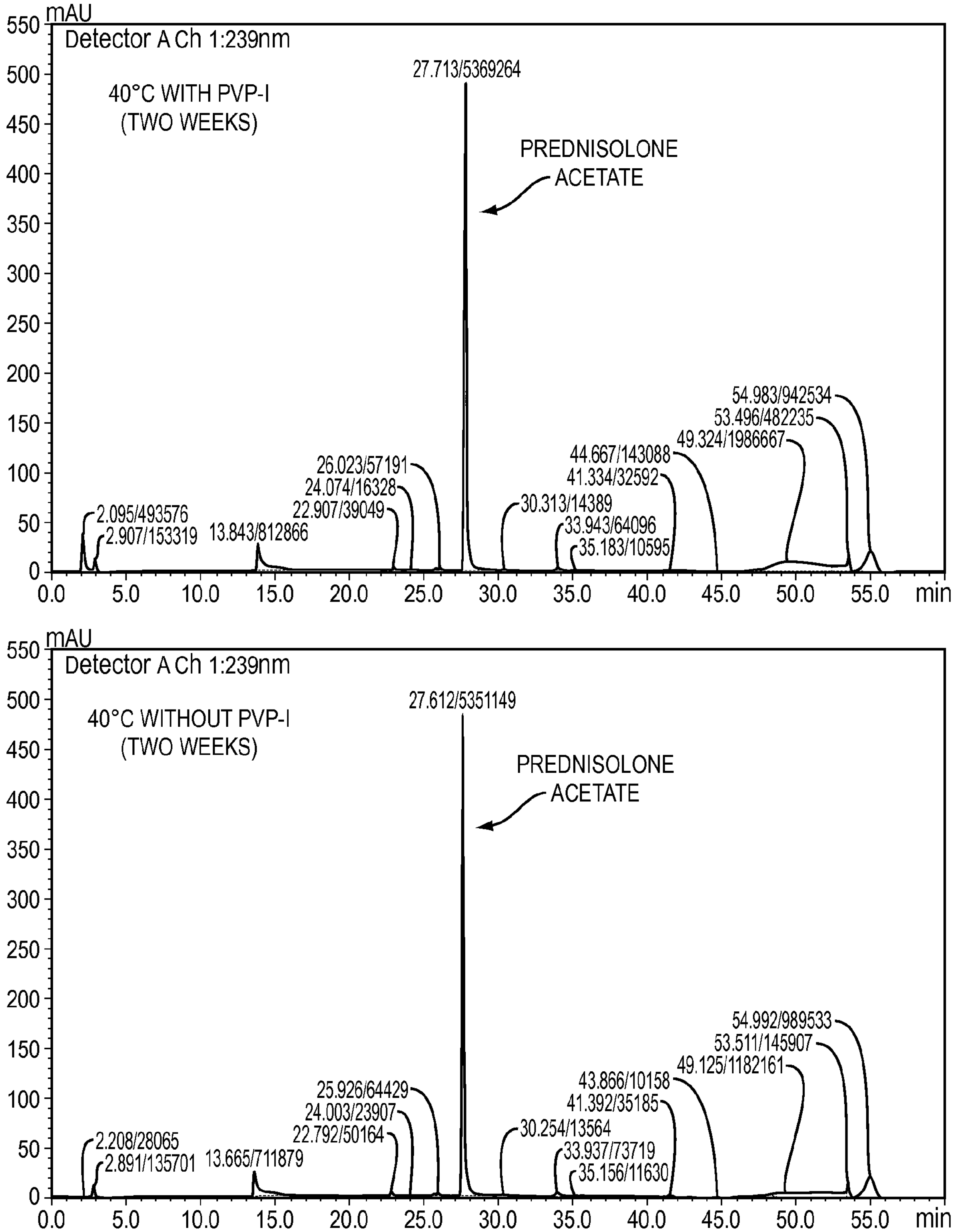


FIG. 21

HPLC/UV Chromatograms of Prednisolone Acetate in PVP-I for One Month

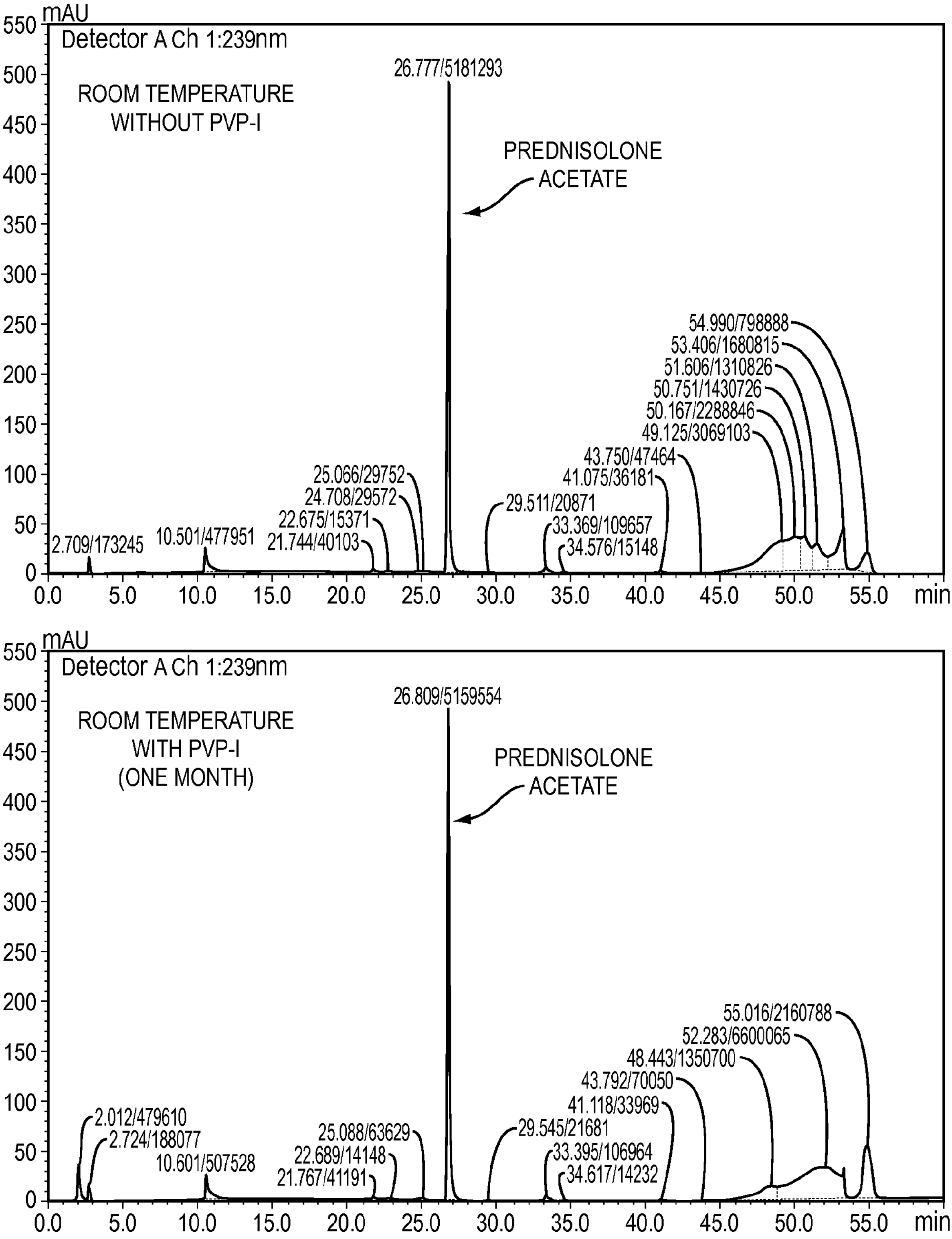


FIG. 22

HPLC/UV Chromatograms of Prednisolone Acetate in PVP-I for One Month

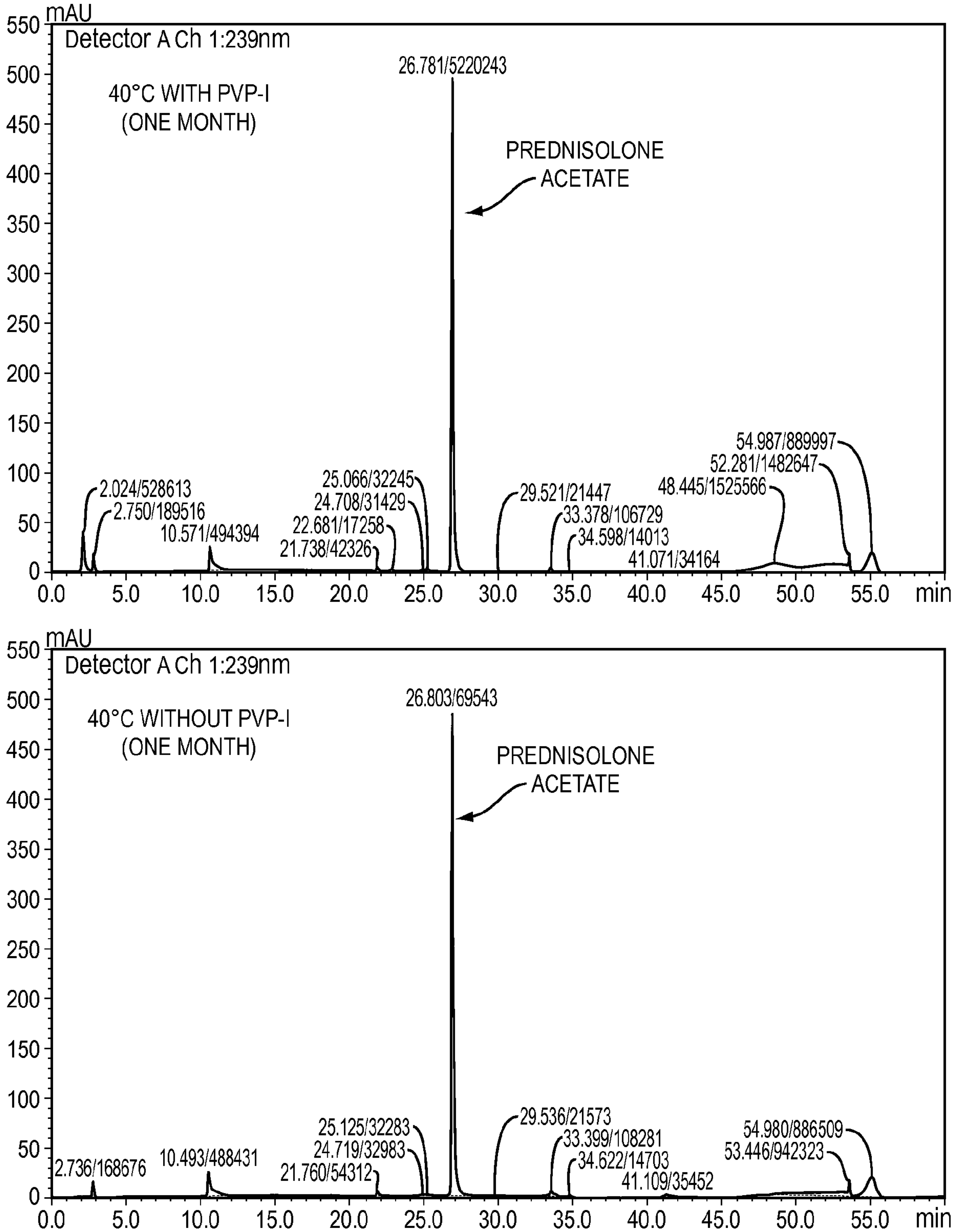


FIG. 23

Mass Ion Chromatograms (MRM Mode) of Prednisolone Acetate in Reference Standard Samples

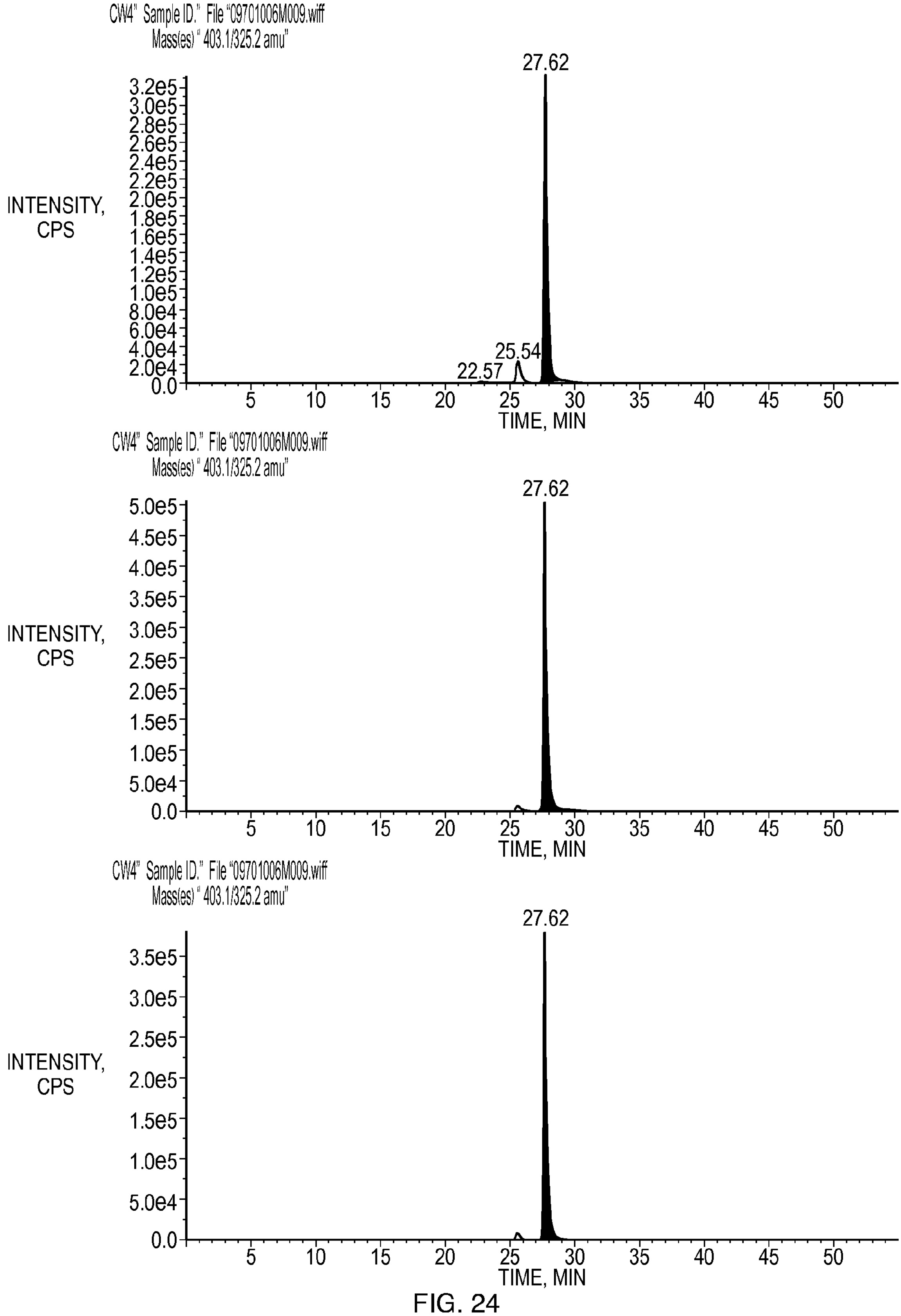


FIG. 24

Mass Ion Chromatograms (MRM Mode) of Prednisolone Acetate in One Month Room Temperature Stability Sample in the Presence of PVP-1

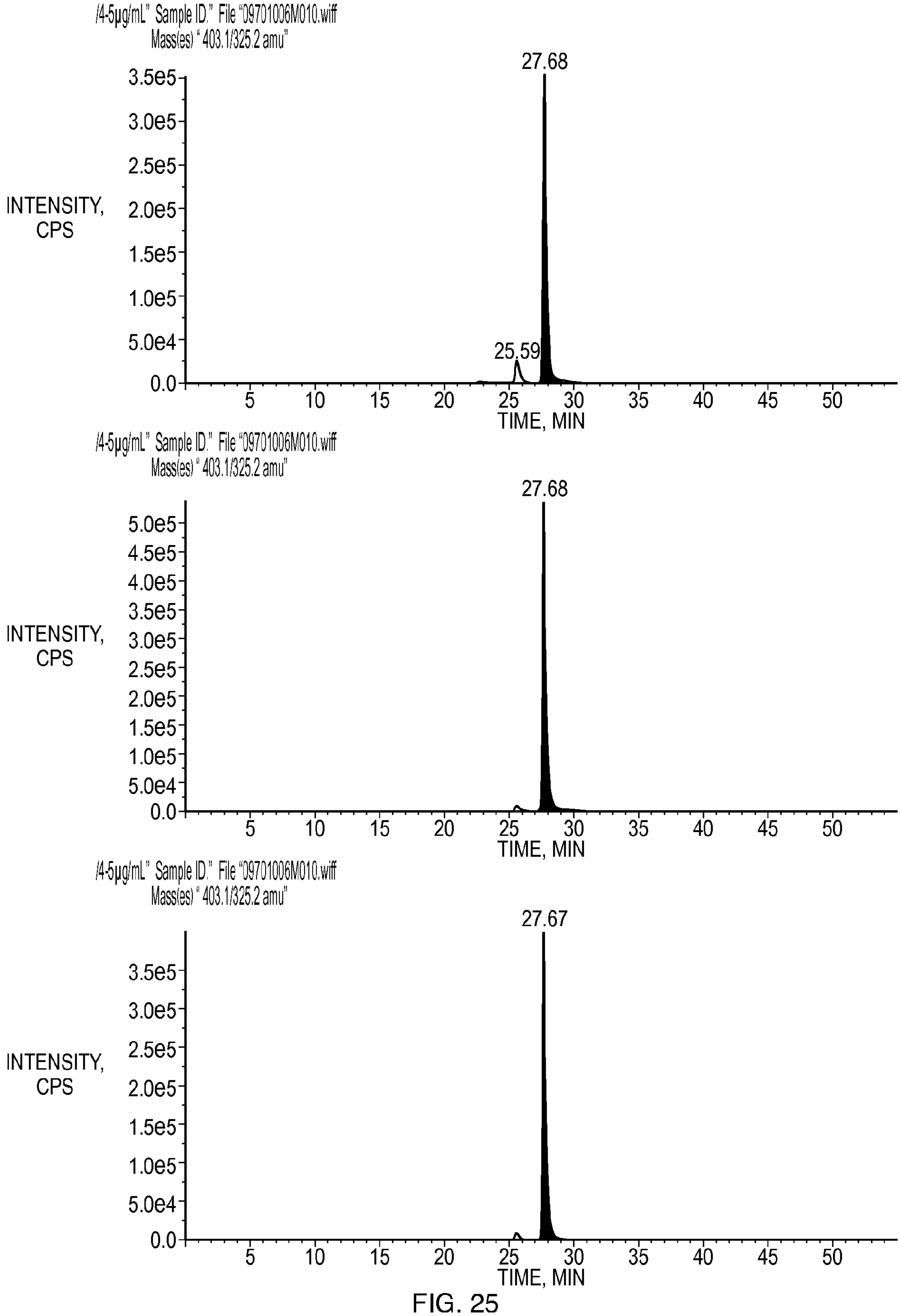


FIG. 25

Mass Ion Chromatograms (MRM Mode) of Prednisolone Acetate in One Month 40°C Stability Sample in the Presence of PVP-I

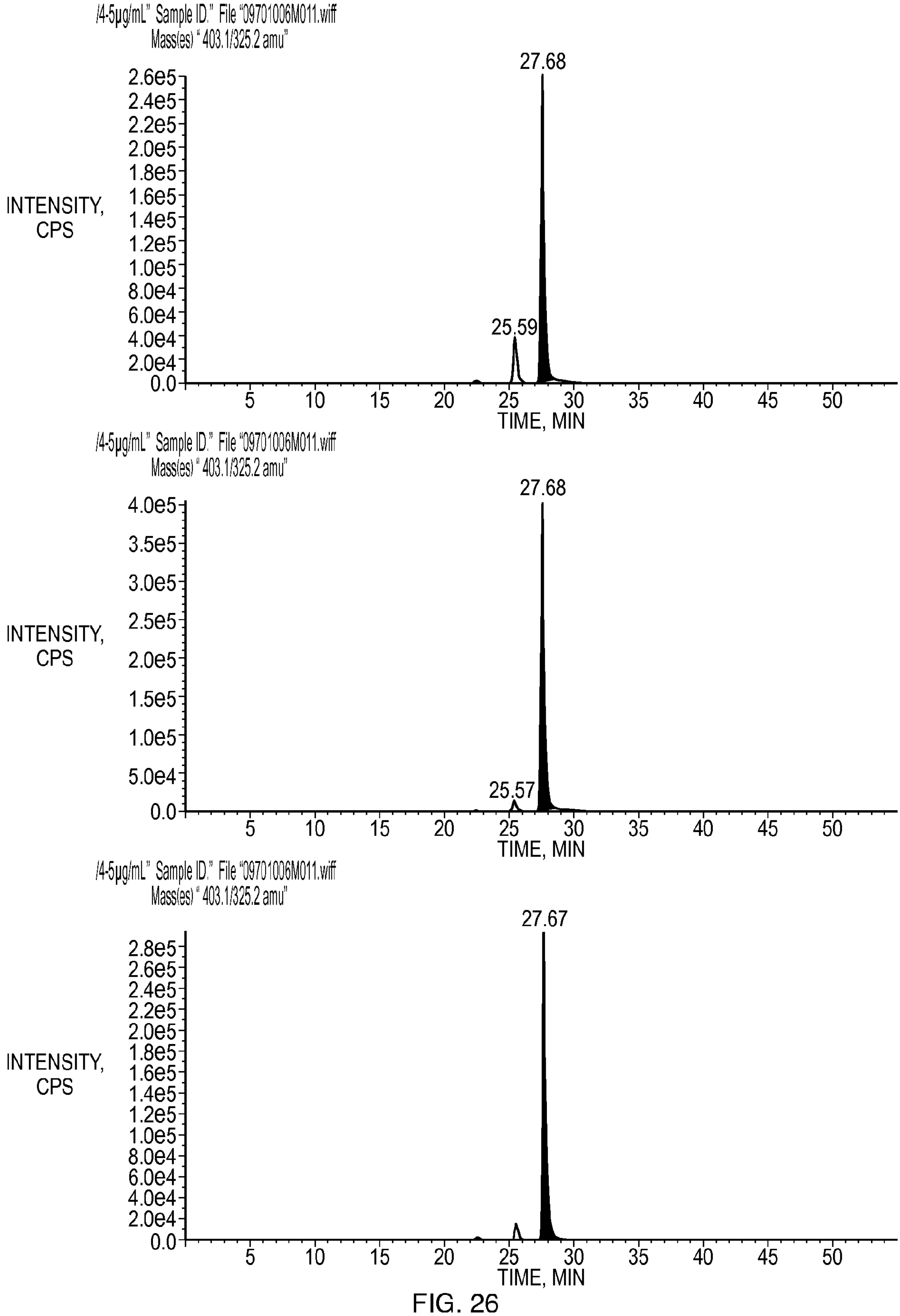


FIG. 26

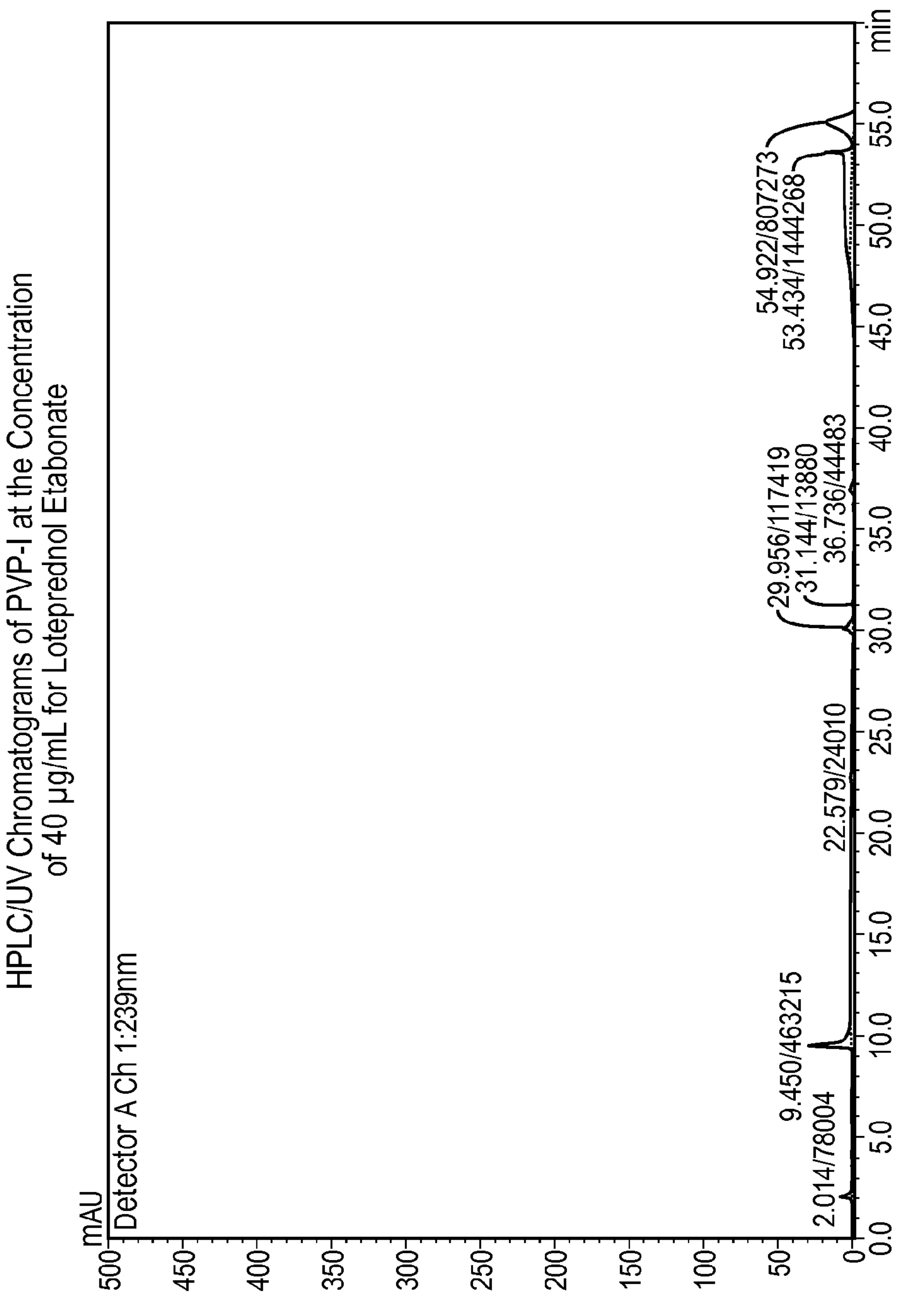
HPLC/UV Chromatograms of PVP-I at the Concentration
of 40 µg/mL for Loteprednol Etabonate
mAU
Detector A Ch 1:239nm
500
450
400
350
300
250
200
150
100
50
0
2.014/78004
9.450/463215
22.579/24010
29.956/117419
31.144/13880
36.736/44483
54.922/807273
53.434/1444268
0.0
5.0
10.0
15.0
20.0
25.0
30.0
35.0
40.0
45.0
50.0
55.0
min

FIG. 27

HPLC/UV Chromatograms of Loteprednol Etabonate in PVP-I for Day 0

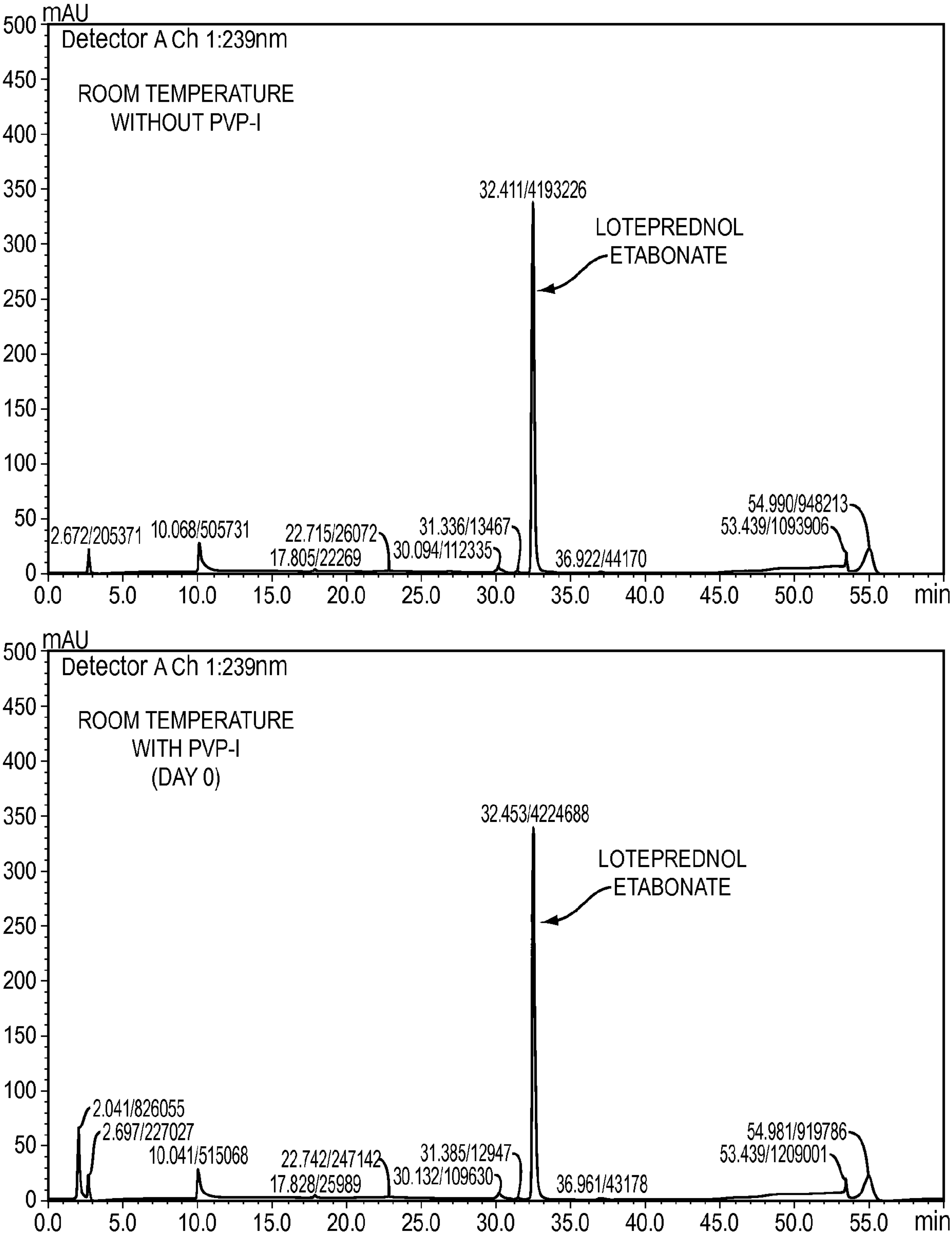


FIG. 28

HPLC/UV Chromatograms of Loteprednol Etabonate in PVP-I for Day 0

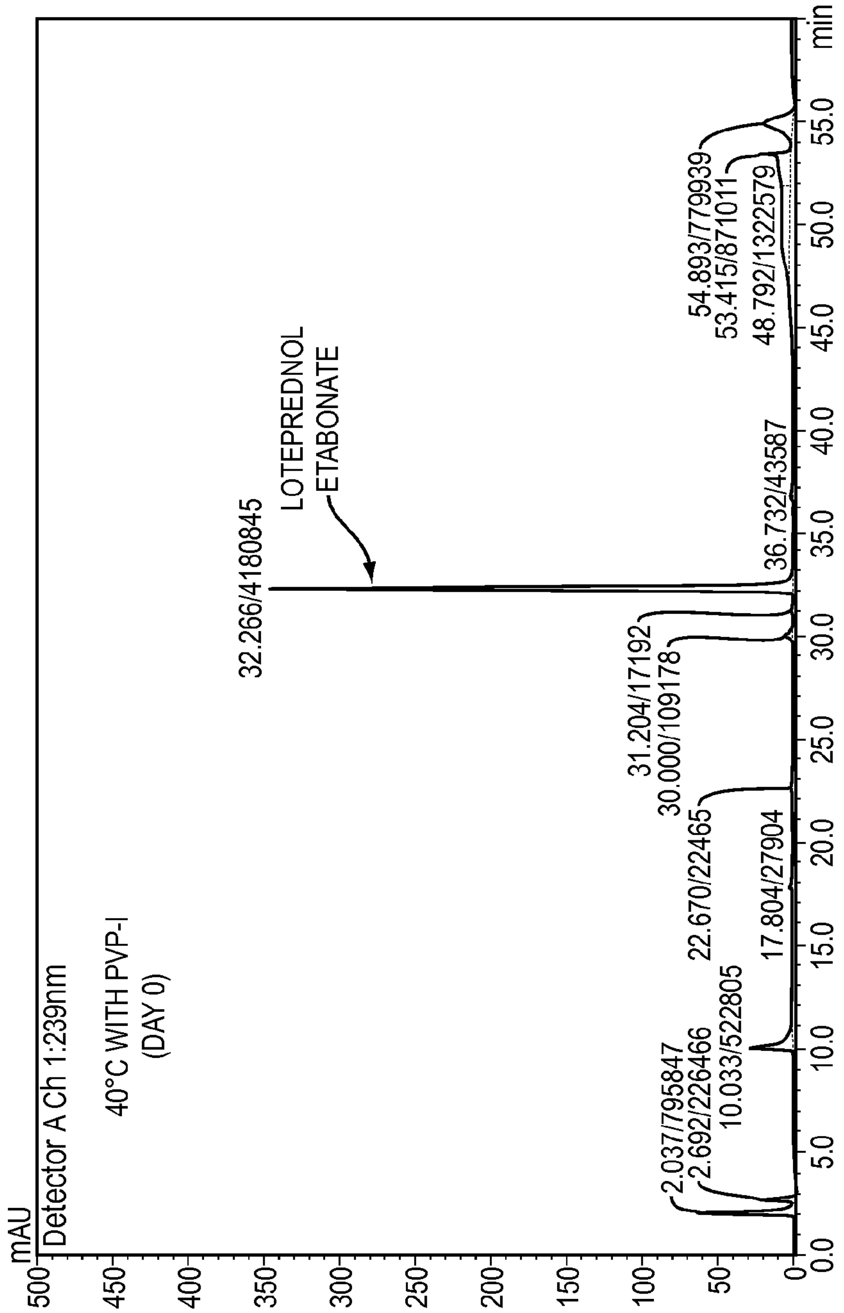


FIG. 29

HPLC/UV Chromatograms of Loteprednol Etabonate in PVP-I for Two Weeks

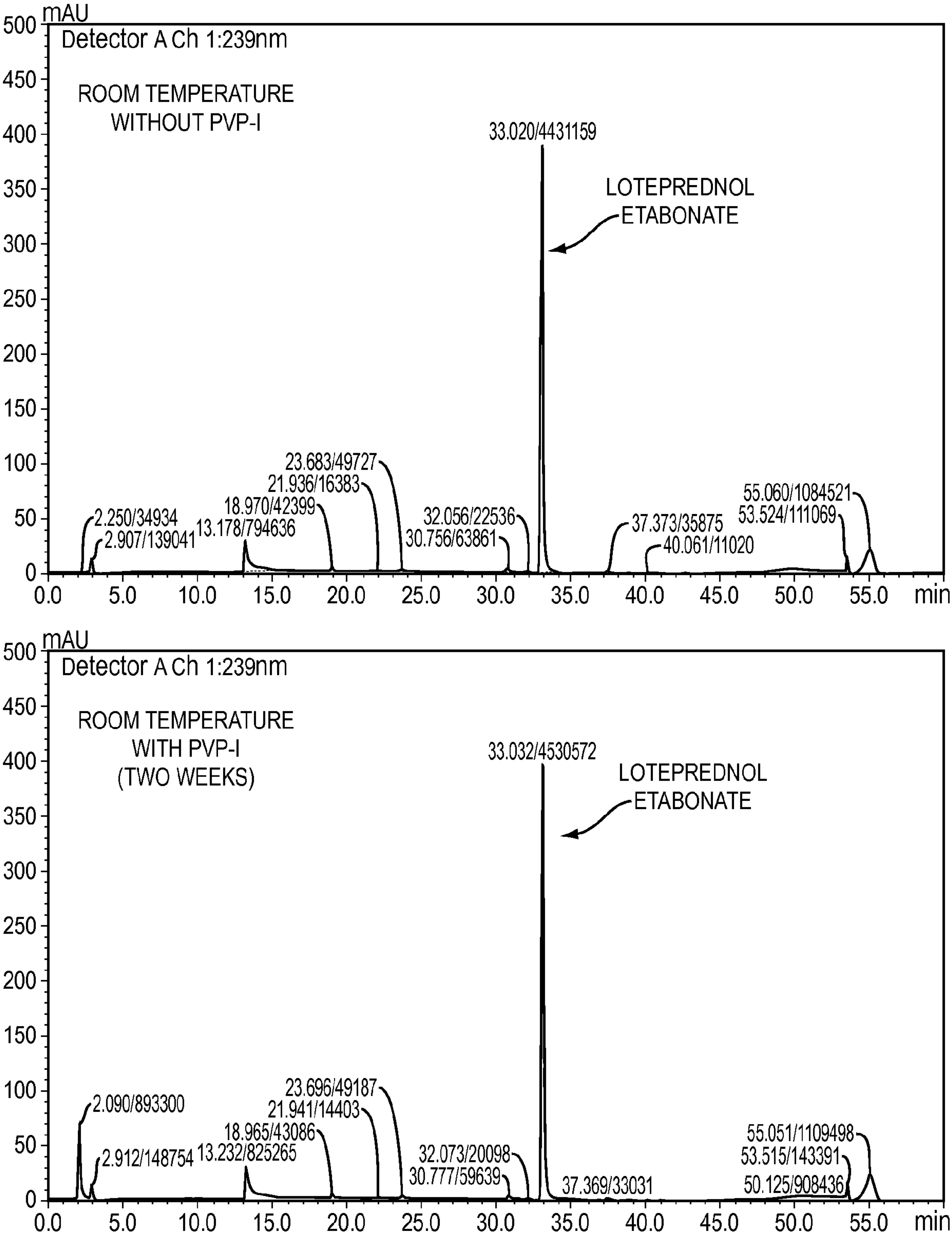


FIG. 30

HPLC/UV Chromatograms of Loteprednol Etabonate in PVP-I for Two Weeks

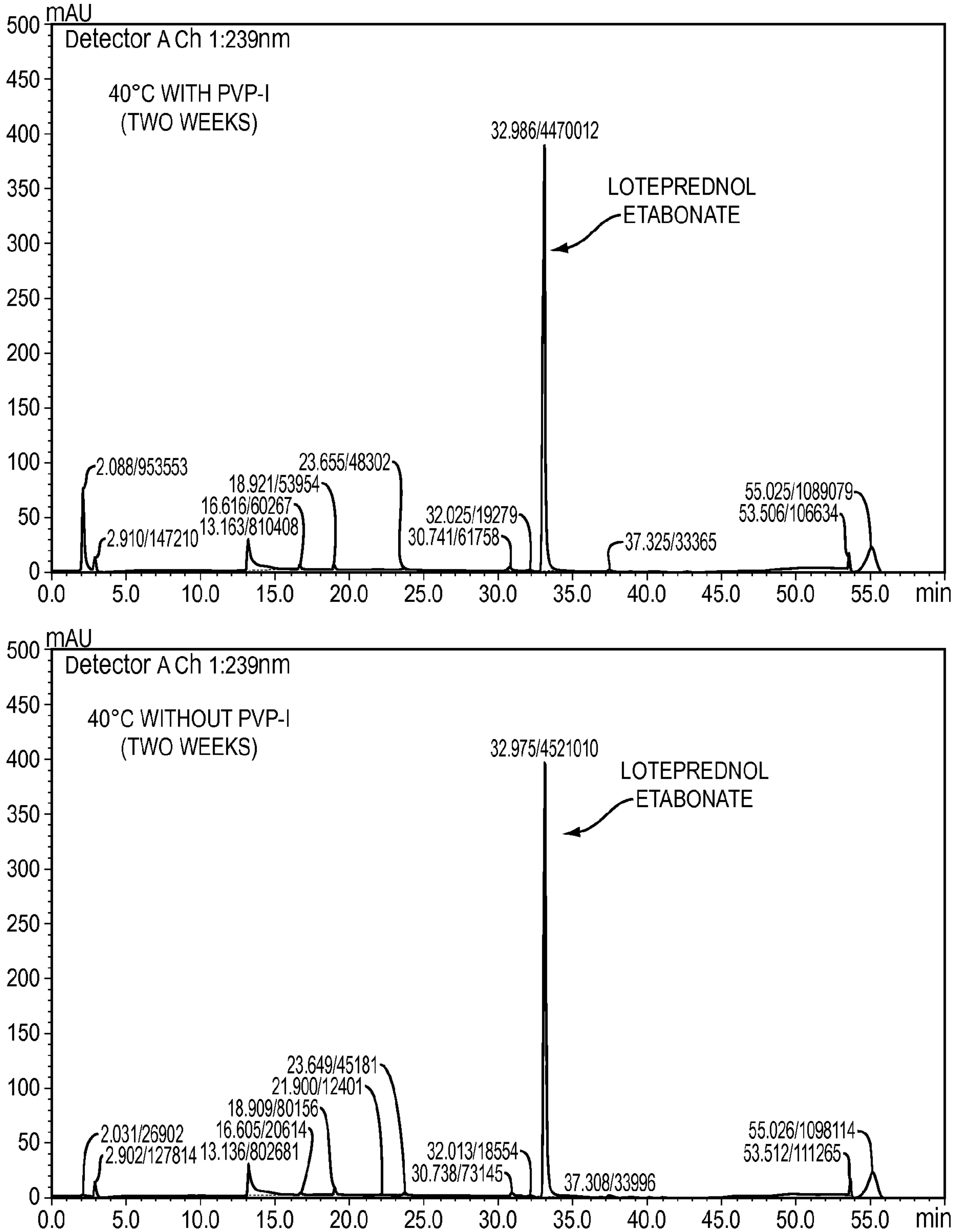


FIG. 31

HPLC/UV Chromatograms of Loteprednol Etabonate in PVP-I for One Month

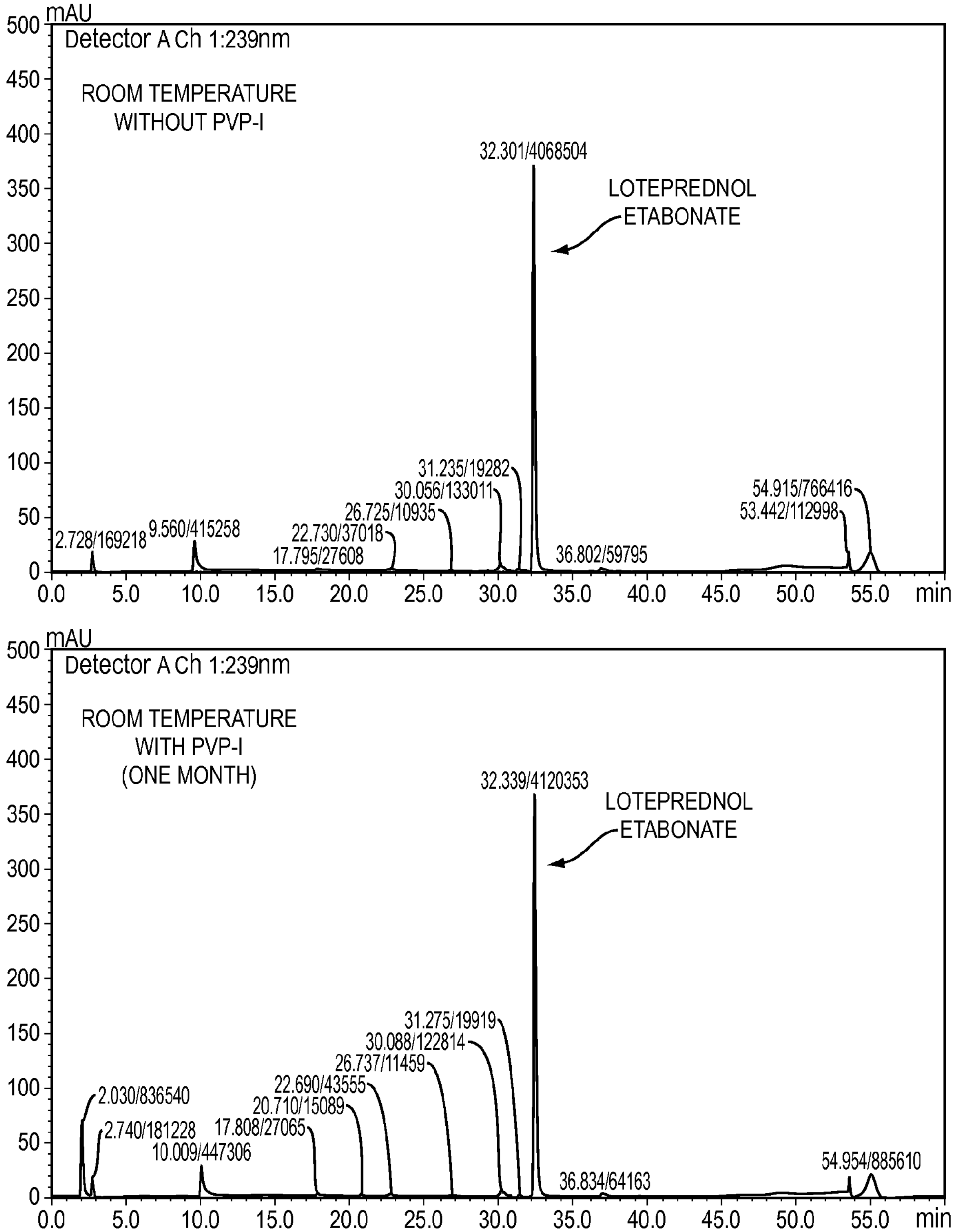


FIG. 32

HPLC/UV Chromatograms of Loteprednol Etabonate in PVP-I for One Month

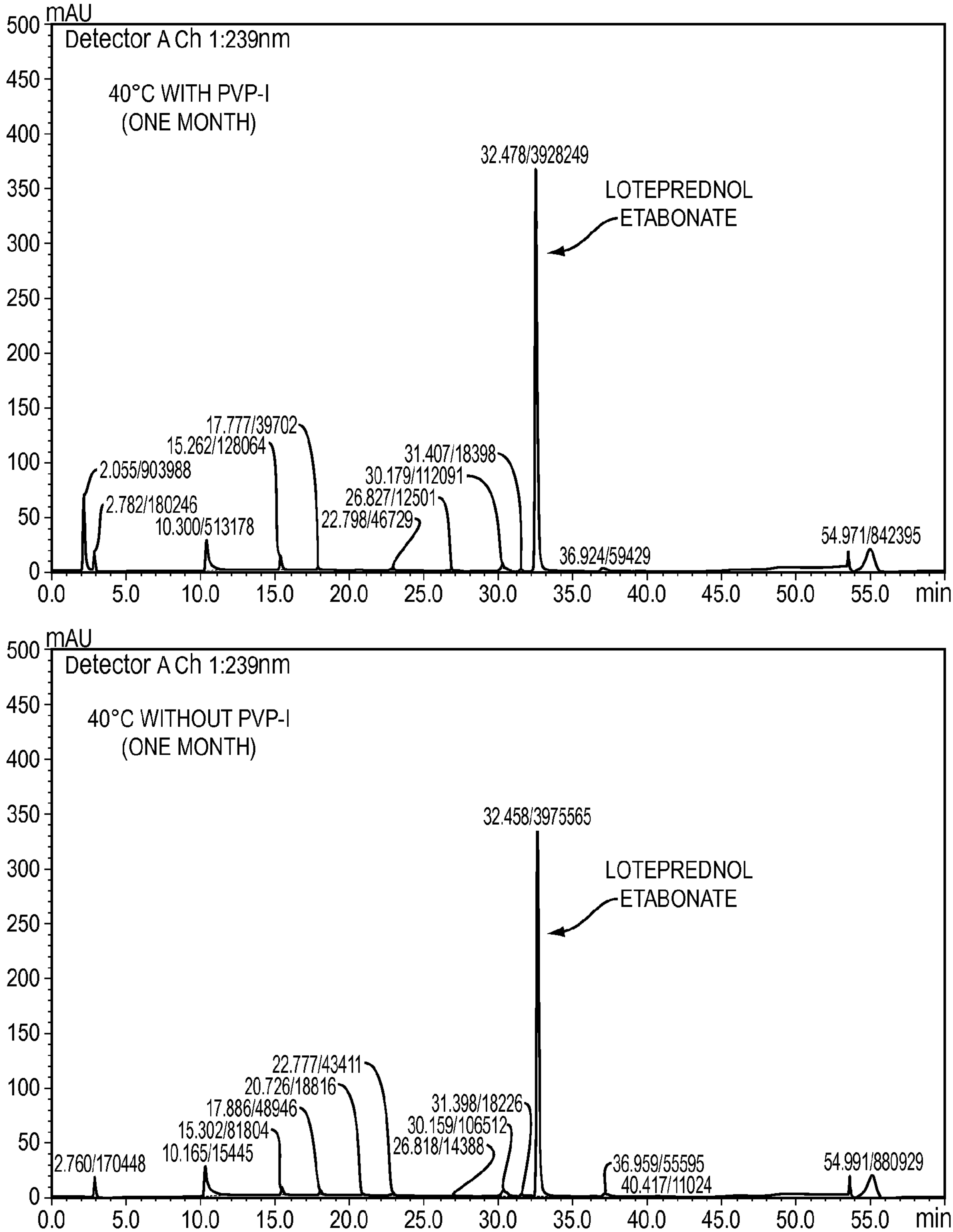


FIG. 33

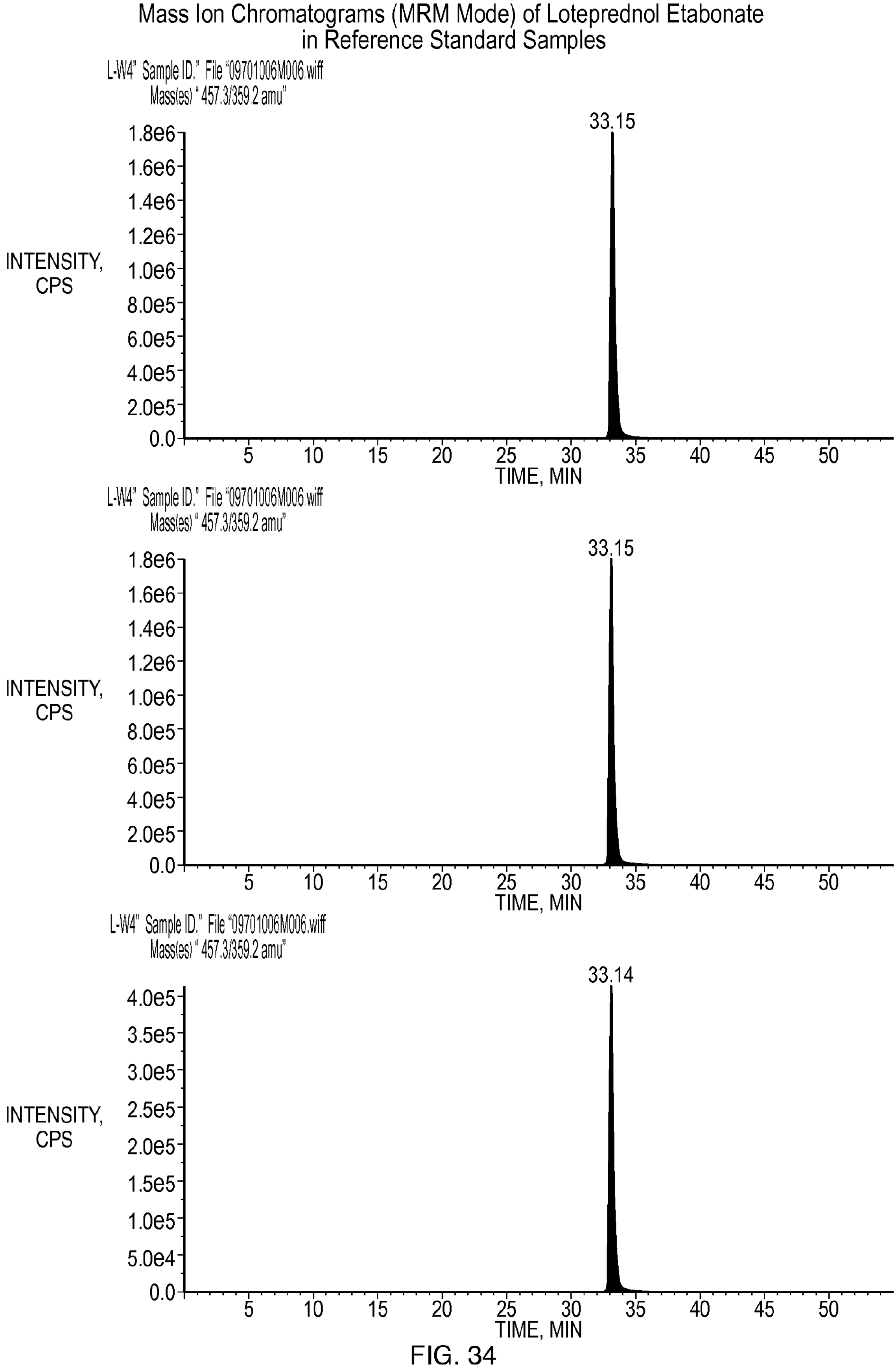
Mass Ion Chromatograms (MRM Mode) of Loteprednol Etabonate in Reference Standard Samples
L-W4" Sample ID." File "09701006M006.wiff
Mass(es) " 457.3/359.2 amu"
33.15
INTENSITY, CPS
TIME, MIN
L-W4" Sample ID." File "09701006M006.wiff
Mass(es) " 457.3/359.2 amu"
33.15
INTENSITY, CPS
TIME, MIN
L-W4" Sample ID." File "09701006M006.wiff
Mass(es) " 457.3/359.2 amu"
33.14
INTENSITY, CPS
TIME, MIN

FIG. 34

Mass Ion Chromatograms (MRM Mode) of Loteprednol Etabonate in One Month Room Temperature Stability Sample in the Presence of PVP-I

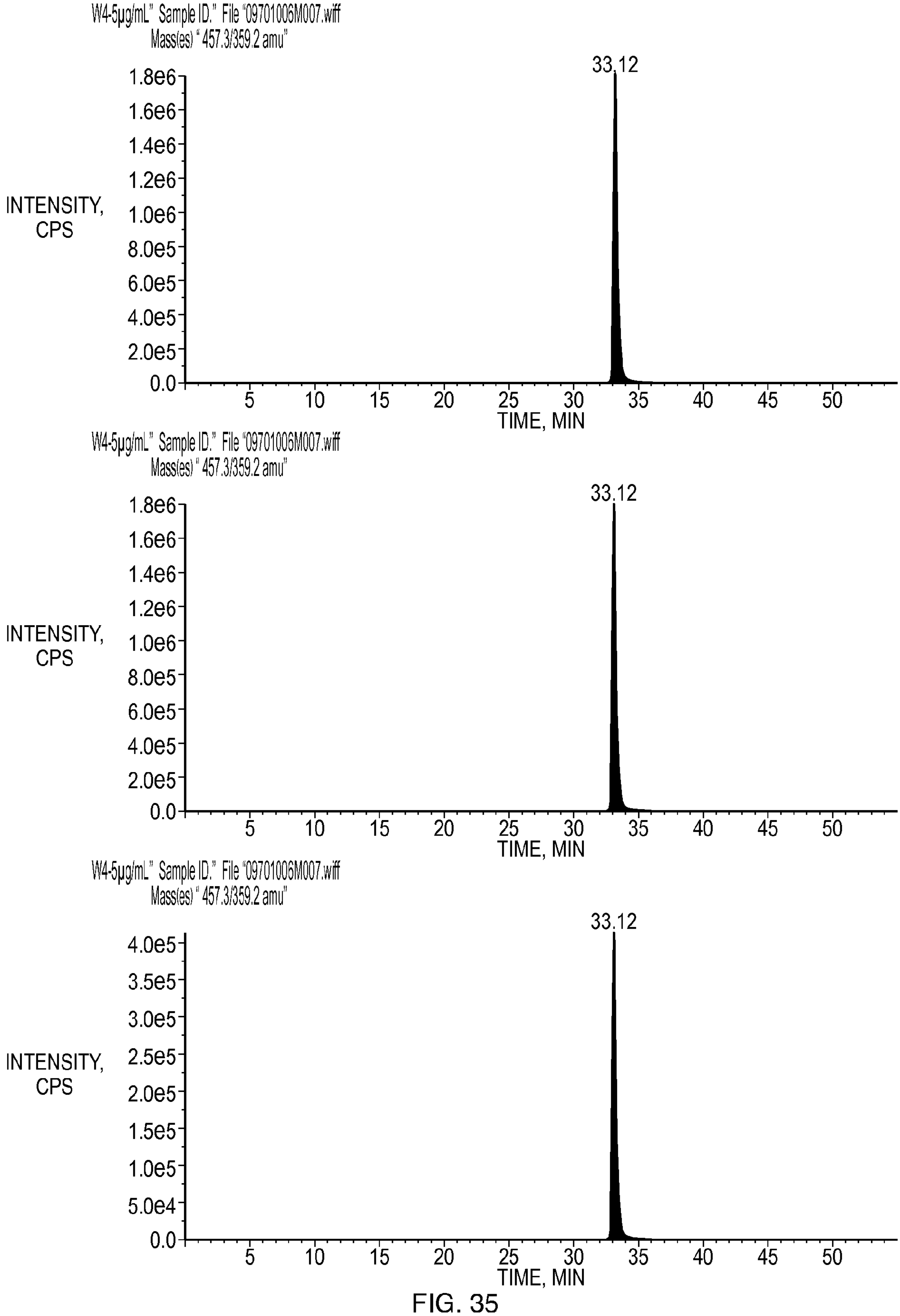


FIG. 35

Mass Ion Chromatograms (MRM Mode) of Loteprednol Etabonate in One Month 40°C Stability Sample in the Presence of PVP-I

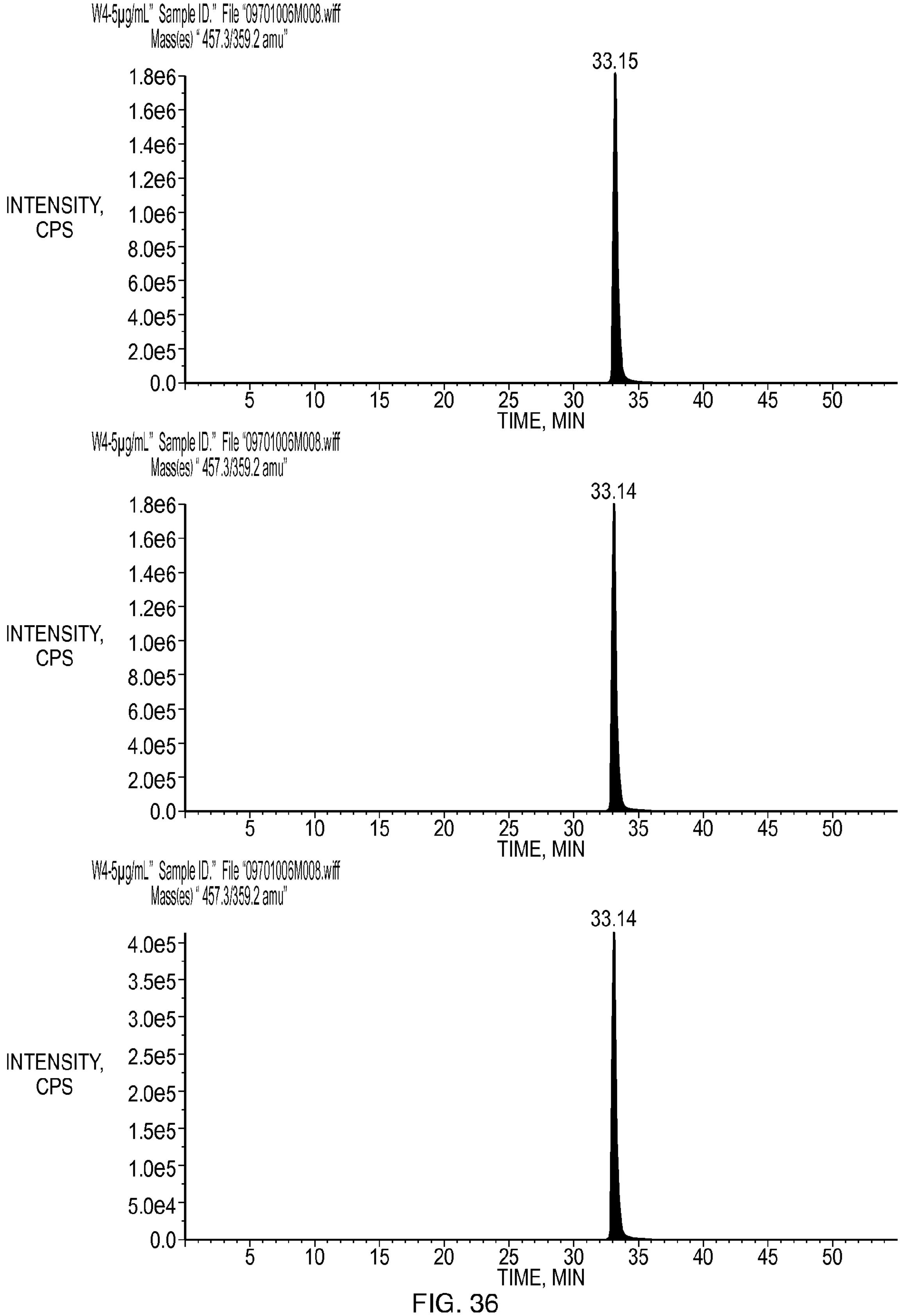


FIG. 36

HPLC/UV Chromatograms of PVP-I at the Concentration of 400 µg/mL for Difluprednate

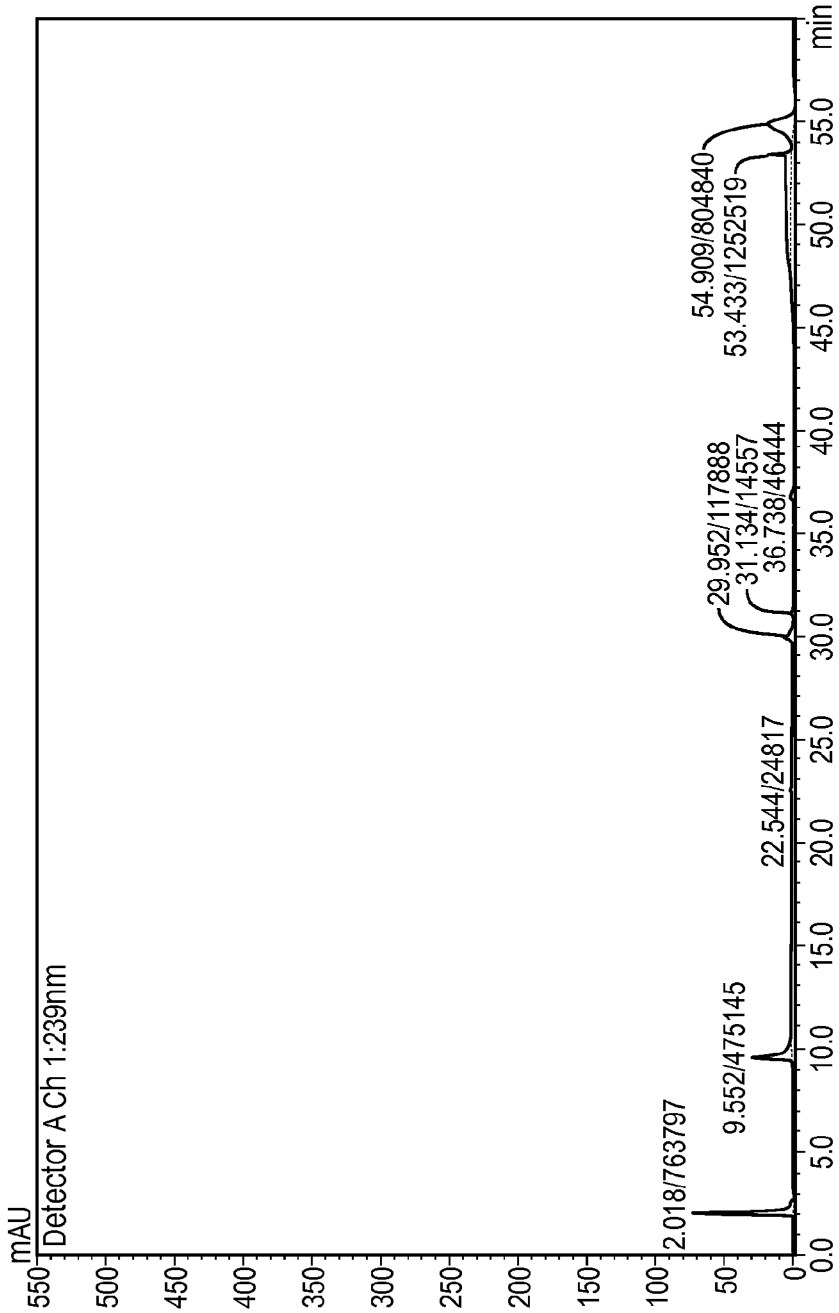


FIG. 37

HPLC/UV Chromatograms of Difluprednate in PVP-I for Day 0

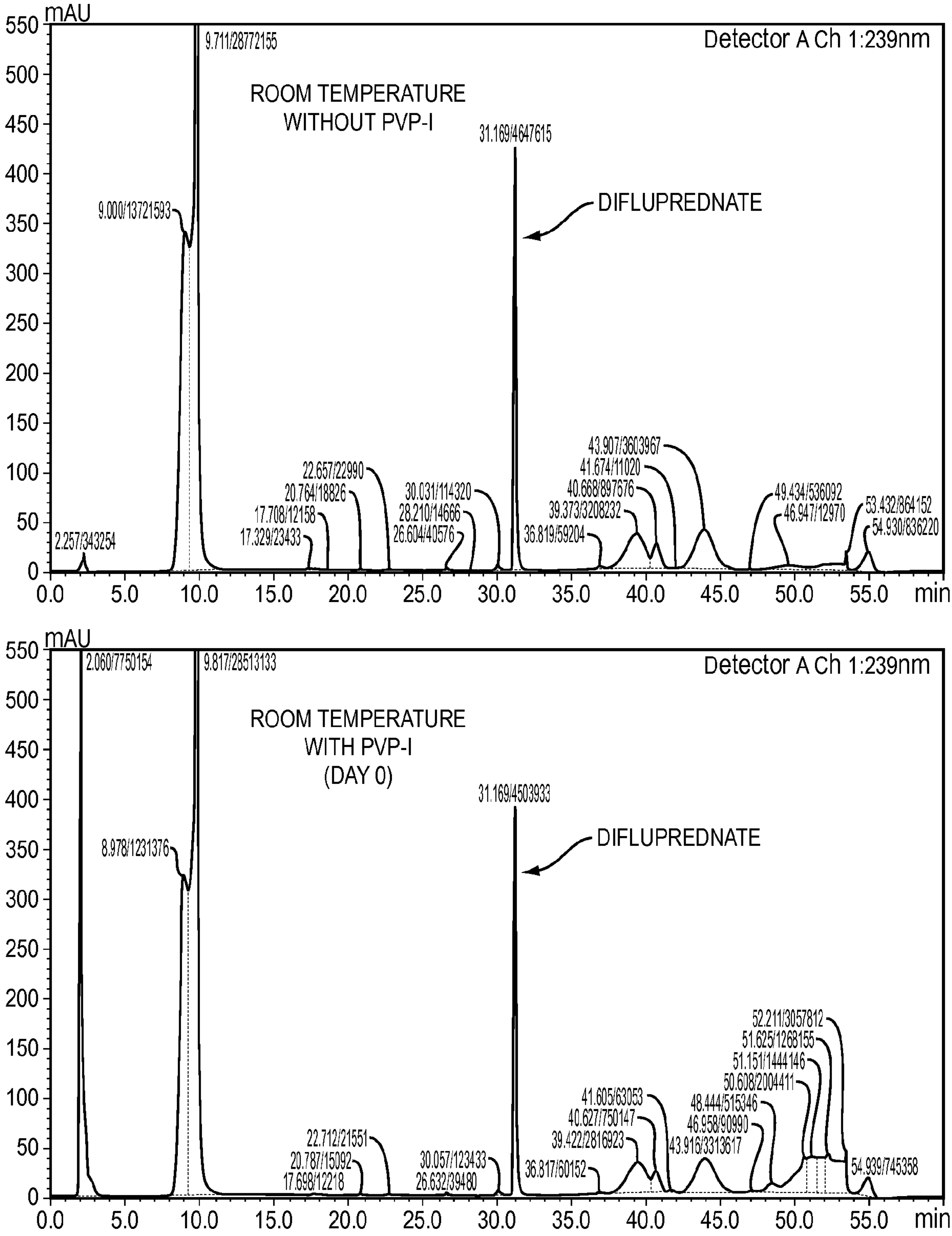


FIG. 38

HPLC/UV Chromatograms of Difluprednate in PVP-I for Day 0

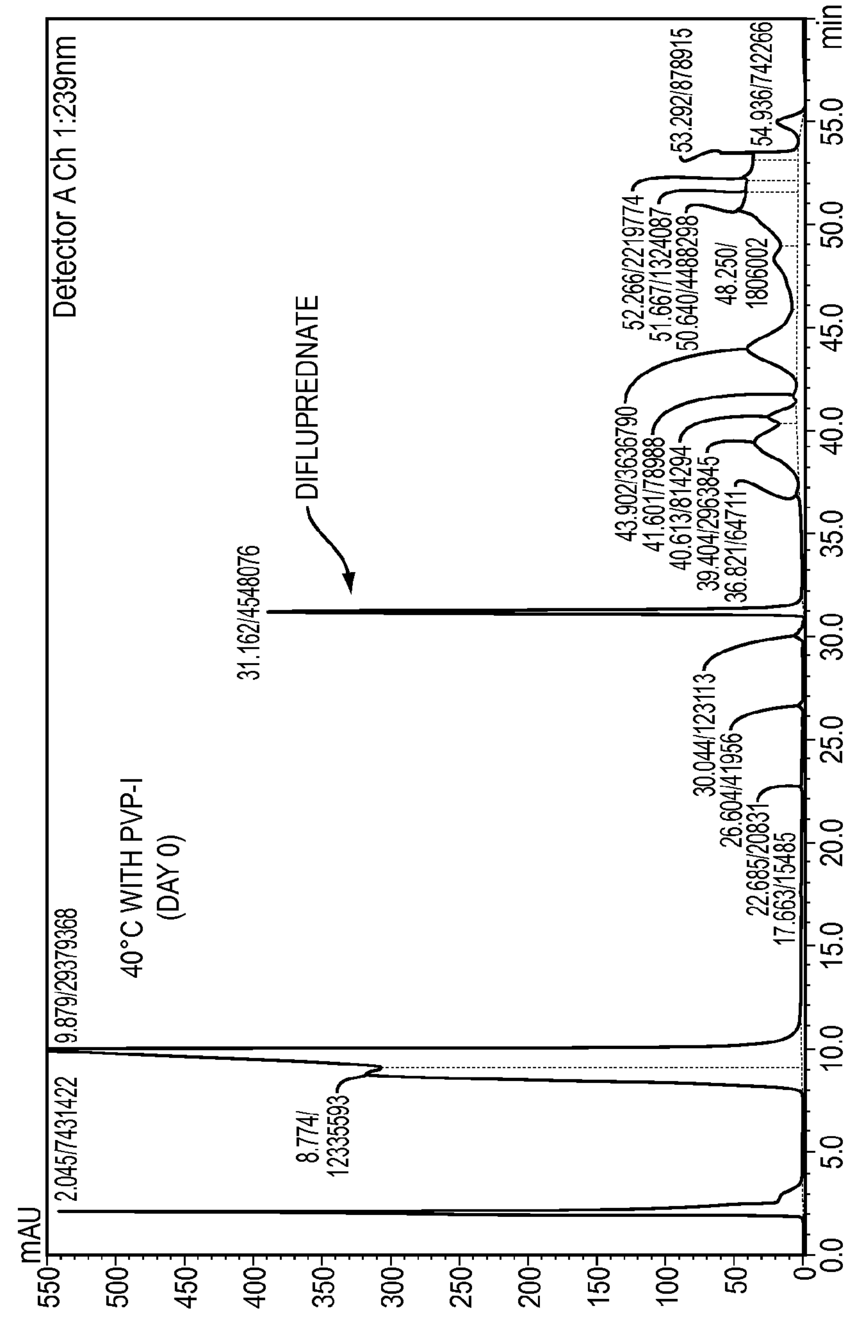


FIG. 39

HPLC/UV Chromatograms of Difluprednate in PVP-I for Two Weeks

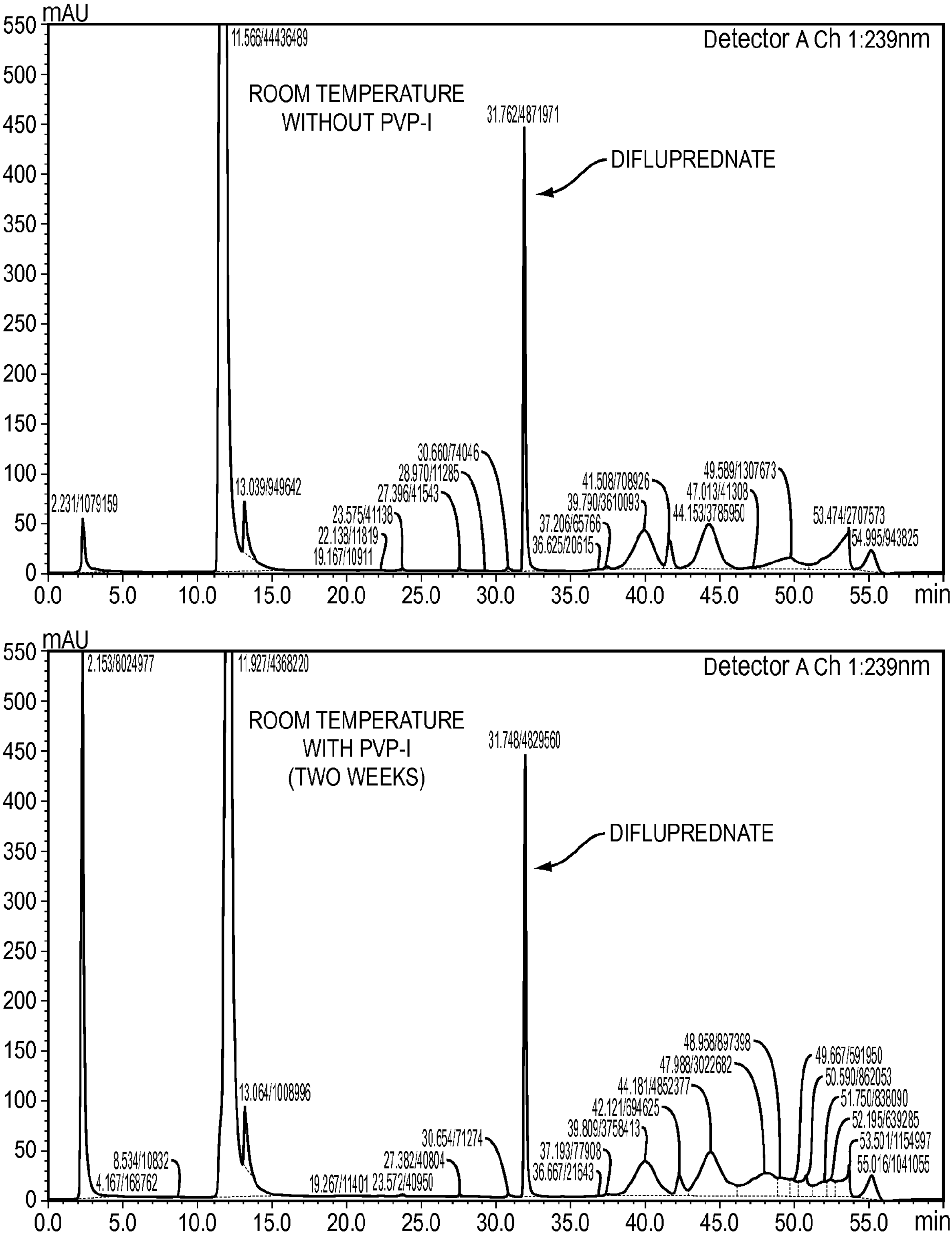


FIG. 40

HPLC/UV Chromatograms of Difluprednate in PVP-I for Two Weeks

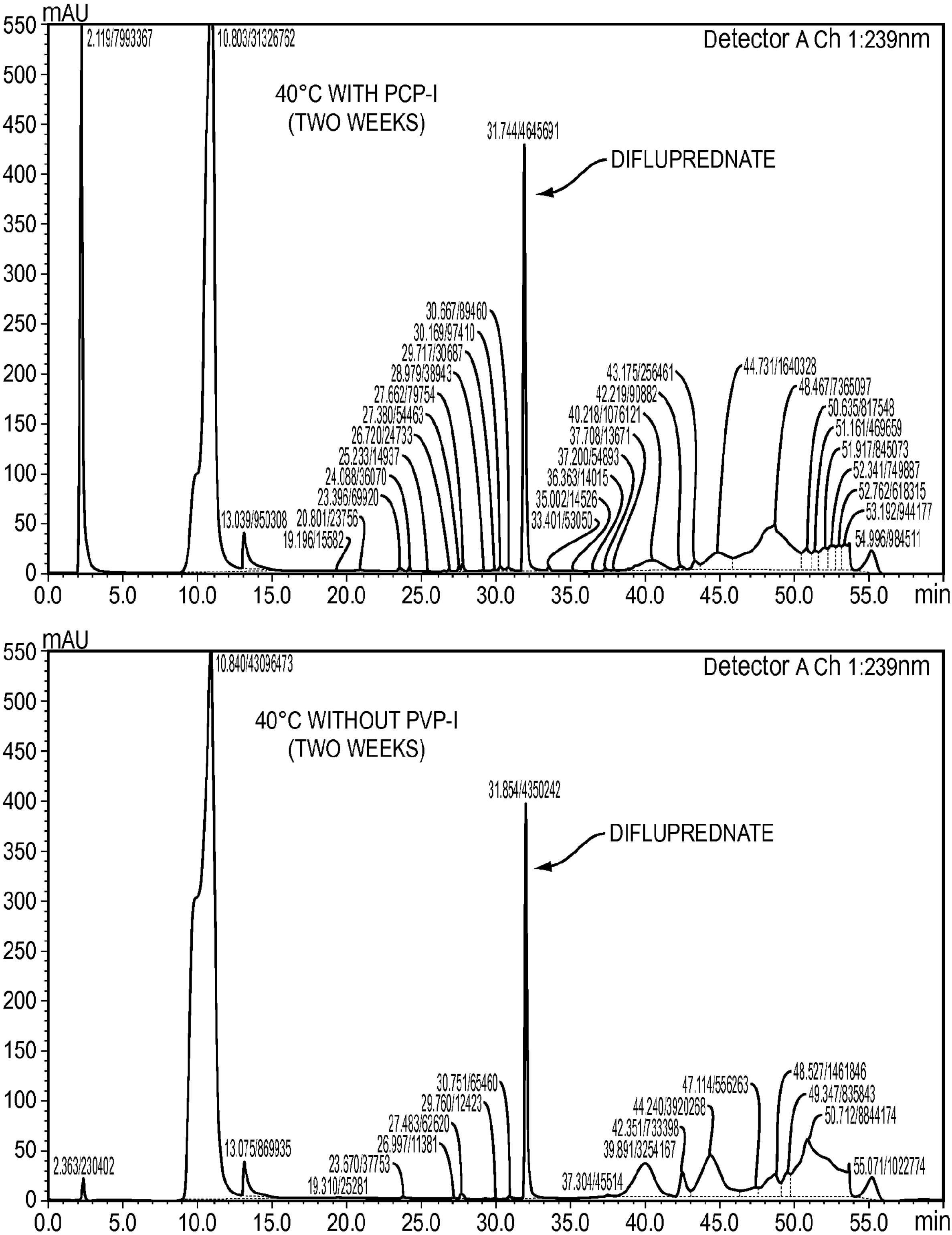


FIG. 41

HPLC/UV Chromatograms of Difluprednate in PVP-I for One Month

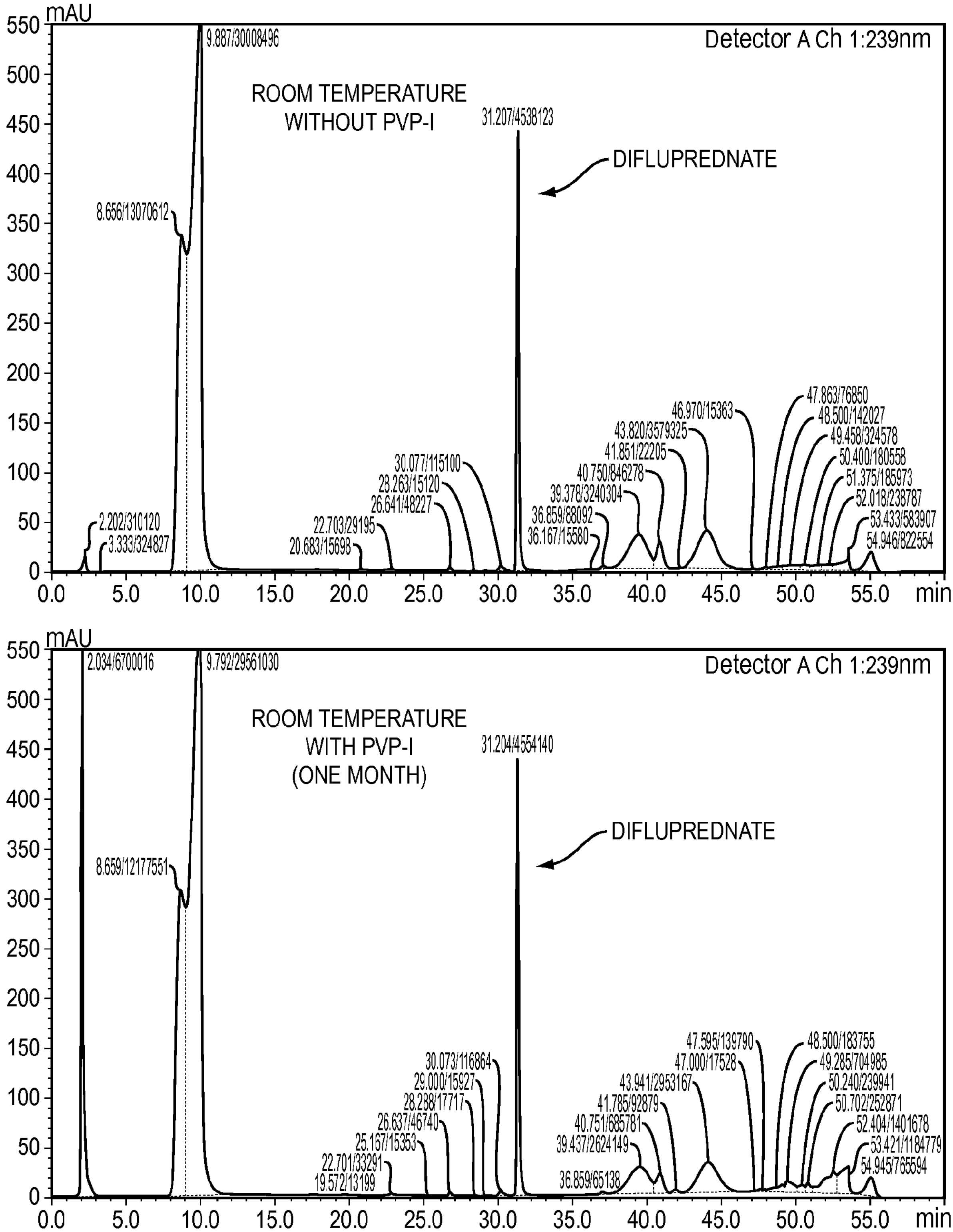


FIG. 42

HPLC/UV Chromatograms of Difluprednate in PVP-I for One Month

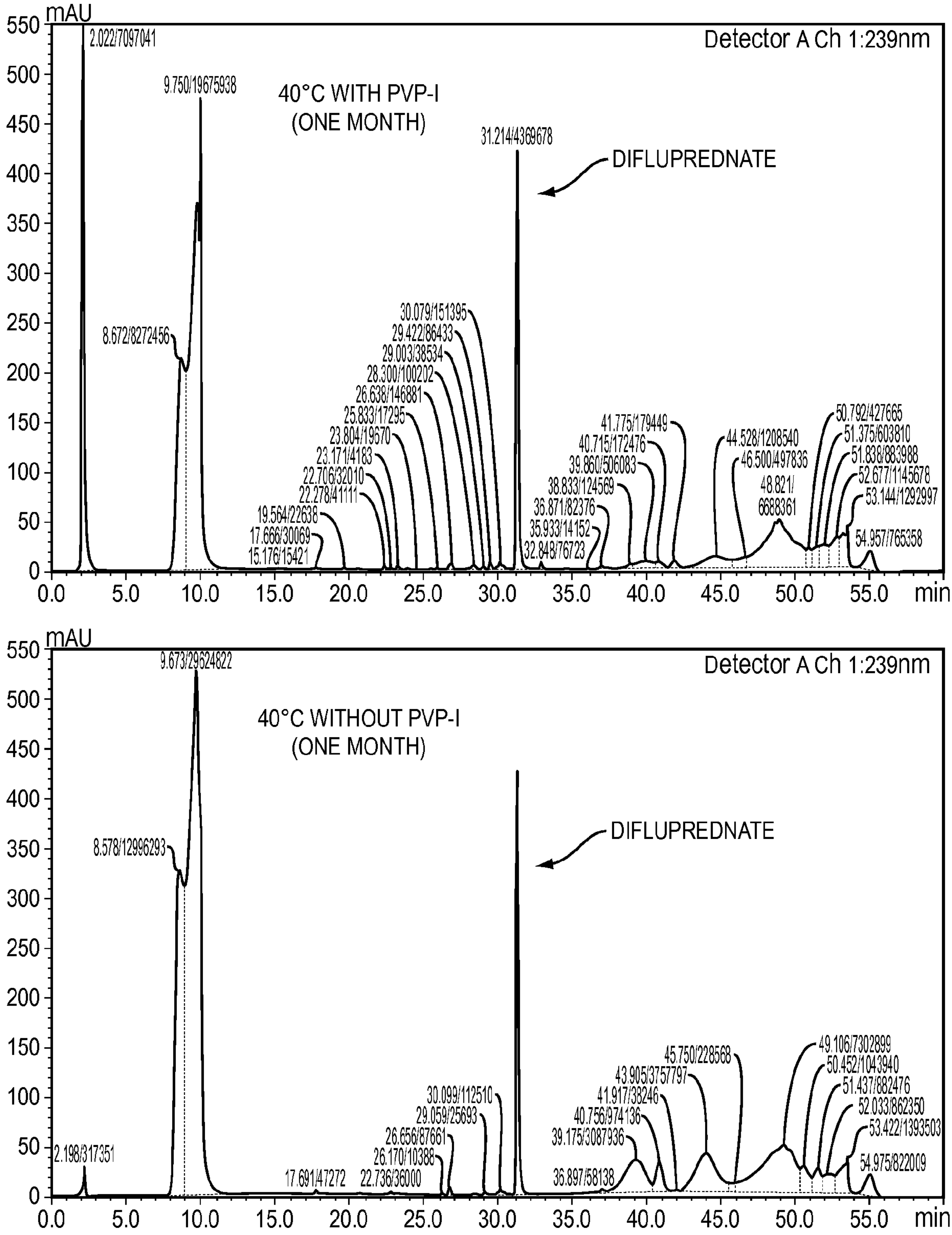


FIG. 43

Mass Ion Chromatograms (MRM Mode) of Difluprednate in Reference Standard Samples

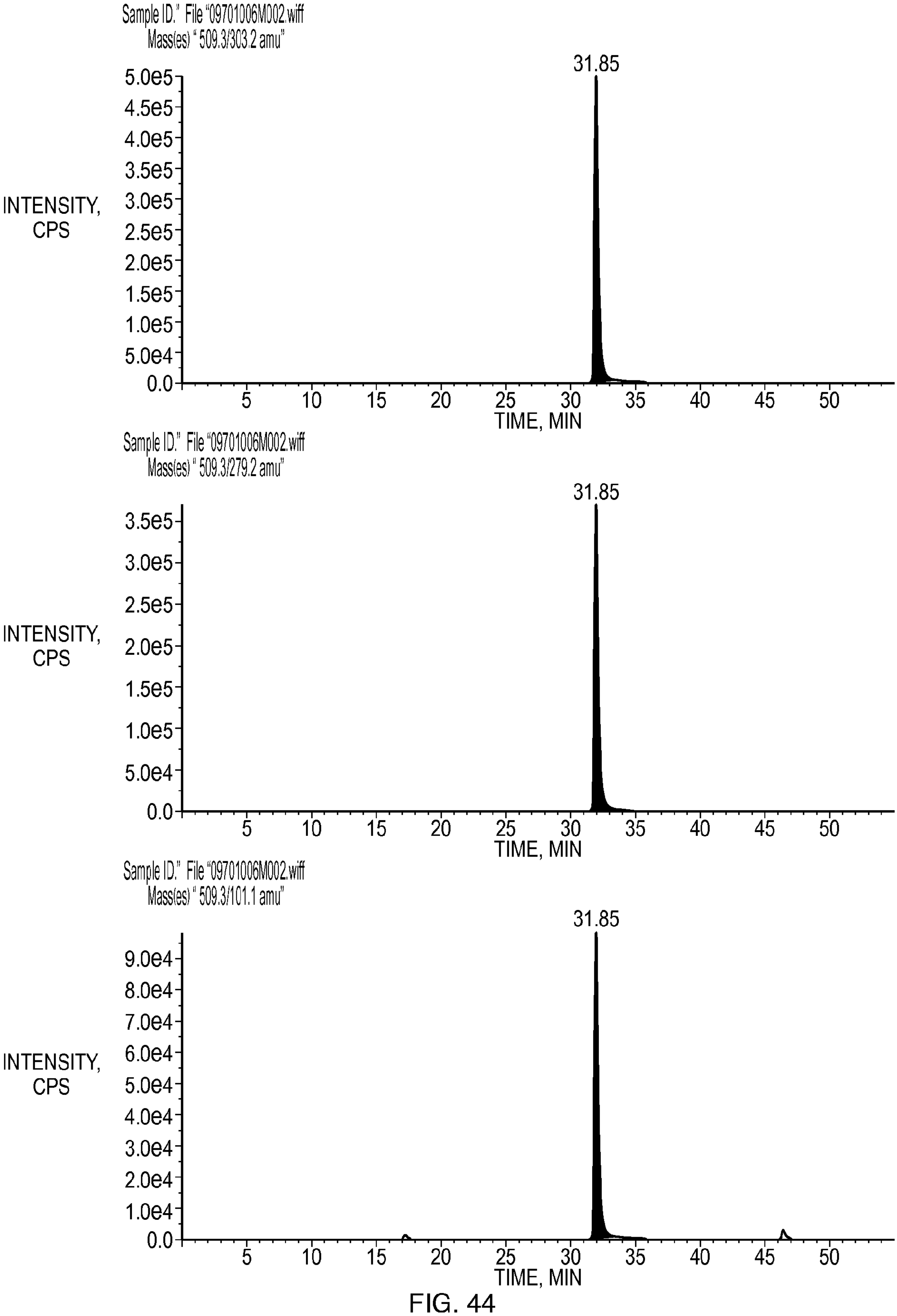


FIG. 44

Mass Ion Chromatograms (MRM Mode) of Difluprednate in One Month Room Temperature Stability Sample in the Presence of PVP-I

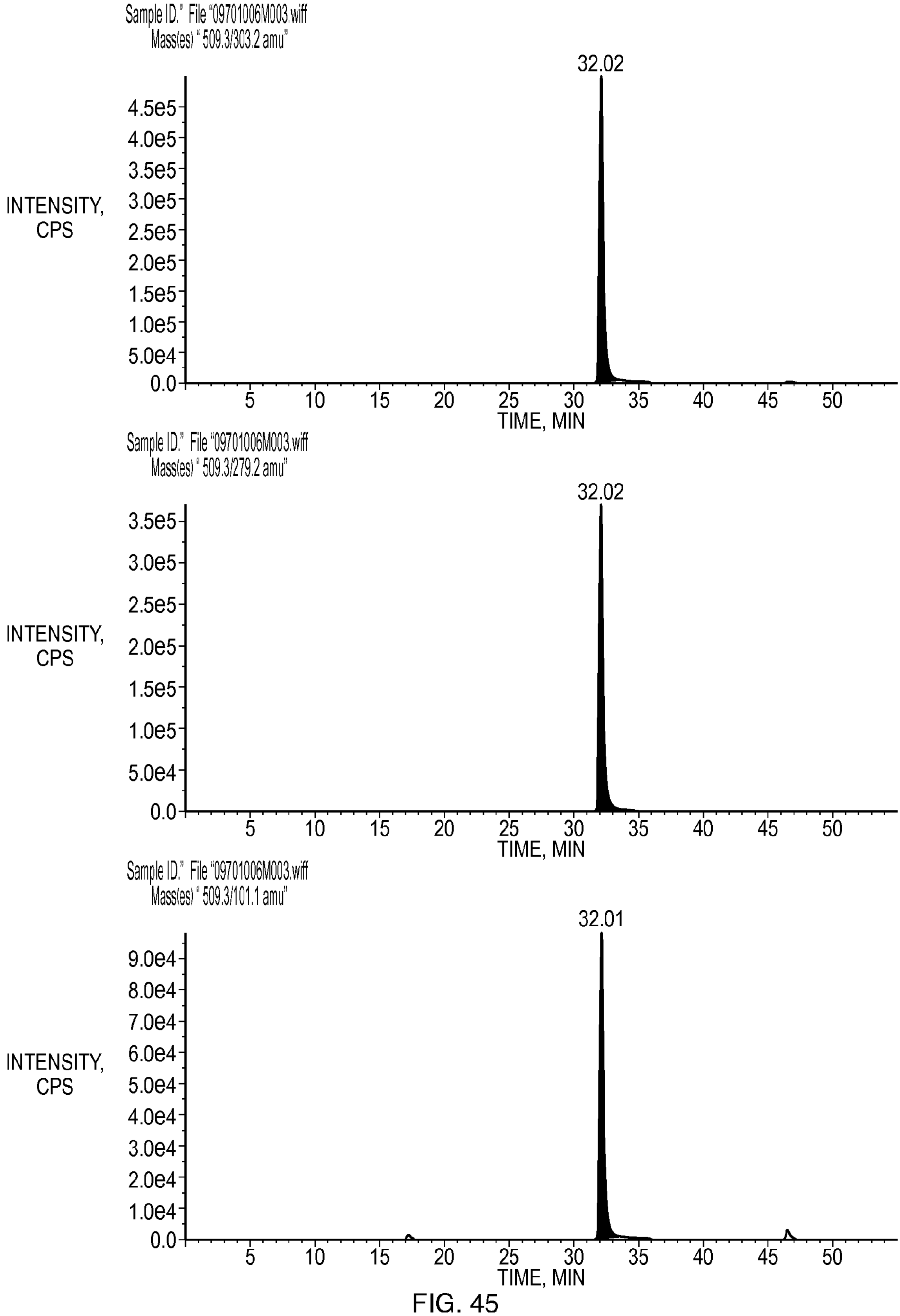


FIG. 45

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 7767217 B
- US 3886268 A

### Non-patent literature cited in the description

- **RACKUR H.J.** *Hosp. Infect.,* 1985, vol. 6, 13-23